# EUROPEAN PATENT APPLICATION

(11) **EP 4 371 572 A1**
(43) Date of publication of application: **22.05.2024**
(21) Application number: 22841350.6
(22) Date of filing: 12.07.2022
(51) Int. Cl.: A61K 39/395, C07K 16/28, C12N 15/13, C12N 15/09, A61P 35/00, A61P 27/00, A61P 37/00

(54) **ANTIBODY THAT SPECIFICALLY RECOGNIZES CD40 AND APPLICATION THEREOF**

(30) Priority: 14.07.2021 CN 202110793032
(71) Applicant: Staidson (Beijing) Biopharmaceuticals Co., Ltd., Beijing 100176 (CN)
(72) Inventor: ZHANG, Chao, Beijing 100176 (CN); YU, Debin, Beijing 100176 (CN); LI, Ping, Beijing 100176 (CN); YAN, Li, Beijing 100176 (CN)
(74) Representative: Graf von Stosch Patentanwaltsgesellschaft mbH
(86) International application number: PCT/CN2022/105107
(87) International publication number: WO 2023/284714

(57) **Abstract**

Provided are an antibody or antigen-binding fragment that specifically recognizes CD40, and a preparation method therefor and the use thereof.

## Description

### CROSS REFERENCE TO RELATED APPLICATIONS

This application claims priority to Chinese Patent Application No. 202110793032.1, filed on July 14, 2021 and entitled "ANTIBODIES SPECIFICALLY RECOGNIZING CD40 AND USES THEREOF", the contents of which are incorporated herein by reference in their entirety.

### REFERENCE TO AN ELECTRONIC SEQUENCE LISTING

The contents of the electronic sequence listing (file name: CN_202106295612_SEQLIST.xml, date recorded: 2022.07.12, size: 141 KB) is herein incorporated by reference in its entirety.

### FIELD OF THE APPLICATION

This application pertains to antibodies that specifically recognize CD40, and methods of manufacture and uses thereof.

### BACKGROUND OF THE APPLICATION

CD40 is a costimulatory member of the tumor necrosis factor receptor (TNFR) superfamily (Croft, Michael et al. Nature reviews. Drug discovery vol. 12,2 (2013): 147-68). The first functional characterization of CD40 was performed on human B lymphocytes, where it is constitutively expressed. CD40 signal could promote B cell activation and proliferation (Clark, E A, and J A Ledbetter. Proceedings of the National Academy of Sciences of the United States of America vol. 83,12 (1986): 4494-8). In addition to human B lymphocytes, CD40 is known to be expressed on other hematopoietic cells, such as monocytes and dendritic cells (DCs), where it promotes cell survival and cytokine production (Stout, R D, and J Suttles. Immunology today vol. 17,10 (1996): 487-92) and induces activation, proliferation of CD40L expressing CD4+ T cells and cytokine production (Grewal, I S, and R A Flavell. Immunology today vol. 17,9 (1996): 410-4). CD40 signal influences cellular responses on non-hematopoietic cells as well, including, endothelial cells, epithelial cells, fibroblasts and neuronal cells (Hollenbaugh, D et al. The Journal of experimental medicine vol. 182,1 (1995): 33-40; Karmann, K et al. Proceedings of the National Academy of Sciences of the United States of America vol. 92,10 (1995): 4342-6; and Tan, Jun et al. The EMBO journal vol. 21,4 (2002): 643-52).

CD40L was initially identified on the surface of activated CD4+ T cells (Hollenbaugh, D et al. The EMBO journal vol. 11,12 (1992): 4313-21), and CD40-CD40L interaction between B and T cells was shown to be critical for germinal center (GC) responses and isotype class switching in response to T-dependent antigens (Kawabe, T et al. Immunity vol. 1,3 (1994): 167-78). Additionally, CD40L has been shown to be expressed on a wide variety of cells including, mast cells, basophils, B cells, natural killer (NK) cells, macrophages, megakaryocytes and platelets, highlighting a broad role of CD40 pathway in cellular biology (Schönbeck, U, and P Libby. Cellular and molecular life sciences: CMLS vol. 58,1 (2001): 4-43; and van Kooten, C, and J Banchereau. Journal of leukocyte biology vol. 67,1 (2000): 2-17).

In a number of animal models, the CD40L/CD40 interaction has been shown to be involved in many diseases, including systemic lupus erythematosus (Davidson, Anne et al. Annals of the New York Academy of Sciences vol. 987 (2003): 188-98), experimental allergic encephalomyelitis (Toubi, Elias, and Yehuda Shoenfeld. Autoimmunity vol. 37,6-7 (2004): 457-64), collagen-induced arthritis (Durie, F H et al. Science (New York, N.Y.) vol. 261,5126 (1993): 1328-30), immunological rejection (Goldwater, R et al. American journal of transplantation vol. 13,4 (2013): 1040-1046), atherogenesis-mediated cardiovascular disorders (Jansen, Matthijs F et al. Basic research in cardiology vol. 111,4 (2016): 38), Sjogren's syndrome (Okimura, K et al. American journal of transplantation vol. 14,6 (2014): 1290-9), systemic sclerosis (Boleto, Gonçalo et al. Frontiers in immunology vol. 9 2998. 18 Dec. 2018), immune thrombocytopenic purpura (Kuwana, Masataka et al. Blood vol. 103,4 (2004): 1229-36), inflammatory bowel disease (Senhaji, Nezha et al. Frontiers in immunology vol. 6 529. 16 Oct. 2015), diabetes (Balasa, B et al. Journal of immunology (Baltimore, Md.: 1950) vol. 159,9 (1997): 4620-7), RA (Gotoh, Haruhiko et al. The Journal of rheumatology vol. 31,8 (2004): 1506-12) and cancer (Li, Rui et al. Molecular biology reports vol. 39,9 (2012): 8741-7; and Li, Rui et al. Immunology letters vol. 131,2 (2010): 120-5).

Neutralization of CD40 binding to CD40L is therefore a therapeutic approach to treating diseases and conditions mediated through CD40/CD40L pathway. An antibody against human CD40, designated ASKP1240 (used as a control in the Examples), was described in WG200563981A1. Another antibody against human CD40, designated CFZ533 (used as a control in the Examples), was described in WO201265950A1.

The disclosures of all publications, patents, patent applications and published patent applications referred to herein are hereby incorporated herein by reference in their entirety.

### BRIEF SUMMARY OF THE APPLICATION

In some embodiments, there is provided an isolated anti-CD40 antibody comprising: a heavy chain variable domain (V_{H}) comprising a heavy chain complementarity determining region (HC-CDR) 1 comprising GYX₁VN (SEQ ID NO: 81), wherein X₁ is L, T, or V; an HC-CDR2 comprising X₁IX₂PX₃X₄GX₅TX₆YNQKFKG (SEQ ID NO: 82), wherein X₁ is L or M, X₂ is A, N, or V, X₃ is E, K, L, M, R, S, T, or Y, X₄ is H, N, S, or T, X₅ is G or R, and X₆ is A, S, T, or W; and an HC-CDR3 comprising SX₁X₂X₃X₄PYAX₅DY (SEQ ID NO: 83), wherein X₁ is G, 1, L, M, P, R, S, or Y, X₂ is W or Y, X₃ is S or V, X₄ is D, K, L, N, R, T, or V, and X₅ is A, G, M, N, Q, or S; and a light chain variable domain (V_{L}) comprising a light chain complementarity determining region (LC-CDR) 1 comprising KASQNVHTX₁ VA (SEQ ID NO: 84), wherein X₁ is A, H, N, or R; an LC-CDR2 comprising LX₁SDRRT (SEQ ID NO: 85), wherein X₁ is A or R; and an LC-CDR3 comprising LQX₁ WX₂X₃PLT (SEQ ID NO: 86), wherein X₁ is A or H, X₂ is N, S, or T, and X₃ is A, Q, R, S, or Y.

In some embodiments, there is provided an isolated anti-CD40 antibody comprising: a V_{H} comprising an HC-CDR1 comprising the amino acid sequence of any one of SEQ ID NOs: 1-3, or a variant thereof comprising up to about 3 amino acid substitutions; an HC-CDR2 comprising the amino acid sequence of any one of SEQ ID NOs: 9-26, or a variant thereof comprising up to about 3 amino acid substitutions; and an HC-CDR3 comprising the amino acid sequence of any one of SEQ ID NOs: 32-52, or a variant thereof comprising up to about 3 amino acid substitutions; and a V_{L} comprising an LC-CDR1 comprising the amino acid sequence of any one of SEQ ID NOs: 58-61, or a variant thereof comprising up to about 3 amino acid substitutions; an LC-CDR2 comprising the amino acid sequence of any one of SEQ ID NOs: 65-66, or a variant thereof comprising up to about 3 amino acid substitutions; and an LC-CDR3 comprising the amino acid sequence of any one of SEQ ID NOs: 70-77, or a variant thereof comprising up to about 3 amino acid substitutions.

In some embodiments, there is provided an isolated anti-CD40 antibody comprising a V_{H} comprising an HC-CDR1, an HC-CDR2, and an HC-CDR3 of the V_{H} comprising the amino acid sequence of any one of SEQ ID NOs: 87-119; and a V_{L} comprising an LC-CDR1, an LC-CDR2, and an LC-CDR3 of the V_{L} comprising the amino acid sequence of any one of SEQ ID NOs: 126-138.

In some embodiments, there is provided an isolated anti-CD40 antibody comprising: (i) a V_{H} comprising an HC-CDR1, an HC-CDR2, and an HC-CDR3 of a V_{H} comprising the amino acid sequence of SEQ ID NO: 87; and a V_{L} comprising an LC-CDR1, an LC-CDR2, and an LC-CDR3 of a V_{L} comprising the amino acid sequence of SEQ ID NO: 126; (ii) a V_{H} comprising an HC-CDR1, an HC-CDR2, and an HC-CDR3 of a V_{H} comprising the amino acid sequence of SEQ ID NO: 88; and a V_{L} comprising an LC-CDR1, an LC-CDR2, and an LC-CDR3 of a V_{L} comprising the amino acid sequence of SEQ ID NO: 127; (iii) a V_{H} comprising an HC-CDR1, an HC-CDR2, and an HC-CDR3 of a V_{H} comprising the amino acid sequence of SEQ ID NO: 89; and a V_{L} comprising an LC-CDR1, an LC-CDR2, and an LC-CDR3 of a V_{L} comprising the amino acid sequence of SEQ ID NO: 128; (iv) a V_{H} comprising an HC-CDR1, an HC-CDR2, and an HC-CDR3 of a V_{H} comprising the amino acid sequence of SEQ ID NO: 94; and a V_{L} comprising an LC-CDR1, an LC-CDR2, and an LC-CDR3 of a V_{L} comprising the amino acid sequence of SEQ ID NO: 128; (v) a V_{H} comprising an HC-CDR1, an HC-CDR2, and an HC-CDR3 of a V_{H} comprising the amino acid sequence of SEQ ID NO: 96; and a V_{L} comprising an LC-CDR1, an LC-CDR2, and an LC-CDR3 of a V_{L} comprising the amino acid sequence of SEQ ID NO: 132; (vi) a V_{H} comprising an HC-CDR1, an HC-CDR2, and an HC-CDR3 of a V_{H} comprising the amino acid sequence of SEQ ID NO: 101; and a V_{L} comprising an LC-CDR1, an LC-CDR2, and an LC-CDR3 of a V_{L} comprising the amino acid sequence of SEQ ID NO: 128; (vii) a V_{H} comprising an HC-CDR1, an HC-CDR2, and an HC-CDR3 of a V_{H} comprising the amino acid sequence of SEQ ID NO: 110; and a V_{L} comprising an LC-CDR1, an LC-CDR2, and an LC-CDR3 of a V_{L} comprising the amino acid sequence of SEQ ID NO: 128; (viii) a V_{H} comprising an HC-CDR1, an HC-CDR2, and an HC-CDR3 of a V_{H} comprising the amino acid sequence of SEQ ID NO: 114; and a V_{L} comprising an LC-CDR1, an LC-CDR2, and an LC-CDR3 of a V_{L} comprising the amino acid sequence of SEQ ID NO: 128; (ix) a V_{H} comprising an HC-CDR1, an HC-CDR2, and an HC-CDR3 of a V_{H} comprising the amino acid sequence of SEQ ID NO: 115; and a V_{L} comprising an LC-CDR1, an LC-CDR2, and an LC-CDR3 of a V_{L} comprising the amino acid sequence of SEQ ID NO: 129; or (x) a V_{H} comprising an HC-CDR1, an HC-CDR2, and an HC-CDR3 of a V_{H} comprising the amino acid sequence of SEQ ID NO: 117; and a V_{L} comprising an LC-CDR1, an LC-CDR2, and an LC-CDR3 of a V_{L} comprising the amino acid sequence of SEQ ID NO: 129.

In some embodiments, there is provided an isolated anti-CD40 antibody comprising: (i) a V_{H} comprising an HC-CDR1 comprising the amino acid sequence of SEQ ID NO: 1, an HC-CDR2 comprising the amino acid sequence of SEQ ID NO: 9, and an HC-CDR3 comprising the amino acid sequence of SEQ ID NO: 32, or a variant thereof comprising up to about 5 amino acid substitutions in the HC-CDRs; and a V_{L} comprising an LC-CDR1 comprising the amino acid sequence of SEQ ID NO: 58, an LC-CDR2 comprising the amino acid sequence of SEQ ID NO: 65, and an LC-CDR3 comprising the amino acid sequence of SEQ ID NO: 70, or a variant thereof comprising up to about 5 amino acid substitutions in the LC-CDRs; (ii) a V_{H} comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 32, or a variant thereof comprising up to about 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70, or a variant thereof comprising up to about 5 amino acid substitutions in the LC-CDRs; (iii) a V_{H} comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 34, or a variant thereof comprising up to about 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70, or a variant thereof comprising up to about 5 amino acid substitutions in the LC-CDRs; (iv) a V_{H} comprising an HC-CDR1 comprising the amino acid sequence of SEQ ID NO: 1, an HC-CDR2 comprising the amino acid sequence of SEQ ID NO: 15, and an HC-CDR3 comprising the amino acid sequence of SEQ ID NO: 37, or a variant thereof comprising up to about 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 73, or a variant thereof comprising up to about 5 amino acid substitutions in the LC-CDRs; (v) a V_{H} comprising an HC-CDR1 comprising the amino acid sequence of SEQ ID NO: 1, an HC-CDR2 comprising the amino acid sequence of SEQ ID NO: 20, and an HC-CDR3 comprising the amino acid sequence of SEQ ID NO: 42, or a variant thereof comprising up to about 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70, or a variant thereof comprising up to about 5 amino acid substitutions in the LC-CDRs; (vi) a V_{H} comprising an HC-CDR1 comprising the amino acid sequence of SEQ ID NO: 1, an HC-CDR2 comprising the amino acid sequence of SEQ ID NO: 14, and an HC-CDR3 comprising the amino acid sequence of SEQ ID NO: 43, or a variant thereof comprising up to about 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70, or a variant thereof comprising up to about 5 amino acid substitutions in the LC-CDRs; (vii) a V_{H} comprising an HC-CDR1 comprising the amino acid sequence of SEQ ID NO: 1, an HC-CDR2 comprising the amino acid sequence of SEQ ID NO: 15, and an HC-CDR3 comprising the amino acid sequence of SEQ ID NO: 51, or a variant thereof comprising up to about 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70, or a variant thereof comprising up to about 5 amino acid substitutions in the LC-CDRs; (viii) a V_{H} comprising an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 48, or a variant thereof comprising up to about 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 71, or a variant thereof comprising up to about 5 amino acid substitutions in the LC-CDRs; or (ix) a V_{H} comprising an HC-CDR1 comprising the amino acid sequence of SEQ ID NO: 1, an HC-CDR2 comprising the amino acid sequence of SEQ ID NO: 18, and an HC-CDR3 comprising the amino acid sequence of SEQ ID NO: 47, or a variant thereof comprising up to about 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 71, or a variant thereof comprising up to about 5 amino acid substitutions in the LC-CDRs.

In some embodiments, according to any one of the isolated anti-CD40 antibodies described above, the isolated anti-CD40 antibody comprises: a V_{H} comprising the amino acid sequence of any one of SEQ ID NOs: 87-119, or a variant thereof having at least about 90% sequence identity to the amino acid sequence of any one of SEQ ID NOs: 87-119; and a V_{L} comprising the amino acid sequence of any one of SEQ ID NOs: 126-138, or a variant thereof having at least about 90% sequence identity to the amino acid sequence of any one of SEQ ID NOs: 126-138.

In some embodiments, the isolated anti-CD40 antibody comprises: (i) a V_{H} comprising the amino acid sequence of SEQ ID NO: 87, or a variant thereof having at least about 90% sequence identity to the amino acid sequence of SEQ ID NO: 87; and a V_{L} comprising the amino acid sequence of SEQ ID NO: 126, or a variant thereof having at least about 90% sequence identity to the amino acid sequence of SEQ ID NO: 126; (ii) a V_{H} comprising the amino acid sequence of SEQ ID NO: 88, or a variant thereof having at least about 90% sequence identity to the amino acid sequence of SEQ ID NO: 88; and a V_{L} comprising the amino acid sequence of SEQ ID NO: 127, or a variant thereof having at least about 90% sequence identity to the amino acid sequence of SEQ ID NO: 127; (iii) a V_{H} comprising the amino acid sequence of SEQ ID NO: 89, or a variant thereof having at least about 90% sequence identity to the amino acid sequence of SEQ ID NO: 89; and a V_{L} comprising the amino acid sequence of SEQ ID NO: 128, or a variant thereof having at least about 90% sequence identity to the amino acid sequence of SEQ ID NO: 128; (iv) a V_{H} comprising the amino acid sequence of SEQ ID NO: 94, or a variant thereof having at least about 90% sequence identity to the amino acid sequence of SEQ ID NO: 94; and a V_{L} comprising the amino acid sequence of SEQ ID NO: 128, or a variant thereof having at least about 90% sequence identity to the amino acid sequence of SEQ ID NO: 128; (v) a V_{H} comprising the amino acid sequence of SEQ ID NO: 96, or a variant thereof having at least about 90% sequence identity to the amino acid sequence of SEQ ID NO: 96; and a V_{L} comprising the amino acid sequence of SEQ ID NO: 132, or a variant thereof having at least about 90% sequence identity to the amino acid sequence of SEQ ID NO: 132; (vi) a V_{H} comprising the amino acid sequence of SEQ ID NO: 101, or a variant thereof having at least about 90% sequence identity to the amino acid sequence of SEQ ID NO: 101; and a V_{L} comprising the amino acid sequence of SEQ ID NO: 128, or a variant thereof having at least about 90% sequence identity to the amino acid sequence of SEQ ID NO: 128; (vii) a V_{H} comprising the amino acid sequence of SEQ ID NO: 110, or a variant thereof having at least about 90% sequence identity to the amino acid sequence of SEQ ID NO: 110; and a V_{L} comprising the amino acid sequence of SEQ ID NO: 128, or a variant thereof having at least about 90% sequence identity to the amino acid sequence of SEQ ID NO: 128; (viii) a V_{H} comprising the amino acid sequence of SEQ ID NO: 114, or a variant thereof having at least about 90% sequence identity to the amino acid sequence of SEQ ID NO: 114; and a V_{L} comprising the amino acid sequence of SEQ ID NO: 128, or a variant thereof having at least about 90% sequence identity to the amino acid sequence of SEQ ID NO: 128; (ix) a V_{H} comprising the amino acid sequence of SEQ ID NO: 115, or a variant thereof having at least about 90% sequence identity to the amino acid sequence of SEQ ID NO: 115; and a V_{L} comprising the amino acid sequence of SEQ ID NO: 129, or a variant thereof having at least about 90% sequence identity to the amino acid sequence of SEQ ID NO: 129; or (x) a V_{H} comprising the amino acid sequence of SEQ ID NO: 117, or a variant thereof having at least about 90% sequence identity to the amino acid sequence of SEQ ID NO: 117; and a V_{L} comprising the amino acid sequence of SEQ ID NO: 129, or a variant thereof having at least about 90% sequence identity to the amino acid sequence of SEQ ID NO: 129.

In some embodiments, there is provided an isolated anti-CD40 antibody comprising: a V_{H} comprising an HC-CDR1, an HC-CDR2, and an HC-CDR3 of a V_{H} comprising the amino acid sequence of SEQ ID NO: 120; and a V_{L} comprising an LC-CDR1, an LC-CDR2, and an LC-CDR3 of a V_{L} comprising the amino acid sequence of SEQ ID NO: 139.

In some embodiments, there is provided an isolated anti-CD40 antibody comprising: a V_{H} comprising an HC-CDR1 comprising the amino acid sequence of SEQ ID NO: 4, an HC-CDR2 comprising the amino acid sequence of SEQ ID NO: 27, and an HC-CDR3 comprising the amino acid sequence of SEQ ID NO: 53, or a variant thereof comprising up to about 5 amino acid substitutions in the HC-CDRs; and a V_{L} comprising an LC-CDR1 comprising the amino acid sequence of SEQ ID NO: 62, an LC-CDR2 comprising the amino acid sequence of SEQ ID NO: 67, and an LC-CDR3 comprising the amino acid sequence of SEQ ID NO: 78, or a variant thereof comprising up to about 5 amino acid substitutions in the LC-CDRs.

In some embodiments, according to any one of the isolated anti-CD40 antibodies described above, the isolated anti-CD40 antibody comprises: a V_{H} comprising the amino acid sequence of SEQ ID NO: 120, or a variant thereof having at least about 90% sequence identity to the amino acid sequence of SEQ ID NO: 120; and a V_{L} comprising the amino acid sequence of SEQ ID NO: 139, or a variant thereof having at least about 90% sequence identity to the amino acid sequence of SEQ ID NO: 139.

In some embodiments, there is provided an isolated anti-CD40 antibody comprising: a V_{H} comprising an HC-CDR1, an HC-CDR2, and an HC-CDR3 of a V_{H} comprising the amino acid sequence of SEQ ID NO: 121; and a V_{L} comprising an LC-CDR1, an LC-CDR2, and an LC-CDR3 of a V_{L} comprising the amino acid sequence of SEQ ID NO: 140.

In some embodiments, there is provided an isolated anti-CD40 antibody comprising: a V_{H} comprising an HC-CDR1 comprising the amino acid sequence of SEQ ID NO: 5, an HC-CDR2 comprising the amino acid sequence of SEQ ID NO: 27, and an HC-CDR3 comprising the amino acid sequence of SEQ ID NO: 54, or a variant thereof comprising up to about 5 amino acid substitutions in the HC-CDRs; and a V_{L} comprising an LC-CDR1 comprising the amino acid sequence of SEQ ID NO: 62, an LC-CDR2 comprising the amino acid sequence of SEQ ID NO: 67, and an LC-CDR3 comprising the amino acid sequence of SEQ ID NO: 78, or a variant thereof comprising up to about 5 amino acid substitutions in the LC-CDRs.

In some embodiments, according to any one of the isolated anti-CD40 antibodies described above, the isolated anti-CD40 antibody comprises: a V_{H} comprising the amino acid sequence of SEQ ID NO: 121, or a variant thereof having at least about 90% sequence identity to the amino acid sequence of SEQ ID NO: 121; and a V_{L} comprising the amino acid sequence of SEQ ID NO: 140, or a variant thereof having at least about 90% sequence identity to the amino acid sequence of SEQ ID NO: 140.

In some embodiments, there is provided an isolated anti-CD40 antibody comprising: a V_{H} comprising an HC-CDR1, an HC-CDR2, and an HC-CDR3 of a V_{H} comprising the amino acid sequence of SEQ ID NO: 122; and a V_{L} comprising an LC-CDR1, an LC-CDR2, and an LC-CDR3 of a V_{L} comprising the amino acid sequence of SEQ ID NO: 141.

In some embodiments, there is provided an isolated anti-CD40 antibody comprising: a V_{H} comprising an HC-CDR1 comprising the amino acid sequence of SEQ ID NO: 6, an HC-CDR2 comprising the amino acid sequence of SEQ ID NO: 28, and an HC-CDR3 comprising the amino acid sequence of SEQ ID NO: 55, or a variant thereof comprising up to about 5 amino acid substitutions in the HC-CDRs; and a V_{L} comprising an LC-CDR1 comprising the amino acid sequence of SEQ ID NO: 63, an LC-CDR2 comprising the amino acid sequence of SEQ ID NO: 68, and an LC-CDR3 comprising the amino acid sequence of SEQ ID NO: 79, or a variant thereof comprising up to about 5 amino acid substitutions in the LC-CDRs.

In some embodiments, according to any one of the isolated anti-CD40 antibodies described above, the isolated anti-CD40 antibody comprises: a V_{H} comprising the amino acid sequence of SEQ ID NO: 122, or a variant thereof having at least about 90% sequence identity to the amino acid sequence of SEQ ID NO: 122; and a V_{L} comprising the amino acid sequence of SEQ ID NO: 141, or a variant thereof having at least about 90% sequence identity to the amino acid sequence of SEQ ID NO: 141.

In some embodiments, there is provided an isolated anti-CD40 antibody comprising: a V_{H} comprising an HC-CDR1, an HC-CDR2, and an HC-CDR3 of a V_{H} comprising the amino acid sequence of SEQ ID NO: 123; and a V_{L} comprising an LC-CDR1, an LC-CDR2, and an LC-CDR3 of a V_{L} comprising the amino acid sequence of SEQ ID NO: 142.

In some embodiments, there is provided an isolated anti-CD40 antibody comprising: a V_{H} comprising an HC-CDR1 comprising the amino acid sequence of SEQ ID NO: 7, an HC-CDR2 comprising the amino acid sequence of SEQ ID NO: 29, and an HC-CDR3 comprising the amino acid sequence of SEQ ID NO: 56, or a variant thereof comprising up to about 5 amino acid substitutions in the HC-CDRs; and a V_{L} comprising an LC-CDR1 comprising the amino acid sequence of SEQ ID NO: 64, an LC-CDR2 comprising the amino acid sequence of SEQ ID NO: 69, and an LC-CDR3 comprising the amino acid sequence of SEQ ID NO: 80, or a variant thereof comprising up to about 5 amino acid substitutions in the LC-CDRs.

In some embodiments, according to any one of the isolated anti-CD40 antibodies described above, the isolated anti-CD40 antibody comprises: a V_{H} comprising the amino acid sequence of SEQ ID NO: 123, or a variant thereof having at least about 90% sequence identity to the amino acid sequence of SEQ ID NO: 123; and a V_{L} comprising the amino acid sequence of SEQ ID NO: 142, or a variant thereof having at least about 90% sequence identity to the amino acid sequence of SEQ ID NO: 142.

In some embodiments, there is provided an isolated anti-CD40 antibody comprising: a V_{H} comprising an HC-CDR1, an HC-CDR2, and an HC-CDR3 of a V_{H} comprising the amino acid sequence of SEQ ID NO: 124; and a V_{L} comprising an LC-CDR1, an LC-CDR2, and an LC-CDR3 of a V_{L} comprising the amino acid sequence of SEQ ID NO: 143.

In some embodiments, there is provided an isolated anti-CD40 antibody comprising: a V_{H} comprising an HC-CDR1 comprising the amino acid sequence of SEQ ID NO: 8, an HC-CDR2 comprising the amino acid sequence of SEQ ID NO: 30, and an HC-CDR3 comprising the amino acid sequence of SEQ ID NO: 57, or a variant thereof comprising up to about 5 amino acid substitutions in the HC-CDRs; and a V_{L} comprising an LC-CDR1 comprising the amino acid sequence of SEQ ID NO: 62, an LC-CDR2 comprising the amino acid sequence of SEQ ID NO: 67, and an LC-CDR3 comprising the amino acid sequence of SEQ ID NO: 80, or a variant thereof comprising up to about 5 amino acid substitutions in the LC-CDRs.

In some embodiments, according to any one of the isolated anti-CD40 antibodies described above, the isolated anti-CD40 antibody comprises: a V_{H} comprising the amino acid sequence of SEQ ID NO: 124, or a variant thereof having at least about 90% sequence identity to the amino acid sequence of SEQ ID NO: 124; and a V_{L} comprising the amino acid sequence of SEQ ID NO: 143, or a variant thereof having at least about 90% sequence identity to the amino acid sequence of SEQ ID NO: 143.

In some embodiments, there is provided an isolated anti-CD40 antibody comprising: a V_{H} comprising an HC-CDR1, an HC-CDR2, and an HC-CDR3 of a V_{H} comprising the amino acid sequence of SEQ ID NO: 125; and a V_{L} comprising an LC-CDR1, an LC-CDR2, and an LC-CDR3 of a V_{L} comprising the amino acid sequence of SEQ ID NO: 144.

In some embodiments, there is provided an isolated anti-CD40 antibody comprising: a V_{H} comprising an HC-CDR1 comprising the amino acid sequence of SEQ ID NO: 4, an HC-CDR2 comprising the amino acid sequence of SEQ ID NO: 31, and an HC-CDR3 comprising the amino acid sequence of SEQ ID NO: 53, or a variant thereof comprising up to about 5 amino acid substitutions in the HC-CDRs; and a V_{L} comprising an LC-CDR1 comprising the amino acid sequence of SEQ ID NO: 62, an LC-CDR2 comprising the amino acid sequence of SEQ ID NO: 68, and an LC-CDR3 comprising the amino acid sequence of SEQ ID NO: 79, or a variant thereof comprising up to about 5 amino acid substitutions in the LC-CDRs.

In some embodiments, according to any one of the isolated anti-CD40 antibodies described above, the isolated anti-CD40 antibody comprises: a V_{H} comprising the amino acid sequence of SEQ ID NO: 125, or a variant thereof having at least about 90% sequence identity to the amino acid sequence of SEQ ID NO: 125; and a V_{L} comprising the amino acid sequence of SEQ ID NO: 144, or a variant thereof having at least about 90% sequence identity to the amino acid sequence of SEQ ID NO: 144.

In some embodiments, there is provided an isolated anti-CD40 antibody that specifically binds to the human CD40 with a Kd from about 0.1pM to about 1nM.

In some embodiments, there is provided an isolated anti-CD40 antibody that specifically binds to CD40 competitively with any one of the isolated anti-CD40 antibodies described above. In some embodiments, there is provided an isolated anti-CD40 antibody that specifically binds to the same epitope as any one of isolated anti-CD40 antibodies described above.

In some embodiments according to any of the isolated anti-CD40 antibodies described above, the isolated anti-CD40 antibody comprises an Fc fragment. In some embodiments, the isolated anti-CD40 antibody is a full-length IgG antibody. In some embodiments, the isolated anti-CD40 antibody is a full-length IgG1, IgG2, IgG3, or IgG4 antibody. In some embodiments, the anti-CD40 antibody is a chimeric, human, or humanized antibody. In some embodiments, the anti-CD40 antibody is an antigen binding fragment selected from the group consisting of a Fab, a Fab', a F(ab)'₂, a Fab'-SH, a single-chain Fv (scFv), an Fv fragment, a dAb, a Fd, a nanobody, a diabody, and a linear antibody.

In some embodiments, there is provided isolated nucleic acid molecule(s) that encodes any one of the anti-CD40 antibodies described above. In some embodiments, there is provided a vector comprising any one of the nucleic acid molecules described above. In some embodiments, there is provided a host cell comprising any one of the anti-CD40 antibodies described above, any one of the nucleic acid molecules described above, or any one of the vectors described above. In some embodiments, there is provided a method of producing an anti-CD40 antibody, comprising: a) culturing any one of the host cells described above under conditions effective to express the anti-CD40 antibody; and b) obtaining the expressed anti-CD40 antibody from the host cell.

In some embodiments, there is provided a method of treating a disease or condition in an individual in need thereof, comprising administering to the individual an effective amount of any one of the anti-CD40 antibodies described above. In some embodiments, there is provided the use of any one of the anti-CD40 antibodies described herein for the preparation of pharmaceutical compositions for treating a disease or condition in an individual in need. In some embodiments, provided is the use of any one of the anti-CD40 antibodies described above, or a pharmaceutical composition comprising any one of anti-CD40 antibodies described above in the manufacture of a medicament for treating a disease or condition. In some embodiments, the disease or condition is associated with CD40, comprising autoimmune disease and/or inflammatory disease and/or cancer and/or transplantation-related disease or condition. In some embodiments, the disease or condition is selected from the group consisting of Systemic Lupus Erythematosus (SLE), discoid lupus, lupus nephritis, inflammatory arthritis: including but not limited to juvenile arthritis, rheumatoid arthritis, psoriatic arthritis, Reiter's syndrome, ankylosing spondylitis and gouty arthritis, organ or tissue transplant rejection: hyperacute, acute or chronic rejection and/or graftversus-host disease, multiple sclerosis, hyper IgE syndrome, polyarteritis nodosa, primary biliary cirrhosis, inflammatory bowel disease, Crohn's disease, celiac disease (gluten-sensitive bowel disease), autoimmune hepatitis, pernicious anemia, autoimmune hemolytic anemia, scleroderma, myasthenia gravis, idiopathic thrombocytopenic purpura, autoimmune thyroiditis, Grave's disease, Hasimoto's thyroiditis, immune complex diseases, Chronic Fatigue Immune Dysfunction Syndrome (CFIDS), polymyositis, dermatomyositis, cryoglobulinemia, thrombolysis, cardiomyopathy, pemphigus vulgaris, interstitial pulmonary fibrosis, type I and type II diabetes, type 1, 2, 3 and 4 delayed-type hypersensitivity, allergic or allergic disorders, unwanted/unintended immune reactions to therapeutic proteins(e.g., factor VII in hemophilia), asthma, Rich-Strauss syndrome (allergic granulomatosis), atopic dermatitis, allergic and irritant contact dermatitis, urticaria, IgE-mediated allergy, atherosclerosis, vasculitis, idiopathic inflammatory myopathy, hemolytic diseases, Alzheimer's disease, chronic inflammatory demyelinating polyneuropathy, Sjogren's disease, psoriasis, hidradenitis suppurativa, suppurative urethritis, sarcoidosis, ulcerative colitis, cancer: including but not limited to Non-Hodgkins lymphoma, chronic lymphocytic leukemia, multiple myeloma, B-cell lymphoma, high-level/mid-level/lower B-cell lymphoma, acute B-cell lymphoblastic leukemia, myeloblastic leukemia and Hodgkin's disease, nephritis, respiratory distress syndrome, epididymitis, glomerulonephritis, hyperthyroidism.

Also provided are pharmaceutical compositions, kits and articles of manufacture comprising any one of the anti-CD40 antibodies described above.

### BRIEF DESCRIPTION OF THE DRAWINGS

FIG.1A shows the anti-CD40 antibody D1, D6 or D7 inhibited human CD40L-induced CD95 expression in Ramos cells as compared to the reference antibody ASKP1240. FIG.1B shows the anti-CD40 antibody D1, D7 or the reference antibody ASKP1240 had no side effects of activating CD40 pathway after cross-linking with anti-IgG antibody, but the reference antibody CFZ533 showed certain agonistic activity.
FIG.2 shows the anti-CD40 antibody D1, D6 or D7 inhibited CD40L-induced CD23 expression in PBMCs as compared to the reference antibody ASKP1240.
FIGS. 3A-3B show the anti-CD40 antibody D1, D6 or D7 inhibited CD40L-induced cytokine secretion in iDCs as compared to the reference antibody ASKP1240. FIG.3A shows the anti-CD40 antibody D1, D6 or D7 inhibited CD40L-induced IL12p40 secretion in iDCs. FIG.3B shows the anti-CD40 antibody D1, D6 or D7 inhibited CD40L-induced ILTNF-α secretion in iDCs.
FIG.4 shows the optimized anti-CD40 antibodies D21, D26, D33, D42, D47, D49 inhibited CD40L-induced NF-κB Activation as compared to the reference antibody ASKP1240.
FIGS. 5A-5B show the binding affinity of optimized anti-CD40 antibodies with CD40 of different species. FIG.5A shows the binding curves of the optimized anti-CD40 antibodies D21, D26, D33, D42, D47, D49 with human CD40. FIG.5B shows the binding curves of the optimized anti-CD40 antibodies D21, D26, D33, D42, D47, D49 with rhesus monkey CD40.
FIG.6 shows the optimized anti-CD40 antibodies D21, D26, D33, D42, D47, D49 inhibited human CD40L-induced CD95 expression in Ramos cells as compared to the reference antibody ASKP1240.
FIG.7 shows the optimized anti-CD40 antibodies D33, D42, D47 inhibited CD40L-induced CD23 expression in PBMCs as compared to the reference antibody ASKP1240.

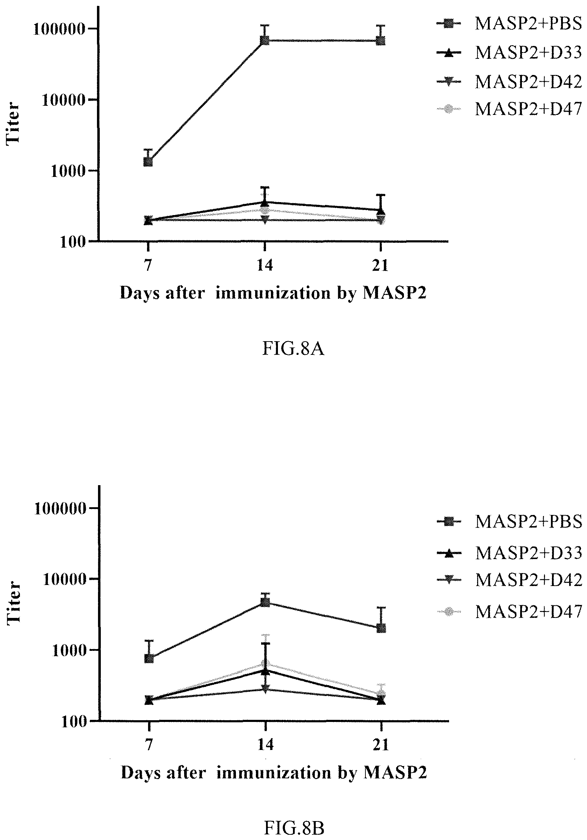

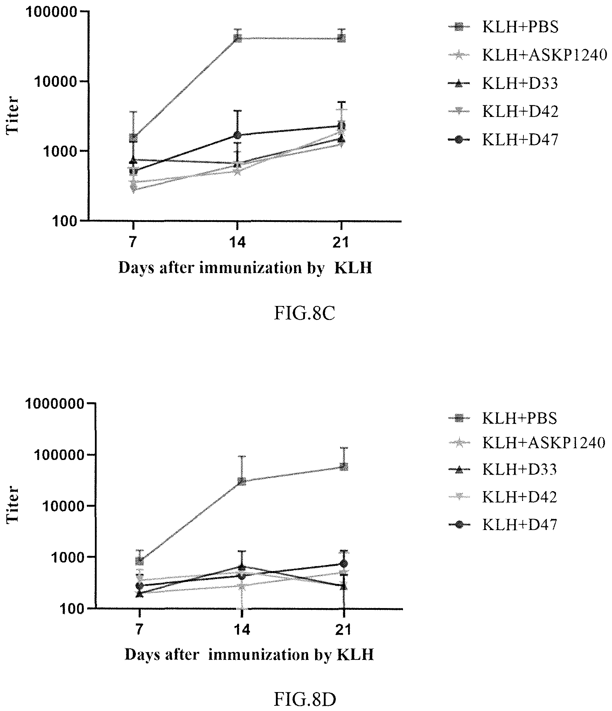

FIGS. 8A-8D show the blocking effect of the anti-CD40 antibody on T-cell dependent antibody response as compared to the reference antibody ASKP1240. FIG.8A shows the optimized anti-CD40 antibodies D33, D42 or D47 nearly completely blocked the generation of IgG specific to MASP2 antigen at a dose of 3mpk. FIG.8B shows the optimized anti-CD40 antibodies D33, D42 or D47 nearly completely blocked the generation of IgM specific to MASP2 antigen at a dose of 3mpk. FIG.8D shows the optimized anti-CD40 antibodies D33, D42 or D47 nearly completely blocked the generation of IgM specific to KLH antigen at a dose of 1mpk.
FIG.9 shows the drug serum concentrations curves in Rats in vivo of the optimized anti-CD40 antibodies D33, D42 or D47 as compared to the reference antibody ASKP1240.

### DETAILED DESCRIPTION OF THE APPLICATION

The present application in one aspect provides an isolated anti-CD40 antibody. By using a combination of selections on naïve scFv phage libraries, humanization of antibody, affinity maturation and appropriately designed biochemical and biological assays, we have identified highly potent antibody molecules that bind to human CD40 and inhibit the action of human CD40 to its receptor. The results presented he.rein indicate that the present application antibodies bind human and/or Rhesus monkey CD40 with high affinity, compared with the known anti-CD40 antibody ASKP1240, and surprisingly are even more potent than ASKP1240 as demonstrated in a variety of biological assays.

The anti-CD40 antibodies provided by the present application include, for example, full-length anti-CD40 antibodies, anti-CD40 scFvs, anti-CD40 Fc fusion proteins, multi-specific (such as bispecific) anti-CD40 antibodies, anti-CD40 immunoconjugates, and the like.

In some embodiments, the isolated anti-CD40 antibody comprises: a heavy chain variable domain (V_{H}) comprising a heavy chain complementarity determining region (HC-CDR) 1 comprising GYX₁VN (SEQ ID NO: 81), wherein X₁ is L, T, or V; an HC-CDR2 comprising X₁IX₂PX₃X₄GX₅TX₆YNQKFKG (SEQ ID NO: 82), wherein X₁ is L or M, X₂ is A, N, or V, X₃ is E, K, L, M, R, S, T, or Y, X₄ is H, N, S, or T, X₅ is G or R, and X₆ is A, S, T, or W; and an HC-CDR3 comprising SX₁X₂X₃X₄PYAX₅DY (SEQ ID NO: 83), wherein X₁ is G, I, L, M, P, R, S, or Y, X₂ is W or Y, X₃ is S or V, X₄ is D, K, L, N, R, T, or V, and X₅ is A, G, M, N, Q, or S; and a light chain variable domain (V_{L}) comprising a light chain complementarity determining region (LC-CDR) 1 comprising KASQNVHTX₁VA (SEQ ID NO: 84), wherein X₁ is A, H, N, or R; an LC-CDR2 comprising LX₁SDRRT (SEQ ID NO: 85), wherein X₁ is A or R; and an LC-CDR3 comprising LQX₁WX₂X₃PLT (SEQ ID NO: 86), wherein X₁ is A or H, X₂ is N, S, or T, and X₃ is A, Q, R, S, or Y.

In some embodiments, the isolated anti-CD40 antibody comprises: a heavy chain variable domain (V_{H}) comprising a heavy chain complementarity determining region (HC-CDR) 1 comprising DAYMH (SEQ ID NO: 4); an HC-CDR2 comprising RIDPANGNTKYDPKFQG (SEQ ID NO: 27); and an HC-CDR3 comprising NYYGSRYPFAY (SEQ ID NO: 53); and a light chain variable domain (V_{L}) comprising a light chain complementarity determining region (LC-CDR) 1 comprising SASSSVSYMH (SEQ ID NO: 62); an LC-CDR2 comprising STSNLAS (SEQ ID NO: 67); and an LC-CDR3 comprising QQGSSYPLT (SEQ ID NO: 78).

In some embodiments, the isolated anti-CD40 antibody comprises: a heavy chain variable domain (V_{H}) comprising a heavy chain complementarity determining region (HC-CDR) 1 comprising DSYMH (SEQ ID NO: 5); an HC-CDR2 comprising RIDPANGNTKYDPKFQG (SEQ ID NO: 27); and an HC-CDR3 comprising NYYGAAYPFAY (SEQ ID NO: 54); and a light chain variable domain (V_{L}) comprising a light chain complementarity determining region (LC-CDR) 1 comprising SASSSVSYMH (SEQ ID NO: 62); an LC-CDR2 comprising STSNLAS (SEQ ID NO: 67); and an LC-CDR3 comprising QQGSSYPLT (SEQ ID NO: 78).

In some embodiments, the isolated anti-CD40 antibody comprises: a heavy chain variable domain (V_{H}) comprising a heavy chain complementarity determining region (HC-CDR) 1 comprising DSYIH (SEQ ID NO: 6); an HC-CDR2 comprising RIDPTNGNTNFDPKFRG (SEQ ID NO: 28); and an HC-CDR3 comprising NYYGSRYPFSY (SEQ ID NO: 55); and a light chain variable domain (V_{L}) comprising a light chain complementarity determining region (LC-CDR) 1 comprising SGSSSVSYMH (SEQ ID NO: 63); an LC-CDR2 comprising FTSNLAS (SEQ ID NO: 68); and an LC-CDR3 comprising QQGSTYPLT (SEQ ID NO: 79).

In some embodiments, the isolated anti-CD40 antibody comprises: a heavy chain variable domain (V_{H}) comprising a heavy chain complementarity determining region (HC-CDR) 1 comprising DHGMH (SEQ ID NO: 7); an HC-CDR2 comprising VISTSYGVATYNQKFKG (SEQ ID NO: 29); and an HC-CDR3 comprising RDSKSPFDY (SEQ ID NO: 56); and a light chain variable domain (V_{L}) comprising a light chain complementarity determining region (LC-CDR) 1 comprising SASSSISSNYLH (SEQ ID NO: 64); an LC-CDR2 comprising RTSNLAS (SEQ ID NO: 69); and an LC-CDR3 comprising HQYHRSPLT (SEQ ID NO: 80).

In some embodiments, the isolated anti-CD40 antibody comprises: a heavy chain variable domain (V_{H}) comprising a heavy chain complementarity determining region (HC-CDR) 1 comprising DHAIH (SEQ ID NO: 8); an HC-CDR2 comprising AISTNYGAATYNQKFKG (SEQ ID NO: 30); and an HC-CDR3 comprising RGSHGDWFAY (SEQ ID NO: 57); and a light chain variable domain (V_{L}) comprising a light chain complementarity determining region (LC-CDR) 1 comprising SASSSVSYMH (SEQ ID NO: 62); an LC-CDR2 comprising STSNLAS (SEQ ID NO: 67); and an LC-CDR3 comprising HQYHRSPLT (SEQ ID NO: 80).

In some embodiments, the isolated anti-CD40 antibody comprises: a heavy chain variable domain (V_{H}) comprising a heavy chain complementarity determining region (HC-CDR) 1 comprising DAYMH (SEQ ID NO: 4); an HC-CDR2 comprising RIDPANGYTKSDPKFQG (SEQ ID NO: 31); and an HC-CDR3 comprising NYYGSRYPFAY (SEQ ID NO: 53); and a light chain variable domain (V_{L}) comprising a light chain complementarity determining region (LC-CDR) 1 comprising SASSSVSYMH (SEQ ID NO: 62); an LC-CDR2 comprising FTSNLAS (SEQ ID NO: 68); and an LC-CDR3 comprising QQGSTYPLT (SEQ ID NO: 79).

Also provided are nucleic acids encoding the anti-CD40 antibodies, compositions comprising the anti-CD40 antibodies, and methods of making and using the anti-CD40 antibodies.

### Definitions

As used herein, "treatment" or "treating" is an approach for obtaining beneficial or desired results, including clinical results. For purposes of this application, beneficial or desired clinical results include, but are not limited to, one or more of the following: alleviating one or more symptoms resulting from the disease, diminishing the extent of the disease, stabilizing the disease (e.g., preventing or delaying the worsening of the disease), preventing or delaying the spread (*e.g.*, metastasis) of the disease, preventing or delaying the recurrence of the disease, delaying or slowing the progression of the disease, ameliorating the disease state, providing a remission (partial or total) of the disease, decreasing the dose of one or more of other medications required to treat the disease, delaying the progression of the disease, increasing or improving the quality of life, increasing weight gain, and/or prolonging survival. Also encompassed by "treatment" is a reduction of pathological consequence of the disease (such as, for example, tumor volume for cancer). The methods of the application contemplate any one or more of these aspects of treatment.

The term "antibody" includes full-length antibodies and antigen-binding fragments thereof. A full-length antibody comprises two heavy chains and two light chains. The variable regions of the light and heavy chains are responsible for antigen binding. The variable regions in both chains generally contain three highly variable loops called the complementarity determining regions (CDRs) (light chain (LC) CDRs including LC-CDR1, LC-CDR2, and LC-CDR3, heavy chain (HC) CDRs including HC-CDR1, HC-CDR2, and HC-CDR3). CDR boundaries for the antibodies and antigen-binding fragments disclosed herein may be defined or identified by the conventions of Kabat, Chothia, or Al-Lazikani (Al-Lazikani 1997; Chothia 1985; Chothia 1987; Chothia 1989; Kabat 1987; Kabat 1991). The three CDRs of the heavy or light chains are interposed between flanking stretches known as framework regions (FRs), which are more highly conserved than the CDRs and form a scaffold to support the hypervariable loops. The constant regions of the heavy and light chains are not involved in antigen binding, but exhibit various effector functions. Antibodies are assigned to classes based on the amino acid sequence of the constant region of their heavy chain. The five major classes or isotypes of antibodies are IgA, IgD, IgE, IgG, and IgM, which are characterized by the presence of α, δ, ε, γ, and µ heavy chains, respectively. Several of the major antibody classes are divided into subclasses such as IgG1 (γ1 heavy chain), IgG2 (γ2 heavy chain), IgG3 (y3 heavy chain), IgG4 (γ4 heavy chain), IgA1 (α1 heavy chain), or IgA2 (α2 heavy chain).

The term "antigen-binding fragment" as used herein includes an antibody fragment including, for example, a diabody, a Fab, a Fab', a F(ab')₂, an Fv fragment, a disulfide stabilized Fv fragment (dsFv), a (dsFv)₂, a bispecific dsFv (dsFv-dsFv'), a disulfide stabilized diabody (ds diabody), a single-chain Fv (scFv), an scFv dimer (bivalent diabody), a multispecific antibody formed from a portion of an antibody comprising one or more CDRs, a single domain antibody, a nanobody, a domain antibody, a bivalent domain antibody, or any other antibody fragments that bind to an antigen but do not comprise a complete antibody structure. An antigen-binding fragment also includes a fusion protein comprising the antibody fragment described above. An antigen-binding fragment is capable of binding to the same antigen to which the parent antibody or a parent antibody fragment (*e.g*., a parent scFv) binds. In some embodiments, an antigen-binding fragment may comprise one or more CDRs from a particular human antibody grafted to a framework region from one or more different human antibodies.

The term "epitope" as used herein refers to the specific group of atoms or amino acids on an antigen to which an antibody or antibody moiety binds. Two antibodies or antibody moieties may bind the same epitope within an antigen if they exhibit competitive binding for the antigen.

As used herein, a first antibody "competes" for binding to a target CD40 with a second antibody when the first antibody inhibits target CD40 binding of the second antibody by at least about 50% (such as at least about any of 55%, 60%, 65%, 70%, 75%, 80%, 85%, 90%, 95%, 98% or 99%) in the presence of an equimolar concentration of the first antibody, or *vice versa.* A high throughput process for "binning" antibodies based upon their cross-competition is described in PCT Publication No. WO 03/48731.

As used herein, the term "specifically binds", "specifically recognizing", or "is specific for" refers to measurable and reproducible interactions, such as binding between a target and an antibody that is determinative of the presence of the target in the presence of a heterogeneous population of molecules, including biological molecules. For example, an antibody that specifically recognizes a target (which can be an epitope) is an antibody that binds to this target with greater affinity, avidity, more readily, and/or with greater duration than its binding to other targets. In some embodiments, an antibody that specifically recognizes an antigen reacts with one or more antigenic determinants of the antigen with a binding affinity that is at least about 10 times its binding affinity for other targets.

An "isolated" anti-CD40 antibody as used herein refers to an anti-CD40 antibody that (1) is not associated with proteins found in nature, (2) is free of other proteins from the same source, (3) is expressed by a cell from a different species, or, (4) does not occur in nature.

The term "isolated nucleic acid" as used herein is intended to mean a nucleic acid of genomic, cDNA, or synthetic origin or some combination thereof, which by virtue of its origin the "isolated nucleic acid" (1) is not associated with all or a portion of a polynucleotide in which the "isolated nucleic acid" is found in nature, (2) is operably linked to a polynucleotide which it is not linked to in nature, or (3) does not occur in nature as part of a larger sequence.

As used herein, the term "CDR" or "complementarity determining region" is intended to mean the non-contiguous antigen combining sites found within the variable region of both heavy and light chain polypeptides. These particular regions have been described by Kabat et al., J. Biol. Chem. 252:6609-6616 (1977); Kabat et al., U.S. Dept. of Health and Human Services, "Sequences of proteins of immunological interest" (1991); Chothia et al., J. Mol. Biol. 196:901-917 (1987); Al-Lazikani B. et al., J. Mol. Biol., 273: 927-948 (1997); MacCallum et al., J. Mol. Biol. 262:732-745 (1996); Abhinandan and Martin, Mol. Immunol., 45: 3832-3839 (2008); Lefranc M.P. et al., Dev. Comp. Immunol., 27: 55-77 (2003); and Honegger and Plückthun, J. Mol. Biol., 309:657-670 (2001), where the definitions include overlapping or subsets of amino acid residues when compared against each other. Nevertheless, application of either definition to refer to a CDR of an antibody or grafted antibodies or variants thereof is intended to be within the scope of the term as defined and used herein. The amino acid residues which encompass the CDRs as defined by each of the above cited references are set forth below in Table 1 as a comparison. CDR prediction algorithms and interfaces are known in the art, including, for example, Abhinandan and Martin, Mol. Immunol., 45: 3832-3839 (2008); Ehrenmann F. et al., Nucleic Acids Res., 38: D301-D307 (2010); and Adolf-Bryfogle J. et al., Nucleic Acids Res., 43: D432-D438 (2015). The contents of the references cited in this paragraph are incorporated herein by reference in their entireties for use in the present application and for possible inclusion in one or more claims herein.

**TABLE 1: CDR DEFINITIONS**

| | **Kabat¹** | **Chothia²** | **MacCallum³** | **IMGT⁴** | **AHo⁵** |
|---|---|---|---|---|---|
| V_{H} CDR1 | 31-35 | 26-32 | 30-35 | 27-38 | 25-40 |
| V_{H} CDR2 | 50-65 | 53-55 | 47-58 | 56-65 | 58-77 |
| V_{H} CDR3 | 95-102 | 96-101 | 93-101 | 105-117 | 109-137 |
| V_{L} CDR1 | 24-34 | 26-32 | 30-36 | 27-38 | 25-40 |
| V_{L} CDR2 | 50-56 | 50-52 | 46-55 | 56-65 | 58-77 |
| V_{L} CDR3 | 89-97 | 91-96 | 89-96 | 105-117 | 109-137 |

| | | | | | |
|---|---|---|---|---|---|
| ¹Residue numbering follows the nomenclature of Kabat *et al., supra* ²Residue numbering follows the nomenclature of Chothia *et al., supra* ³Residue numbering follows the nomenclature of MacCallum *et al., supra* ⁴Residue numbering follows the nomenclature of Lefranc *et al., supra* ⁵Residue numbering follows the nomenclature of Honegger and Plückthun, *supra* | | | | | |

The term "chimeric antibody" refers to an antibody in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit a biological activity of this application (*see* U.S. Patent No. 4,816,567; and Morrison et al., Proc. Natl. Acad. Sci. USA, 81:6851-6855 (1984)).

"Fv" is the minimum antibody fragment which contains a complete antigen-recognition and binding site. This fragment consists of a dimer of one heavy- and one light-chain variable region domain in tight, non-covalent association. From the folding of these two domains emanate six hypervariable loops (3 loops each from the heavy and light chain) that contribute the amino acid residues for antigen binding and confer antigen binding specificity to the antibody. However, even a single variable domain (or half of an Fv comprising only three CDRs specific for an antigen) has the ability to recognize and bind antigen, although at a lower affinity than the entire binding site.

"Single-chain Fv", also abbreviated as "sFv" or "scFv", are antibody fragments that comprise the V_{H} and V_{L} antibody domains connected into a single polypeptide chain. In some embodiments, the scFv polypeptide further comprises a polypeptide linker between the V_{H} and V_{L} domains which enables the scFv to form the desired structure for antigen binding. For a review of scFv, *see* Pluckthun in The Pharmacology of Monoclonal Antibodies, vol. 113, Rosenburg and Moore eds., Springer-Verlag, New York, pp. 269-315 (1994).

The term "diabodies" refers to small antibody fragments prepared by constructing scFv fragments (see preceding paragraph) typically with short linkers (such as about 5 to about 10 residues) between the V_{H} and V_{L} domains such that inter-chain but not intra-chain pairing of the V domains is achieved, resulting in a bivalent fragment, *i.e.,* fragment having two antigen-binding sites. Bispecific diabodies are heterodimers of two "crossover" scFv fragments in which the V_{H} and V_{L} domains of the two antibodies are present on different polypeptide chains. Diabodies are described more fully in, for example, EP 404,097; WO 93/11161; and Hollinger et al., Proc. Natl. Acad. Sci. USA, 90:6444-6448 (1993).

"Humanized" forms of non-human (*e.g*., rodent) antibodies are chimeric antibodies that contain minimal sequence derived from the non-human antibody. For the most part, humanized antibodies are human immunoglobulins (recipient antibody) in which residues from a hypervariable region (HVR) of the recipient are replaced by residues from a hypervariable region of a non-human species (donor antibody) such as mouse, rat, rabbit or non-human primate having the desired antibody specificity, affinity, and capability. In some instances, framework region (FR) residues of the human immunoglobulin are replaced by corresponding non-human residues. Furthermore, humanized antibodies can comprise residues that are not found in the recipient antibody or in the donor antibody. These modifications are made to further refine antibody performance. In general, the humanized antibody will comprise substantially at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, *see* Jones et al., Nature 321 :522-525 (1986); Riechmann et al., Nature 332:323-329 (1988); and Presta, Curr. Op. Struct. Biol. 2:593-596 (1992).

"Percent (%) amino acid sequence identity" or "homology" with respect to the polypeptide and antibody sequences identified herein is defined as the percentage of amino acid residues in a candidate sequence that are identical with the amino acid residues in the polypeptide being compared, after aligning the sequences considering any conservative substitutions as part of the sequence identity. Alignment for purposes of determining percent amino acid sequence identity can be achieved in various ways that are within the skilled in the art, for instance, using publicly available computer software such as BLAST, BLAST-2, ALIGN, Megalign (DNASTAR), or MUSCLE software. Those skilled in the art can determine appropriate parameters for measuring alignment, including any algorithms needed to achieve maximal alignment over the full-length of the sequences being compared. For purposes herein, however, % amino acid sequence identity values are generated using the sequence comparison computer program MUSCLE (Edgar, R.C., Nucleic Acids Research 32(5):1792-1797, 2004; Edgar, R.C., BMC Bioinformatics 5(1): 113, 2004).

The terms "Fc receptor" or "FcR" are used to describe a receptor that binds to the Fc region of an antibody. In some embodiments, an FcR of this application is one that binds to an IgG antibody (a γ receptor) and includes receptors of the FcyRI, FcyRII, and FcγRIII subclasses, including allelic variants and alternatively spliced forms of these receptors. FcyRII receptors include FcyRIIA (an "activating receptor") and FcγRIIB (an "inhibiting receptor"), which have similar amino acid sequences that differ primarily in the cytoplasmic domains thereof. Activating receptor FcyRIIA contains an immunoreceptor tyrosine-based activation motif (ITAM) in its cytoplasmic domain. Inhibiting receptor FcyRIIB contains an immunoreceptor tyrosine-based inhibition motif (ITIM) in its cytoplasmic domain (see review M. in Daëron, Annu. Rev. Immunol. 15:203-234 (1997)). The term includes allotypes, such as FcyRIIIA allotypes: FcγRIIIA-Phe158, FcyRIIIA-Va1158, FcyRIIA-R131 and/or FcγRIIA-H131. FcRs are reviewed in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976) and Kim et al., J. Immunol. 24:249 (1994)).

The term "FcRn" refers to the neonatal Fc receptor (FcRn). FcRn is structurally similar to major histocompatibility complex (MHC) and consists of an α-chain noncovalently bound to β2-microglobulin. The multiple functions of the neonatal Fc receptor FcRn are reviewed in Ghetie and Ward (2000) Annu. Rev. Immunol. 18, 739-766. FcRn plays a role in the passive delivery of immunoglobulin IgGs from mother to young and the regulation of serum IgG levels. FcRn can act as a salvage receptor, binding and transporting pinocytosed IgGs in intact form both within and across cells, and rescuing them from a default degradative pathway.

The "CH1 domain" of a human IgG Fc region usually extends from about amino acid 118 to about amino acid 215 (EU numbering system).

"Hinge region" is generally defined as stretching from Glu216 to Pro230 of human IgG1 (Burton, Molec. Immunol.22:161-206 (1985)). Hinge regions of other IgG isotypes may be aligned with the IgG1 sequence by placing the first and last cysteine residues forming inter-heavy chain S-S bonds in the same positions.

The "CH2 domain" of a human IgG Fc region usually extends from about amino acid 231 to about amino acid 340. The CH2 domain is unique in that it is not closely paired with another domain. Rather, two N-linked branched carbohydrate chains are interposed between the two CH2 domains of an intact native IgG molecule. It has been speculated that the carbohydrate may provide a substitute for the domaindomain pairing and help stabilize the CH2 domain. Burton, Molec Immunol. 22:161-206 (1985).

The "CH3 domain" comprises the stretch of residues of C-terminal to a CH2 domain in an Fc region (*i.e.* from about amino acid residue 341 to the C-terminal end of an antibody sequence, typically at amino acid residue 446 or 447 of an IgG).

A "functional Fc fragment" possesses an "effector function" of a native sequence Fc region. Exemplary "effector functions" include C1q binding; complement dependent cytotoxicity (CDC); Fc receptor binding; antibody-dependent cell-mediated cytotoxicity (ADCC); phagocytosis; down regulation of cell surface receptors (*e.g*., B cell receptor; BCR), etc. Such effector functions generally require the Fc region to be combined with a binding domain (*e.g.*, an antibody variable domain) and can be assessed using various assays known in the art.

An antibody with a variant IgG Fc with "altered" FcR binding affinity or ADCC activity is one which has either enhanced or diminished FcR binding activity (*e.g*., FcyR or FeRn) and/or ADCC activity compared to a parent polypeptide or to a polypeptide comprising a native sequence Fc region. The variant Fc which "exhibits increased binding" to an FcR binds at least one FcR with higher affinity (*e.g*., lower apparent Kd or IC₅₀ value) than the parent polypeptide or a native sequence IgG Fc. According to some embodiments, the improvement in binding compared to a parent polypeptide is about 3-fold, such as about any of 5, 10, 25, 50, 60, 100, 150, 200, or up to 500-fold, or about 25% to 1000% improvement in binding. The polypeptide variant which "exhibits decreased binding" to an FcR, binds at least one FcR with lower affinity (*e.g*., higher apparent Kd or IC₅₀ value) than a parent polypeptide. The decrease in binding compared to a parent polypeptide may be about 40% or more decrease in binding.

"Antibody-dependent cell-mediated cytotoxicity" or "ADCC" refers to a form of cytotoxicity in which secreted Ig bound to Fc receptors (FcRs) present on certain cytotoxic cells (*e.g*., Natural Killer (NK) cells, neutrophils, and macrophages) enable these cytotoxic effector cells to bind specifically to an antigenbearing target cell and subsequently kill the target cell with cytotoxins. The antibodies "arm" the cytotoxic cells and are required for such killing. The primary cells for mediating ADCC, NK cells, express FcγRIII only, whereas monocytes express FcyRI, FcγRII and FcγRIII. FcR expression on hematopoietic cells is summarized in Table 3 on page 464 of Ravetch and Kinet, Annu. Rev. Immunol 9:457-92 (1991). To assess ADCC activity of a molecule of interest, an *in vitro* ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes etal. PNAS (USA) 95:652-656 (1998).

The polypeptide comprising a variant Fc region which "exhibits increased ADCC" or mediates ADCC in the presence of human effector cells more effectively than a polypeptide having wild type IgG Fc or a parent polypeptide is one which *in vitro* or *in vivo* is substantially more effective at mediating ADCC, when the amounts of polypeptide with variant Fc region and the polypeptide with wild type Fc region (or the parent polypeptide) in the assay are essentially the same. Generally, such variants will be identified using any *in vitro* ADCC assay known in the art, such as assays or methods for determining ADCC activity, *e.g.,* in an animal model etc. In some embodiments, the variant is from about 5-fold to about 100-fold, *e.g.* from about 25 to about 50-fold, more effective at mediating ADCC than the wild type Fc (or parent polypeptide).

"Complement dependent cytotoxicity" or "CDC" refers to the lysis of a target cell in the presence of complement. Activation of the classical complement pathway is initiated by the binding of the first component of the complement system (C1q) to antibodies (of the appropriate subclass) which are bound to their cognate antigen. To assess complement activation, a CDC assay, *e.g.* as described in Gazzano-Santoro et al., J. Immunol. Methods 202:163 (1996), may be performed. Polypeptide variants with altered Fc region amino acid sequences and increased or decreased C1q binding capability are described in US patent No. 6,194,551B1 and WO99/51642. The contents of those patent publications are specifically incorporated herein by reference. *See* also, Idusogie et al. J. Immunol. 164: 4178-4184 (2000).

Unless otherwise specified, a "nucleotide sequence encoding an amino acid sequence" includes all nucleotide sequences that are degenerate versions of each other and that encode the same amino acid sequence. The phrase nucleotide sequence that encodes a protein or a RNA may also include introns to the extent that the nucleotide sequence encoding the protein may in some version contain an intron(s).

The term "operably linked" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein coding regions, in the same reading frame.

"Homologous" refers to the sequence similarity or sequence identity between two polypeptides or between two nucleic acid molecules. When a position in both of the two compared sequences is occupied by the same base or amino acid monomer subunit, *e.g*., if a position in each of two DNA molecules is occupied by adenine, then the molecules are homologous at that position. The percent of homology between two sequences is a function of the number of matching or homologous positions shared by the two sequences divided by the number of positions compared times 100. For example, if 6 of 10 of the positions in two sequences are matched or homologous then the two sequences are 60% homologous. By way of example, the DNA sequences ATTGCC and TATGGC share 50% homology. Generally, a comparison is made when two sequences are aligned to give maximum homology.

An "effective amount" of an anti-CD40 antibody or composition as disclosed herein, is an amount sufficient to carry out a specifically stated purpose. An "effective amount" can be determined empirically and by known methods relating to the stated purpose.

The term "therapeutically effective amount" refers to an amount of an anti-CD40 antibody or composition as disclosed herein, effective to "treat" a disease or disorder in an individual. As used herein, the term "effective amount" refers to a sufficient degree of severity and/or duration of treatment to reduce or ameliorate a disorder or one or more symptoms thereof; preventing the progression of the disorder; causing regression of the condition; preventing the recurrence, development, onset, or progression of one or more symptoms associated with the disorder; detecting a condition; or an amount that enhances or improves the prophylactic or therapeutic effect of another therapy (e.g., prophylactic or therapeutic agent)

As used herein, by "pharmaceutically acceptable" or "pharmacologically compatible" is meant a material that is not biological or otherwise undesirable, *e.g*., the material may be incorporated into a pharmaceutical composition administered to a patient without causing any significant undesirable biological effects or interacting in a deleterious manner with any of the other components of the composition in which it is contained. Pharmaceutically acceptable carriers or excipients have preferably met the required standards of toxicological and manufacturing testing and/or are included on the Inactive Ingredient Guide prepared by the U.S. Food and Drug Administration.

It is understood that embodiments of the application described herein include "consisting of" and/or "consisting essentially of" embodiments.

Reference to "about" a value or parameter herein includes (and describes) variations that are directed to that value or parameter per se. For example, description referring to "about X" includes description of "X".

As used herein, reference to "not" a value or parameter generally means and describes "other than" a value or parameter. For example, the method is not used to treat cancer of type X means the method is used to treat cancer of types other than X.

As used herein and in the appended claims, the singular forms "a", "an", and "the" include plural referents unless the context clearly dictates otherwise.

### Anti-CD40 antibodies

In one aspect, the present application provides anti-CD40 antibodies that specifically bind to CD40. Anti-CD40 antibodies include, but are not limited to, humanized antibodies, chimeric antibodies, mouse antibodies, human antibodies, and antibodies comprising the heavy chain and/or light chain CDRs discussed herein. In one aspect, the present application provides isolated antibodies that bind to CD40. Contemplated anti-CD40 antibodies include, for example, full-length anti-CD40 antibodies (*e.g*., full-length IgG1 or IgG4), anti-CD40 scFvs, anti-CD40 Fc fusion proteins, multi-specific (such as bispecific) anti-CD40 antibodies, anti-CD40 immunoconjugates, and the like. In some embodiments, the anti-CD40 antibody is a full-length antibody (*e.g*., full-length IgG1 or IgG4) or antigen-binding fragment thereof, which specifically binds to CD40. In some embodiments, the anti-CD40 antibody is a Fab, a Fab', a F(ab)'₂, a Fab'-SH, a single-chain Fv (scFv), an Fv fragment, a dAb, a Fd,a nanobody, a diabody, or a linear antibody. In some embodiments, reference to an antibody that specifically binds to CD40 means that the antibody binds to CD40 with an affinity that is at least about 10 times (including for example at least about any one of 10, 10², 10³, 10⁴, 10⁵, 10⁶, or 10⁷ times) more tightly than its binding affinity for a non-target. In some embodiments, the non-target is an antigen that is not CD40. Binding affinity can be determined by methods known in the art, such as ELISA, fluorescence activated cell sorting (FACS) analysis, or radioimmunoprecipitation assay (RIA). Kd can be determined by methods known in the art, such as surface plasmon resonance (SPR) assay or biolayer interferometry (BLI).

Although anti-CD40 antibodies containing human sequences (*e.g.*, human heavy and light chain variable domain sequences comprising human CDR sequences) are extensively discussed herein, non-human anti-CD40 antibodies are also contemplated. In some embodiments, non-human anti-CD40 antibodies comprise human CDR sequences from an anti-CD40 antibody as described herein and non-human framework sequences. Non-human framework sequences include, in some embodiments, any sequence that can be used for generating synthetic heavy and/or light chain variable domains using one or more human CDR sequences as described herein, including, *e.g.,* mammals, *e.g.,* mouse, rat, rabbit, pig, bovine (*e.g.*, cow, bull, buffalo), deer, sheep, goat, chicken, cat, dog, ferret, primate (*e.g*., marmoset, rhesus monkey), *etc.* In some embodiments, a non-human anti-CD40 antibody includes an anti-CD40 antibody generated by grafting one or more human CDR sequences as described herein onto a non-human framework sequence (*e.g*., a mouse or chicken framework sequence).

The complete amino acid sequence of an exemplary native human CD40 comprises or consists of the amino acid sequence of SEQ ID NO: 149.

In some embodiments, the anti-CD40 antibody described herein specifically recognizes an epitope within human CD40. In some embodiments, the anti-CD40 antibody cross-reacts with CD40 from species other than human. In some embodiments, the anti-CD40 antibody is completely specific for human CD40 and does not exhibit cross-reactivity with CD40s from other non-human species.

In some embodiments, the anti-CD40 antibody cross-reacts with at least one allelic variant of the CD40 protein (or fragments thereof). In some embodiments, the allelic variant has up to about 30 (such as about any of 1, 2, 3, 4, 5, 6, 7, 8, 9, 10, 15, 20, 25, or 30) amino acid substitutions (such as a conservative substitution) when compared to the naturally occurring CD40 (or fragments thereof). In some embodiments, the anti-CD40 antibody does not cross-react with any allelic variants of the CD40 protein (or fragments thereof).

In some embodiments, the anti-CD40 antibody cross-reacts with at least one interspecies variant of the CD40 protein. In some embodiments, for example, the CD40 protein (or fragments thereof) is human CD40 and the interspecies variant of the CD40 protein (or fragments thereof) is a cynomolgus monkey variant thereof. In some embodiments, the anti-CD40 antibody does not cross-react with any interspecies variants of the CD40 protein.

In some embodiments, according to any of the anti-CD40 antibodies described herein, the anti-CD40 antibody comprises an antibody heavy chain constant region and an antibody light chain constant region. In some embodiments, the anti-CD40 antibody comprises an IgG1 heavy chain constant region. In some embodiments, the anti-CD40 antibody comprises an IgG2 heavy chain constant region. In some embodiments, the anti-CD40 antibody comprises an IgG3 heavy chain constant region. In some embodiments, the anti-CD40 antibody comprises an IgG4 heavy chain constant region. In some embodiments, the heavy chain constant region comprises (including consisting of or consisting essentially of) the amino acid sequence of SEQ ID NO: 145. In some embodiments, the heavy chain constant region comprises (including consisting of or consisting essentially of) the amino acid sequence of SEQ ID NO: 146. In some embodiments, the anti-CD40 antibody comprises a kappa light chain constant region. In some embodiments, the light chain constant region comprises (including consisting of or consisting essentially of) the amino acid sequence of SEQ ID NO: 147. In some embodiments, the anti-CD40 antibody comprises a lambda light chain constant region. In some embodiments, the light chain constant region comprises (including consisting of or consisting essentially of) the amino acid sequence of SEQ ID NO: 148. In some embodiments, the anti-CD40 antibody comprises an antibody heavy chain variable domain and an antibody light chain variable domain.

In some embodiments, the isolated anti-CD40 antibody comprises: a heavy chain variable domain (V_{H}) comprising a heavy chain complementarity determining region (HC-CDR) 1 comprising GYX₁VN (SEQ ID NO: 81), wherein X₁ is L, T, or V; an HC-CDR2 comprising X₁IX₂PX₃X₄GX₅TX₆YNQKFKG (SEQ ID NO: 82), wherein X₁ is L or M, X₂ is A, N, or V, X₃ is E, K, L, M, R, S, T, or Y, X₄ is H, N, S, or T, X₅ is G or R, and X₆ is A, S, T, or W; and an HC-CDR3 comprising SX₁X₂X₃X₄PYAX₅DY (SEQ ID NO: 83), wherein X₁ is G, I, L, M, P, R, S, or Y, X₂ is W or Y, X₃ is S or V, X₄ is D, K, L, N, R, T, or V, and X₅ is A, G, M, N, Q, or S; and a light chain variable domain (V_{L}) comprising a light chain complementarity determining region (LC-CDR) 1 comprising KASQNVHTX₁ VA (SEQ ID NO: 84), wherein X₁ is A, H, N, or R; an LC-CDR2 comprising LX₁SDRRT (SEQ ID NO: 85), wherein X₁ is A or R; and an LC-CDR3 comprising LQX₁WX₂X₃PLT (SEQ ID NO: 86), wherein X₁ is A or H, X₂ is N, S, or T, and X₃ is A, Q, R, S, or Y

In some embodiments, the anti-CD40 antibody comprises a V_{H} comprising: an HC-CDR1 comprising the amino acid sequence of any one of SEQ ID NOs: 1-3, or a variant thereof comprising up to about 3 (such as about any of 1, 2, or 3) amino acid substitutions, an HC-CDR2 comprising the amino acid sequence of any one of SEQ ID NOs: 9-26, or a variant thereof comprising up to about 3 (such as about any of 1, 2, or 3) amino acid substitutions, and an HC-CDR3 comprising the amino acid sequence of any one of SEQ ID NOs: 32-52, or a variant thereof comprising up to about 3 (such as about any of 1, 2, or 3) amino acid substitutions.

In some embodiments, the anti-CD40 antibody comprises a V_{H} comprising: an HC-CDR1 comprising the amino acid sequence of any one of SEQ ID NOs: 1-3, an HC-CDR2 comprising the amino acid sequence of any one of SEQ ID NOs: 9-26, and an HC-CDR3 comprising the amino acid sequence of any one of SEQ ID NOs: 32-52.

In some embodiments, the anti-CD40 antibody comprises a V_{L} comprising: an LC-CDR1 comprising the amino acid sequence of any one of SEQ ID NOs: 58-61, or a variant thereof comprising up to about 3 (such as about any of 1, 2, or 3) amino acid substitutions, an LC-CDR2 comprising the amino acid sequence of any one of SEQ ID NOs: 65-66, or a variant thereof comprising up to about 3 (such as about any of 1, 2, or 3) amino acid substitutions, and an LC-CDR3 comprising the amino acid sequence of any one of SEQ ID NOs: 70-77, or a variant thereof comprising up to about 3 (such as about any of 1, 2, or 3) amino acid substitutions.

In some embodiments, the anti-CD40 antibody comprises a V_{L} comprising: an LC-CDR1 comprising the amino acid sequence of any one of SEQ ID NOs: 58-61, an LC-CDR2 comprising the amino acid sequence of any one of SEQ ID NOs: 65-66, and an LC-CDR3 comprising the amino acid sequence of any one of SEQ ID NOs: 70-77.

In some embodiments, the anti-CD40 antibody comprises a V_{H} comprising: an HC-CDR1 comprising the amino acid sequence of any one of SEQ ID NOs: 1-3, or a variant thereof comprising up to about 3 (such as about any of 1, 2, or 3) amino acid substitutions, an HC-CDR2 comprising the amino acid sequence of any one of SEQ ID NOs: 9-26, or a variant thereof comprising up to about 3 (such as about any of 1, 2, or 3) amino acid substitutions, and an HC-CDR3 comprising the amino acid sequence of any one of SEQ ID NOs: 32-52, or a variant thereof comprising up to about 3 (such as about any of 1, 2, or 3) amino acid substitutions; and a V_{L} comprising: an LC-CDR1 comprising the amino acid sequence of any one of SEQ ID NOs: 58-61, or a variant thereof comprising up to about 3 (such as about any of 1, 2, or 3) amino acid substitutions, an LC-CDR2 comprising the amino acid sequence of any one of SEQ ID NOs: 65-66, or a variant thereof comprising up to about 3 (such as about any of 1, 2, or 3) amino acid substitutions, and an LC-CDR3 comprising the amino acid sequence of any one of SEQ ID NOs: 70-77, or a variant thereof comprising up to about 3 (such as about any of 1, 2, or 3) amino acid substitutions.

In some embodiments, the anti-CD40 antibody comprises a V_{H} comprising: an HC-CDR1 comprising the amino acid sequence of any one of SEQ ID NOs: 1-3, an HC-CDR2 comprising the amino acid sequence of any one of SEQ ID NOs: 9-26, and an HC-CDR3 comprising the amino acid sequence of any one of SEQ ID NOs: 32-52; and a V_{L} comprising: an LC-CDR1 comprising the amino acid sequence of any one of SEQ ID NOs: 58-61, an LC-CDR2 comprising the amino acid sequence of any one of SEQ ID NOs: 65-66, and an LC-CDR3 comprising the amino acid sequence of any one of SEQ ID NOs: 70-77.

In some embodiments, the anti-CD40 antibody comprises a V_{H} comprising: an HC-CDR1 comprising the amino acid sequence of SEQ ID NO: 1, an HC-CDR2 comprising the amino acid sequence of SEQ ID NO: 9, and an HC-CDR3 comprising the amino acid sequence of SEQ ID NO: 32, or a variant thereof comprising up to about 5 amino acid substitutions in the HC-CDRs; and a V_{L} comprising an LC-CDR1 comprising the amino acid sequence of SEQ ID NO: 58, an LC-CDR2 comprising the amino acid sequence of SEQ ID NO: 65, and an LC-CDR3 comprising the amino acid sequence of SEQ ID NO: 70, or a variant thereof comprising up to about 5 amino acid substitutions in the LC-CDRs.

In some embodiments, the anti-CD40 antibody comprises a V_{H} comprising: an HC-CDR1 comprising the amino acid sequence of SEQ ID NO: 1, an HC-CDR2 comprising the amino acid sequence of SEQ ID NO: 9, and an HC-CDR3 comprising the amino acid sequence of SEQ ID NO: 32; and a V_{L} comprising an LC-CDR1 comprising the amino acid sequence of SEQ ID NO: 58, an LC-CDR2 comprising the amino acid sequence of SEQ ID NO: 65, and an LC-CDR3 comprising the amino acid sequence of SEQ ID NO: 70.

In some embodiments, the anti-CD40 antibody comprises a V_{H} comprising: an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 32, or a variant thereof comprising up to about 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70, or a variant thereof comprising up to about 5 amino acid substitutions in the LC-CDRs.

In some embodiments, the anti-CD40 antibody comprises a V_{H} comprising: an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 32; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70.

In some embodiments, the anti-CD40 antibody comprises a V_{H} comprising: an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 34, or a variant thereof comprising up to about 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70, or a variant thereof comprising up to about 5 amino acid substitutions in the LC-CDRs.

In some embodiments, the anti-CD40 antibody comprises a V_{H} comprising: an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 34; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70.

In some embodiments, the anti-CD40 antibody comprises a V_{H} comprising: an HC-CDR1 comprising the amino acid sequence of SEQ ID NO: 1, an HC-CDR2 comprising the amino acid sequence of SEQ ID NO: 15, and an HC-CDR3 comprising the amino acid sequence of SEQ ID NO: 37, or a variant thereof comprising up to about 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 73, or a variant thereof comprising up to about 5 amino acid substitutions in the LC-CDRs.

In some embodiments, the anti-CD40 antibody comprises a V_{H} comprising: an HC-CDR1 comprising the amino acid sequence of SEQ ID NO: 1, an HC-CDR2 comprising the amino acid sequence of SEQ ID NO: 15, and an HC-CDR3 comprising the amino acid sequence of SEQ ID NO: 37; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 73.

In some embodiments, the anti-CD40 antibody comprises a V_{H} comprising: an HC-CDR1 comprising the amino acid sequence of SEQ ID NO: 1, an HC-CDR2 comprising the amino acid sequence of SEQ ID NO: 20, and an HC-CDR3 comprising the amino acid sequence of SEQ ID NO: 42, or a variant thereof comprising up to about 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70, or a variant thereof comprising up to about 5 amino acid substitutions in the LC-CDRs.

In some embodiments, the anti-CD40 antibody comprises a V_{H} comprising: an HC-CDR1 comprising the amino acid sequence of SEQ ID NO: 1, an HC-CDR2 comprising the amino acid sequence of SEQ ID NO: 20, and an HC-CDR3 comprising the amino acid sequence of SEQ ID NO: 42; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70.

In some embodiments, the anti-CD40 antibody comprises a V_{H} comprising: an HC-CDR1 comprising the amino acid sequence of SEQ ID NO: 1, an HC-CDR2 comprising the amino acid sequence of SEQ ID NO: 14, and an HC-CDR3 comprising the amino acid sequence of SEQ ID NO: 43, or a variant thereof comprising up to about 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70, or a variant thereof comprising up to about 5 amino acid substitutions in the LC-CDRs.

In some embodiments, the anti-CD40 antibody comprises a V_{H} comprising: an HC-CDR1 comprising the amino acid sequence of SEQ ID NO: 1, an HC-CDR2 comprising the amino acid sequence of SEQ ID NO: 14, and an HC-CDR3 comprising the amino acid sequence of SEQ ID NO: 43; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70.

In some embodiments, the anti-CD40 antibody comprises a V_{H} comprising: an HC-CDR1 comprising the amino acid sequence of SEQ ID NO: 1, an HC-CDR2 comprising the amino acid sequence of SEQ ID NO: 15, and an HC-CDR3 comprising the amino acid sequence of SEQ ID NO: 51, or a variant thereof comprising up to about 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70, or a variant thereof comprising up to about 5 amino acid substitutions in the LC-CDRs.

In some embodiments, the anti-CD40 antibody comprises a V_{H} comprising: an HC-CDR1 comprising the amino acid sequence of SEQ ID NO: 1, an HC-CDR2 comprising the amino acid sequence of SEQ ID NO: 15, and an HC-CDR3 comprising the amino acid sequence of SEQ ID NO: 51; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70.

In some embodiments, the anti-CD40 antibody comprises a V_{H} comprising: an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 48, or a variant thereof comprising up to about 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 71, or a variant thereof comprising up to about 5 amino acid substitutions in the LC-CDRs.

In some embodiments, the anti-CD40 antibody comprises a V_{H} comprising: an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 48; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 71.

In some embodiments, the anti-CD40 antibody comprises a V_{H} comprising: an HC-CDR1 comprising the amino acid sequence of SEQ ID NO: 1, an HC-CDR2 comprising the amino acid sequence of SEQ ID NO: 18, and an HC-CDR3 comprising the amino acid sequence of SEQ ID NO: 47, or a variant thereof comprising up to about 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 71, or a variant thereof comprising up to about 5 amino acid substitutions in the LC-CDRs.

In some embodiments, the anti-CD40 antibody comprises a V_{H} comprising: an HC-CDR1 comprising the amino acid sequence of SEQ ID NO: 1, an HC-CDR2 comprising the amino acid sequence of SEQ ID NO: 18, and an HC-CDR3 comprising the amino acid sequence of SEQ ID NO: 47; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 71.

In some embodiments, the anti-CD40 antibody comprises a V_{H} comprising any one of the amino acid sequences of SEQ ID NOs: 1-3, 9-26, 32-52 or a variant thereof comprising up to about 5 amino acid substitutions; and a V_{L} comprising any one of the amino acid sequences of SEQ ID NOs: 58-61, 65-66, 70-77 or a variant thereof comprising up to about 5 amino acid substitutions. In some embodiments, the anti-CD40 antibody comprises a V_{H} comprising any one of the amino acid sequences of SEQ ID NOs: 1-3, 9-26, 32-52; and a V_{L} comprising any one of the amino acid sequences of SEQ ID NOs: 58-61, 65-66, 70-77.

In some embodiments, the anti-CD40 antibody comprises a V_{H} comprising the amino acid sequences of SEQ ID NOs: 1, 9 and 32, or a variant thereof comprising up to about 5 amino acid substitutions; and a V_{L} comprising the amino acid sequences of SEQ ID NOs: 58, 65 and 70, or a variant thereof comprising up to about 5 amino acid substitutions. In some embodiments, the anti-CD40 antibody comprises a V_{H} comprising the amino acid sequences of SEQ ID NOs: 1, 9 and 32; and a V_{L} comprising the amino acid sequences of SEQ ID NOs: 58, 65 and 70.

In some embodiments, the anti-CD40 antibody comprises a V_{H} comprising the amino acid sequences of SEQ ID NOs: 1, 10 and 32, or a variant thereof comprising up to about 5 amino acid substitutions; and a V_{L} comprising the amino acid sequences of SEQ ID NOs: 59, 65 and 70, or a variant thereof comprising up to about 5 amino acid substitutions. In some embodiments, the anti-CD40 antibody comprises a V_{H} comprising the amino acid sequences of SEQ ID NOs: 1, 10 and 32; and a V_{L} comprising the amino acid sequences of SEQ ID NOs: 59, 65 and 70.

In some embodiments, the anti-CD40 antibody comprises a V_{H} comprising the amino acid sequences of SEQ ID NOs: 1, 13 and 34, or a variant thereof comprising up to about 5 amino acid substitutions; and a V_{L} comprising the amino acid sequences of SEQ ID NOs: 59, 65 and 70, or a variant thereof comprising up to about 5 amino acid substitutions. In some embodiments, the anti-CD40 antibody comprises a V_{H} comprising the amino acid sequences of SEQ ID NOs: 1, 13 and 34; and a V_{L} comprising the amino acid sequences of SEQ ID NOs: 59, 65 and 70.

In some embodiments, the anti-CD40 antibody comprises a V_{H} comprising the amino acid sequences of SEQ ID NOs: 1, 15 and 37, or a variant thereof comprising up to about 5 amino acid substitutions; and a V_{L} comprising the amino acid sequences of SEQ ID NOs: 59, 65 and 73, or a variant thereof comprising up to about 5 amino acid substitutions. In some embodiments, the anti-CD40 antibody comprises a V_{H} comprising the amino acid sequences of SEQ ID NOs: 1, 15 and 37; and a V_{L} comprising the amino acid sequences of SEQ ID NOs: 59, 65 and 73.

In some embodiments, the anti-CD40 antibody comprises a V_{H} comprising the amino acid sequences of SEQ ID NOs: 1, 20 and 42, or a variant thereof comprising up to about 5 amino acid substitutions; and a V_{L} comprising the amino acid sequences of SEQ ID NOs: 59, 65 and 70, or a variant thereof comprising up to about 5 amino acid substitutions. In some embodiments, the anti-CD40 antibody comprises a V_{H} comprising the amino acid sequences of SEQ ID NOs: 1, 20 and 42; and a V_{L} comprising the amino acid sequences of SEQ ID NOs: 59, 65 and 70.

In some embodiments, the anti-CD40 antibody comprises a V_{H} comprising the amino acid sequences of SEQ ID NOs: 1, 14 and 43, or a variant thereof comprising up to about 5 amino acid substitutions; and a V_{L} comprising the amino acid sequences of SEQ ID NOs: 59, 65 and 70, or a variant thereof comprising up to about 5 amino acid substitutions. In some embodiments, the anti-CD40 antibody comprises a V_{H} comprising the amino acid sequences of SEQ ID NOs: 1, 14 and 43; and a V_{L} comprising the amino acid sequences of SEQ ID NOs: 59, 65 and 70.

In some embodiments, the anti-CD40 antibody comprises a V_{H} comprising the amino acid sequences of SEQ ID NOs: 1, 15 and 51, or a variant thereof comprising up to about 5 amino acid substitutions; and a V_{L} comprising the amino acid sequences of SEQ ID NOs: 59, 65 and 70, or a variant thereof comprising up to about 5 amino acid substitutions. In some embodiments, the anti-CD40 antibody comprises a V_{H} comprising the amino acid sequences of SEQ ID NOs: 1, 15 and 51; and a V_{L} comprising the amino acid sequences of SEQ ID NOs: 59, 65 and 70.

In some embodiments, the anti-CD40 antibody comprises a V_{H} comprising the amino acid sequences of SEQ ID NOs: 1, 11 and 48, or a variant thereof comprising up to about 5 amino acid substitutions; and a V_{L} comprising the amino acid sequences of SEQ ID NOs: 59, 65 and 71, or a variant thereof comprising up to about 5 amino acid substitutions. In some embodiments, the anti-CD40 antibody comprises a V_{H} comprising the amino acid sequences of SEQ ID NOs: 1, 11 and 48; and a V_{L} comprising the amino acid sequences of SEQ ID NOs: 59, 65 and 71.

In some embodiments, the anti-CD40 antibody comprises a V_{H} comprising the amino acid sequences of SEQ ID NOs: 1, 18 and 47, or a variant thereof comprising up to about 5 amino acid substitutions; and a V_{L} comprising the amino acid sequences of SEQ ID NOs: 59, 65 and 71, or a variant thereof comprising up to about 5 amino acid substitutions. In some embodiments, the anti-CD40 antibody comprises a V_{H} comprising the amino acid sequences of SEQ ID NOs: 1, 18 and 47; and a V_{L} comprising the amino acid sequences of SEQ ID NOs: 59, 65 and 71.

In some embodiments, the anti-CD40 antibody comprises a V_{H} comprising an HC-CDR1, an HC-CDR2 and an HC-CDR3 of the V_{H} comprising the amino acid sequence of any one of SEQ ID NOs: 87-119; and a V_{L} comprising an LC-CDR1, an LC-CDR2, and an LC-CDR3 of the V_{L} comprising the amino acid sequence of any one of SEQ ID NOs: 126-138.

In some embodiments, the anti-CD40 antibody comprises a V_{H} comprising one, two or three HC-CDRs of SEQ ID NO: 87. In some embodiments, the anti-CD40 antibody comprises a V_{H} comprising one, two or three HC-CDRs of SEQ ID NO: 88. In some embodiments, the anti-CD40 antibody comprises a V_{H} comprising one, two or three HC-CDRs of SEQ ID NO: 89. In some embodiments, the anti-CD40 antibody comprises a V_{H} comprising one, two or three HC-CDRs of SEQ ID NO: 94. In some embodiments, the anti-CD40 antibody comprises a V_{H} comprising one, two or three HC-CDRs of SEQ ID NO: 96. In some embodiments, the anti-CD40 antibody comprises a V_{H} comprising one, two or three HC-CDRs of SEQ ID NO: 101. In some embodiments, the anti-CD40 antibody comprises a V_{H} comprising one, two or three HC-CDRs of SEQ ID NO: 110. In some embodiments, the anti-CD40 antibody comprises a V_{H} comprising one, two or three HC-CDRs of SEQ ID NO: 114. In some embodiments, the anti-CD40 antibody comprises a V_{H} comprising one, two or three HC-CDRs of SEQ ID NO: 115. In some embodiments, the anti-CD40 antibody comprises a V_{H} comprising one, two or three HC-CDRs of SEQ ID NO: 117.

In some embodiments, the anti-CD40 antibody comprises a V_{L} comprising one, two or three LC-CDRs of SEQ ID NO: 126. In some embodiments, the anti-CD40 antibody comprises a V_{L} comprising one, two or three LC-CDRs of SEQ ID NO: 127. In some embodiments, the anti-CD40 antibody comprises a V_{L} comprising one, two or three LC-CDRs of SEQ ID NO: 128. In some embodiments, the anti-CD40 antibody comprises a V_{L} comprising one, two or three LC-CDRs of SEQ ID NO: 129. In some embodiments, the anti-CD40 antibody comprises a V_{L} comprising one, two or three LC-CDRs of SEQ ID NO: 132.

In some embodiments, the anti-CD40 antibody comprises a V_{H} comprising an HC-CDR1, an HC-CDR2 and an HC-CDR3 of the V_{H} comprising the amino acid sequence of SEQ ID NO: 87, and a V_{L} comprising an LC-CDR1, an LC-CDR2 and an LC-CDR3 of the V_{L} comprising the amino acid sequence of SEQ ID NO: 126.

In some embodiments, the anti-CD40 antibody comprises a V_{H} comprising an HC-CDR1, an HC-CDR2 and an HC-CDR3 of the V_{H} comprising the amino acid sequence of SEQ ID NO: 88, and a V_{L} comprising an LC-CDR1, an LC-CDR2 and an LC-CDR3 of the V_{L} comprising the amino acid sequence of SEQ ID NO: 127.

In some embodiments, the anti-CD40 antibody comprises a V_{H} comprising an HC-CDR1, an HC-CDR2 and an HC-CDR3 of the V_{H} comprising the amino acid sequence of SEQ ID NO: 89, and a V_{L} comprising an LC-CDR1, an LC-CDR2 and an LC-CDR3 of the V_{L} comprising the amino acid sequence of SEQ ID NO: 128.

In some embodiments, the anti-CD40 antibody comprises a V_{H} comprising an HC-CDR1, an HC-CDR2 and an HC-CDR3 of the V_{H} comprising the amino acid sequence of SEQ ID NO: 94, and a V_{L} comprising an LC-CDR1, an LC-CDR2 and an LC-CDR3 of the V_{L} comprising the amino acid sequence of SEQ ID NO: 128.

In some embodiments, the anti-CD40 antibody comprises a V_{H} comprising an HC-CDR1, an HC-CDR2 and an HC-CDR3 of the V_{H} comprising the amino acid sequence of SEQ ID NO: 96, and a V_{L} comprising an LC-CDR1, an LC-CDR2 and an LC-CDR3 of the V_{L} comprising the amino acid sequence of SEQ ID NO: 132.

In some embodiments, the anti-CD40 antibody comprises a V_{H} comprising an HC-CDR1, an HC-CDR2 and an HC-CDR3 of the V_{H} comprising the amino acid sequence of SEQ ID NO: 101, and a V_{L} comprising an LC-CDR1, an LC-CDR2 and an LC-CDR3 of the V_{L} comprising the amino acid sequence of SEQ ID NO: 128.

In some embodiments, the anti-CD40 antibody comprises a V_{H} comprising an HC-CDR1, an HC-CDR2 and an HC-CDR3 of the V_{H} comprising the amino acid sequence of SEQ ID NO: 110, and a V_{L} comprising an LC-CDR1, an LC-CDR2 and an LC-CDR3 of the V_{L} comprising the amino acid sequence of SEQ ID NO: 128.

In some embodiments, the anti-CD40 antibody comprises a V_{H} comprising an HC-CDR1, an HC-CDR2 and an HC-CDR3 of the V_{H} comprising the amino acid sequence of SEQ ID NO: 114, and a V_{L} comprising an LC-CDR1, an LC-CDR2 and an LC-CDR3 of the V_{L} comprising the amino acid sequence of SEQ ID NO: 128.

In some embodiments, the anti-CD40 antibody comprises a V_{H} comprising an HC-CDR1, an HC-CDR2 and an HC-CDR3 of the V_{H} comprising the amino acid sequence of SEQ ID NO: 115, and a V_{L} comprising an LC-CDR1, an LC-CDR2 and an LC-CDR3 of the V_{L} comprising the amino acid sequence of SEQ ID NO: 129.

In some embodiments, the anti-CD40 antibody comprises a V_{H} comprising an HC-CDR1, an HC-CDR2 and an HC-CDR3 of the V_{H} comprising the amino acid sequence of SEQ ID NO: 117, and a V_{L} comprising an LC-CDR1, an LC-CDR2 and an LC-CDR3 of the V_{L} comprising the amino acid sequence of SEQ ID NO: 129.

In some embodiments, the anti-CD40 antibody comprises a V_{H} comprising the amino acid sequence of any one of SEQ ID NOs: 87-119, or a variant thereof having at least about 90% (such as at least about any of 91%, 92%, 93%, 94%, 95%, 96%, 97%, 98%, or 99%) sequence identity, and a V_{L} comprising the amino acid sequence of any one of SEQ ID NOs: 126-138, or a variant thereof having at least about 90% (such as at least about any of 91%, 92%, 93%, 94%, 95%, 96%, 97%, 98%, or 99%) sequence identity. In some embodiments, the anti-CD40 antibody comprises a V_{H} comprising the amino acid sequence of any one of SEQ ID NOs: 87-119, and a V_{L} comprising the amino acid sequence of any one of SEQ ID NOs: 126-138.

In some embodiments, the anti-CD40 antibody comprises a V_{H} comprising the amino acid sequence of SEQ ID NO: 87, or a variant thereof having at least about 90% (such as at least about any of 91%, 92%, 93%, 94%, 95%, 96%, 97%, 98%, or 99%) sequence identity, and a V_{L} comprising the amino acid sequence of SEQ ID NO: 126, or a variant thereof having at least about 90% (such as at least about any of 91%, 92%, 93%, 94%, 95%, 96%, 97%, 98%, or 99%) sequence identity. In some embodiments, the anti-CD40 antibody comprises a V_{H} comprising the amino acid sequence of SEQ ID NO: 87 and a V_{L} comprising the amino acid sequence of SEQ ID NO: 126.

In some embodiments, the anti-CD40 antibody comprises a V_{H} comprising the amino acid sequence of SEQ ID NO: 88, or a variant thereof having at least about 90% (such as at least about any of 91%, 92%, 93%, 94%, 95%, 96%, 97%, 98%, or 99%) sequence identity, and a V_{L} comprising the amino acid sequence of SEQ ID NO: 127, or a variant thereof having at least about 90% (such as at least about any of 91%, 92%, 93%, 94%, 95%, 96%, 97%, 98%, or 99%) sequence identity. In some embodiments, the anti-CD40 antibody comprises a V_{H} comprising the amino acid sequence of SEQ ID NO: 88 and a V_{L} comprising the amino acid sequence of SEQ ID NO: 127.

In some embodiments, the anti-CD40 antibody comprises a V_{H} comprising the amino acid sequence of SEQ ID NO: 89, or a variant thereof having at least about 90% (such as at least about any of 91%, 92%, 93%, 94%, 95%, 96%, 97%, 98%, or 99%) sequence identity, and a V_{L} comprising the amino acid sequence of SEQ ID NO: 128, or a variant thereof having at least about 90% (such as at least about any of 91%, 92%, 93%, 94%, 95%, 96%, 97%, 98%, or 99%) sequence identity. In some embodiments, the anti-CD40 antibody comprises a V_{H} comprising the amino acid sequence of SEQ ID NO: 89 and a V_{L} comprising the amino acid sequence of SEQ ID NO: 128.

In some embodiments, the anti-CD40 antibody comprises a V_{H} comprising the amino acid sequence of SEQ ID NO: 94, or a variant thereof having at least about 90% (such as at least about any of 91%, 92%, 93%, 94%, 95%, 96%, 97%, 98%, or 99%) sequence identity, and a V_{L} comprising the amino acid sequence of SEQ ID NO: 128, or a variant thereof having at least about 90% (such as at least about any of 91%, 92%, 93%, 94%, 95%, 96%, 97%, 98%, or 99%) sequence identity. In some embodiments, the anti-CD40 antibody comprises a V_{H} comprising the amino acid sequence of SEQ ID NO: 94 and a V_{L} comprising the amino acid sequence of SEQ ID NO: 128.

In some embodiments, the anti-CD40 antibody comprises a V_{H} comprising the amino acid sequence of SEQ ID NO: 96, or a variant thereof having at least about 90% (such as at least about any of 91%, 92%, 93%, 94%, 95%, 96%, 97%, 98%, or 99%) sequence identity, and a V_{L} comprising the amino acid sequence of SEQ ID NO: 132, or a variant thereof having at least about 90% (such as at least about any of 91%, 92%, 93%, 94%, 95%, 96%, 97%, 98%, or 99%) sequence identity. In some embodiments, the anti-CD40 antibody comprises a V_{H} comprising the amino acid sequence of SEQ ID NO: 96 and a V_{L} comprising the amino acid sequence of SEQ ID NO: 132.

In some embodiments, the anti-CD40 antibody comprises a V_{H} comprising the amino acid sequence of SEQ ID NO: 101, or a variant thereof having at least about 90% (such as at least about any of 91%, 92%, 93%, 94%, 95%, 96%, 97%, 98%, or 99%) sequence identity, and a V_{L} comprising the amino acid sequence of SEQ ID NO: 128, or a variant thereof having at least about 90% (such as at least about any of 91%, 92%, 93%, 94%, 95%, 96%, 97%, 98%, or 99%) sequence identity. In some embodiments, the anti-CD40 antibody comprises a V_{H} comprising the amino acid sequence of SEQ ID NO: 101 and a V_{L} comprising the amino acid sequence of SEQ ID NO: 128.

In some embodiments, the anti-CD40 antibody comprises a V_{H} comprising the amino acid sequence of SEQ ID NO: 110, or a variant thereof having at least about 90% (such as at least about any of 91%, 92%, 93%, 94%, 95%, 96%, 97%, 98%, or 99%) sequence identity, and a V_{L} comprising the amino acid sequence of SEQ ID NO: 128, or a variant thereof having at least about 90% (such as at least about any of 91%, 92%, 93%, 94%, 95%, 96%, 97%, 98%, or 99%) sequence identity. In some embodiments, the anti-CD40 antibody comprises a V_{H} comprising the amino acid sequence of SEQ ID NO: 110 and a V_{L} comprising the amino acid sequence of SEQ ID NO: 128.

In some embodiments, the anti-CD40 antibody comprises a V_{H} comprising the amino acid sequence of SEQ ID NO: 114, or a variant thereof having at least about 90% (such as at least about any of 91%, 92%, 93%, 94%, 95%, 96%, 97%, 98%, or 99%) sequence identity, and a V_{L} comprising the amino acid sequence of SEQ ID NO: 128, or a variant thereof having at least about 90% (such as at least about any of 91%, 92%, 93%, 94%, 95%, 96%, 97%, 98%, or 99%) sequence identity. In some embodiments, the anti-CD40 antibody comprises a V_{H} comprising the amino acid sequence of SEQ ID NO: 114 and a V_{L} comprising the amino acid sequence of SEQ ID NO: 128.

In some embodiments, the anti-CD40 antibody comprises a V_{H} comprising the amino acid sequence of SEQ ID NO: 115, or a variant thereof having at least about 90% (such as at least about any of 91%, 92%, 93%, 94%, 95%, 96%, 97%, 98%, or 99%) sequence identity, and a V_{L} comprising the amino acid sequence of SEQ ID NO: 129, or a variant thereof having at least about 90% (such as at least about any of 91%, 92%, 93%, 94%, 95%, 96%, 97%, 98%, or 99%) sequence identity. In some embodiments, the anti-CD40 antibody comprises a V_{H} comprising the amino acid sequence of SEQ ID NO: 115 and a V_{L} comprising the amino acid sequence of SEQ ID NO: 129.

In some embodiments, the anti-CD40 antibody comprises a V_{H} comprising the amino acid sequence of SEQ ID NO: 117, or a variant thereof having at least about 90% (such as at least about any of 91%, 92%, 93%, 94%, 95%, 96%, 97%, 98%, or 99%) sequence identity, and a V_{L} comprising the amino acid sequence of SEQ ID NO: 129, or a variant thereof having at least about 90% (such as at least about any of 91%, 92%, 93%, 94%, 95%, 96%, 97%, 98%, or 99%) sequence identity. In some embodiments, the anti-CD40 antibody comprises a V_{H} comprising the amino acid sequence of SEQ ID NO: 117 and a V_{L} comprising the amino acid sequence of SEQ ID NO: 129.

In some embodiments, the anti-CD40 antibody comprises a V_{H} comprising: an HC-CDR1 comprising the amino acid sequence of SEQ ID NO: 4, an HC-CDR2 comprising the amino acid sequence of SEQ ID NO: 27, and an HC-CDR3 comprising the amino acid sequence of SEQ ID NO: 53, or a variant thereof comprising up to about 5 amino acid substitutions in the HC-CDRs; and a V_{L} comprising an LC-CDR1 comprising the amino acid sequence of SEQ ID NO: 62, an LC-CDR2 comprising the amino acid sequence of SEQ ID NO: 67, and an LC-CDR3 comprising the amino acid sequence of SEQ ID NO: 78, or a variant thereof comprising up to about 5 amino acid substitutions in the LC-CDRs.

In some embodiments, the anti-CD40 antibody comprises a V_{H} comprising: an HC-CDR1 comprising the amino acid sequence of SEQ ID NO: 4, an HC-CDR2 comprising the amino acid sequence of SEQ ID NO: 27, and an HC-CDR3 comprising the amino acid sequence of SEQ ID NO: 53; and a V_{L} comprising an LC-CDR1 comprising the amino acid sequence of SEQ ID NO: 62, an LC-CDR2 comprising the amino acid sequence of SEQ ID NO: 67, and an LC-CDR3 comprising the amino acid sequence of SEQ ID NO: 78.

In some embodiments, the anti-CD40 antibody comprises a V_{H} comprising the amino acid sequences of SEQ ID NOs: 4, 27 and 53, or a variant thereof comprising up to about 5 amino acid substitutions; and a V_{L} comprising the amino acid sequences of SEQ ID NOs: 62, 67 and 78, or a variant thereof comprising up to about 5 amino acid substitutions. In some embodiments, the anti-CD40 antibody comprises a V_{H} comprising the amino acid sequences of SEQ ID NOs: 4, 27 and 53; and a V_{L} comprising the amino acid sequences of SEQ ID NOs: 62, 67 and 78.

In some embodiments, the anti-CD40 antibody comprises a V_{H} comprising one, two or three HC-CDRs of SEQ ID NO: 120.

In some embodiments, the anti-CD40 antibody comprises a V_{L} comprising one, two or three LC-CDRs of SEQ ID NO: 139.

In some embodiments, the anti-CD40 antibody comprises a V_{H} comprising an HC-CDR1, an HC-CDR2 and an HC-CDR3 of the V_{H} comprising the amino acid sequence of SEQ ID NO: 120, and a V_{L} comprising an LC-CDR1, an LC-CDR2 and an LC-CDR3 of the V_{L} comprising the amino acid sequence of SEQ ID NO: 139.

In some embodiments, the anti-CD40 antibody comprises a V_{H} comprising the amino acid sequence of SEQ ID NO: 120, or a variant thereof having at least about 90% (such as at least about any of 91%, 92%, 93%, 94%, 95%, 96%, 97%, 98%, or 99%) sequence identity, and a V_{L} comprising the amino acid sequence of SEQ ID NO: 139, or a variant thereof having at least about 90% (such as at least about any of 91%, 92%, 93%, 94%, 95%, 96%, 97%, 98%, or 99%) sequence identity. In some embodiments, the anti-CD40 antibody comprises a V_{H} comprising the amino acid sequence of SEQ ID NO: 120 and a V_{L} comprising the amino acid sequence of SEQ ID NO: 139.

In some embodiments, the anti-CD40 antibody comprises a V_{H} comprising: an HC-CDR1 comprising the amino acid sequence of SEQ ID NO: 5, an HC-CDR2 comprising the amino acid sequence of SEQ ID NO: 27, and an HC-CDR3 comprising the amino acid sequence of SEQ ID NO: 54, or a variant thereof comprising up to about 5 amino acid substitutions in the HC-CDRs; and a V_{L} comprising an LC-CDR1 comprising the amino acid sequence of SEQ ID NO: 62, an LC-CDR2 comprising the amino acid sequence of SEQ ID NO: 67, and an LC-CDR3 comprising the amino acid sequence of SEQ ID NO: 78, or a variant thereof comprising up to about 5 amino acid substitutions in the LC-CDRs.

In some embodiments, the anti-CD40 antibody comprises a V_{H} comprising: an HC-CDR1 comprising the amino acid sequence of SEQ ID NO: 5, an HC-CDR2 comprising the amino acid sequence of SEQ ID NO: 27, and an HC-CDR3 comprising the amino acid sequence of SEQ ID NO: 54; and a V_{L} comprising an LC-CDR1 comprising the amino acid sequence of SEQ ID NO: 62, an LC-CDR2 comprising the amino acid sequence of SEQ ID NO: 67, and an LC-CDR3 comprising the amino acid sequence of SEQ ID NO: 78.

In some embodiments, the anti-CD40 antibody comprises a V_{H} comprising the amino acid sequences of SEQ ID NOs: 5, 27 and 54, or a variant thereof comprising up to about 5 amino acid substitutions; and a V_{L} comprising the amino acid sequences of SEQ ID NOs: 62, 67 and 78, or a variant thereof comprising up to about 5 amino acid substitutions. In some embodiments, the anti-CD40 antibody comprises a V_{H} comprising the amino acid sequences of SEQ ID NOs: 5, 27 and 54; and a V_{L} comprising the amino acid sequences of SEQ ID NOs: 62, 67 and 78.

In some embodiments, the anti-CD40 antibody comprises a V_{H} comprising one, two or three HC-CDRs of SEQ ID NO: 121.

In some embodiments, the anti-CD40 antibody comprises a V_{L} comprising one, two or three LC-CDRs of SEQ ID NO: 140.

In some embodiments, the anti-CD40 antibody comprises a V_{H} comprising an HC-CDR1, an HC-CDR2 and an HC-CDR3 of the V_{H} comprising the amino acid sequence of SEQ ID NO: 121, and a V_{L} comprising an LC-CDR1, an LC-CDR2 and an LC-CDR3 of the V_{L} comprising the amino acid sequence of SEQ ID NO: 140.

In some embodiments, the anti-CD40 antibody comprises a V_{H} comprising the amino acid sequence of SEQ ID NO: 121, or a variant thereof having at least about 90% (such as at least about any of 91%, 92%, 93%, 94%, 95%, 96%, 97%, 98%, or 99%) sequence identity, and a V_{L} comprising the amino acid sequence of SEQ ID NO: 140, or a variant thereof having at least about 90% (such as at least about any of 91%, 92%, 93%, 94%, 95%, 96%, 97%, 98%, or 99%) sequence identity. In some embodiments, the anti-CD40 antibody comprises a V_{H} comprising the amino acid sequence of SEQ ID NO: 121 and a V_{L} comprising the amino acid sequence of SEQ ID NO: 140.

In some embodiments, the anti-CD40 antibody comprises a V_{H} comprising: an HC-CDR1 comprising the amino acid sequence of SEQ ID NO: 6, an HC-CDR2 comprising the amino acid sequence of SEQ ID NO: 28, and an HC-CDR3 comprising the amino acid sequence of SEQ ID NO: 55, or a variant thereof comprising up to about 5 amino acid substitutions in the HC-CDRs; and a V_{L} comprising an LC-CDR1 comprising the amino acid sequence of SEQ ID NO: 63, an LC-CDR2 comprising the amino acid sequence of SEQ ID NO: 68, and an LC-CDR3 comprising the amino acid sequence of SEQ ID NO: 79, or a variant thereof comprising up to about 5 amino acid substitutions in the LC-CDRs.

In some embodiments, the anti-CD40 antibody comprises a V_{H} comprising: an HC-CDR1 comprising the amino acid sequence of SEQ ID NO: 6, an HC-CDR2 comprising the amino acid sequence of SEQ ID NO: 28, and an HC-CDR3 comprising the amino acid sequence of SEQ ID NO: 55; and a V_{L} comprising an LC-CDR1 comprising the amino acid sequence of SEQ ID NO: 63, an LC-CDR2 comprising the amino acid sequence of SEQ ID NO: 68, and an LC-CDR3 comprising the amino acid sequence of SEQ ID NO: 79.

In some embodiments, the anti-CD40 antibody comprises a V_{H} comprising the amino acid sequences of SEQ ID NOs: 6, 28 and 55, or a variant thereof comprising up to about 5 amino acid substitutions; and a V_{L} comprising the amino acid sequences of SEQ ID NOs: 63, 68 and 79, or a variant thereof comprising up to about 5 amino acid substitutions. In some embodiments, the anti-CD40 antibody comprises a V_{H} comprising the amino acid sequences of SEQ ID NOs: 6, 28 and 55; and a V_{L} comprising the amino acid sequences of SEQ ID NOs: 63, 68 and 79.

In some embodiments, the anti-CD40 antibody comprises a V_{H} comprising one, two or three HC-CDRs of SEQ ID NO: 122.

In some embodiments, the anti-CD40 antibody comprises a V_{L} comprising one, two or three LC-CDRs of SEQ ID NO: 141.

In some embodiments, the anti-CD40 antibody comprises a V_{H} comprising an HC-CDR1, an HC-CDR2 and an HC-CDR3 of the V_{H} comprising the amino acid sequence of SEQ ID NO: 122, and a V_{L} comprising an LC-CDR1, an LC-CDR2 and an LC-CDR3 of the V_{L} comprising the amino acid sequence of SEQ ID NO: 141.

In some embodiments, the anti-CD40 antibody comprises a V_{H} comprising the amino acid sequence of SEQ ID NO: 122, or a variant thereof having at least about 90% (such as at least about any of 91%, 92%, 93%, 94%, 95%, 96%, 97%, 98%, or 99%) sequence identity, and a V_{L} comprising the amino acid sequence of SEQ ID NO: 141, or a variant thereof having at least about 90% (such as at least about any of 91%, 92%, 93%, 94%, 95%, 96%, 97%, 98%, or 99%) sequence identity. In some embodiments, the anti-CD40 antibody comprises a V_{H} comprising the amino acid sequence of SEQ ID NO: 122 and a V_{L} comprising the amino acid sequence of SEQ ID NO: 141.

In some embodiments, the anti-CD40 antibody comprises a V_{H} comprising: an HC-CDR1 comprising the amino acid sequence of SEQ ID NO: 7, an HC-CDR2 comprising the amino acid sequence of SEQ ID NO: 29, and an HC-CDR3 comprising the amino acid sequence of SEQ ID NO: 56, or a variant thereof comprising up to about 5 amino acid substitutions in the HC-CDRs; and a V_{L} comprising an LC-CDR1 comprising the amino acid sequence of SEQ ID NO: 64, an LC-CDR2 comprising the amino acid sequence of SEQ ID NO: 69, and an LC-CDR3 comprising the amino acid sequence of SEQ ID NO: 80, or a variant thereof comprising up to about 5 amino acid substitutions in the LC-CDRs.

In some embodiments, the anti-CD40 antibody comprises a V_{H} comprising: an HC-CDR1 comprising the amino acid sequence of SEQ ID NO: 7, an HC-CDR2 comprising the amino acid sequence of SEQ ID NO: 29, and an HC-CDR3 comprising the amino acid sequence of SEQ ID NO: 56; and a V_{L} comprising an LC-CDR1 comprising the amino acid sequence of SEQ ID NO: 64, an LC-CDR2 comprising the amino acid sequence of SEQ ID NO: 69, and an LC-CDR3 comprising the amino acid sequence of SEQ ID NO: 80.

In some embodiments, the anti-CD40 antibody comprises a V_{H} comprising the amino acid sequences of SEQ ID NOs: 7, 29 and 56, or a variant thereof comprising up to about 5 amino acid substitutions; and a V_{L} comprising the amino acid sequences of SEQ ID NOs: 64, 69 and 80, or a variant thereof comprising up to about 5 amino acid substitutions. In some embodiments, the anti-CD40 antibody comprises a V_{H} comprising the amino acid sequences of SEQ ID NOs: 7, 29 and 56; and a V_{L} comprising the amino acid sequences of SEQ ID NOs: 64, 69 and 80.

In some embodiments, the anti-CD40 antibody comprises a V_{H} comprising one, two or three HC-CDRs of SEQ ID NO: 123.

In some embodiments, the anti-CD40 antibody comprises a V_{L} comprising one, two or three LC-CDRs of SEQ ID NO: 142.

In some embodiments, the anti-CD40 antibody comprises a V_{H} comprising an HC-CDR1, an HC-CDR2 and an HC-CDR3 of the V_{H} comprising the amino acid sequence of SEQ ID NO: 123, and a V_{L} comprising an LC-CDR1, an LC-CDR2 and an LC-CDR3 of the V_{L} comprising the amino acid sequence of SEQ ID NO: 142.

In some embodiments, the anti-CD40 antibody comprises a V_{H} comprising the amino acid sequence of SEQ ID NO: 123, or a variant thereof having at least about 90% (such as at least about any of 91%, 92%, 93%, 94%, 95%, 96%, 97%, 98%, or 99%) sequence identity, and a V_{L} comprising the amino acid sequence of SEQ ID NO: 142, or a variant thereof having at least about 90% (such as at least about any of 91%, 92%, 93%, 94%, 95%, 96%, 97%, 98%, or 99%) sequence identity. In some embodiments, the anti-CD40 antibody comprises a V_{H} comprising the amino acid sequence of SEQ ID NO: 123 and a V_{L} comprising the amino acid sequence of SEQ ID NO: 142.

In some embodiments, the anti-CD40 antibody comprises a V_{H} comprising: an HC-CDR1 comprising the amino acid sequence of SEQ ID NO: 8, an HC-CDR2 comprising the amino acid sequence of SEQ ID NO: 30, and an HC-CDR3 comprising the amino acid sequence of SEQ ID NO: 57, or a variant thereof comprising up to about 5 amino acid substitutions in the HC-CDRs; and a V_{L} comprising an LC-CDR1 comprising the amino acid sequence of SEQ ID NO: 62, an LC-CDR2 comprising the amino acid sequence of SEQ ID NO: 67, and an LC-CDR3 comprising the amino acid sequence of SEQ ID NO: 80, or a variant thereof comprising up to about 5 amino acid substitutions in the LC-CDRs.

In some embodiments, the anti-CD40 antibody comprises a V_{H} comprising: an HC-CDR1 comprising the amino acid sequence of SEQ ID NO: 8, an HC-CDR2 comprising the amino acid sequence of SEQ ID NO: 30, and an HC-CDR3 comprising the amino acid sequence of SEQ ID NO: 57; and a V_{L} comprising an LC-CDR1 comprising the amino acid sequence of SEQ ID NO: 62, an LC-CDR2 comprising the amino acid sequence of SEQ ID NO: 67, and an LC-CDR3 comprising the amino acid sequence of SEQ ID NO: 80.

In some embodiments, the anti-CD40 antibody comprises a V_{H} comprising the amino acid sequences of SEQ ID NOs: 8, 30 and 57, or a variant thereof comprising up to about 5 amino acid substitutions; and a V_{L} comprising the amino acid sequences of SEQ ID NOs: 62, 67 and 80, or a variant thereof comprising up to about 5 amino acid substitutions. In some embodiments, the anti-CD40 antibody comprises a V_{H} comprising the amino acid sequences of SEQ ID NOs: 8, 30 and 57; and a V_{L} comprising the amino acid sequences of SEQ ID NOs: 62, 67 and 80.

In some embodiments, the anti-CD40 antibody comprises a V_{H} comprising one, two or three HC-CDRs of SEQ ID NO: 124.

In some embodiments, the anti-CD40 antibody comprises a V_{L} comprising one, two or three LC-CDRs of SEQ ID NO: 143

In some embodiments, the anti-CD40 antibody comprises a V_{H} comprising an HC-CDR1, an HC-CDR2 and an HC-CDR3 of the V_{H} comprising the amino acid sequence of SEQ ID NO: 124, and a V_{L} comprising an LC-CDR1, an LC-CDR2 and an LC-CDR3 of the V_{L} comprising the amino acid sequence of SEQ ID NO: 143.

In some embodiments, the anti-CD40 antibody comprises a V_{H} comprising the amino acid sequence of SEQ ID NO: 124, or a variant thereof having at least about 90% (such as at least about any of 91%, 92%, 93%, 94%, 95%, 96%, 97%, 98%, or 99%) sequence identity, and a V_{L} comprising the amino acid sequence of SEQ ID NO: 143, or a variant thereof having at least about 90% (such as at least about any of 91%, 92%, 93%, 94%, 95%, 96%, 97%, 98%, or 99%) sequence identity. In some embodiments, the anti-CD40 antibody comprises a V_{H} comprising the amino acid sequence of SEQ ID NO: 124 and a V_{L} comprising the amino acid sequence of SEQ ID NO: 143.

In some embodiments, the anti-CD40 antibody comprises a V_{H} comprising: an HC-CDR1 comprising the amino acid sequence of SEQ ID NO: 4, an HC-CDR2 comprising the amino acid sequence of SEQ ID NO: 31, and an HC-CDR3 comprising the amino acid sequence of SEQ ID NO: 53, or a variant thereof comprising up to about 5 amino acid substitutions in the HC-CDRs; and a V_{L} comprising an LC-CDR1 comprising the amino acid sequence of SEQ ID NO: 62, an LC-CDR2 comprising the amino acid sequence of SEQ ID NO: 68, and an LC-CDR3 comprising the amino acid sequence of SEQ ID NO: 79, or a variant thereof comprising up to about 5 amino acid substitutions in the LC-CDRs.

In some embodiments, the anti-CD40 antibody comprises a V_{H} comprising: an HC-CDR1 comprising the amino acid sequence of SEQ ID NO: 4, an HC-CDR2 comprising the amino acid sequence of SEQ ID NO: 31, and an HC-CDR3 comprising the amino acid sequence of SEQ ID NO: 53; and a V_{L} comprising an LC-CDR1 comprising the amino acid sequence of SEQ ID NO: 62, an LC-CDR2 comprising the amino acid sequence of SEQ ID NO: 68, and an LC-CDR3 comprising the amino acid sequence of SEQ ID NO: 79.

In some embodiments, the anti-CD40 antibody comprises a V_{H} comprising the amino acid sequences of SEQ ID NOs: 4, 31 and 53, or a variant thereof comprising up to about 5 amino acid substitutions; and a V_{L} comprising the amino acid sequences of SEQ ID NOs: 62, 68 and 79, or a variant thereof comprising up to about 5 amino acid substitutions. In some embodiments, the anti-CD40 antibody comprises a V_{H} comprising the amino acid sequences of SEQ ID NOs: 4, 31 and 53; and a V_{L} comprising the amino acid sequences of SEQ ID NOs: 62, 68 and 79.

In some embodiments, the anti-CD40 antibody comprises a V_{H} comprising one, two or three HC-CDRs of SEQ ID NO: 125.

In some embodiments, the anti-CD40 antibody comprises a V_{L} comprising one, two or three LC-CDRs of SEQ ID NO: 144.

In some embodiments, the anti-CD40 antibody comprises a V_{H} comprising an HC-CDR1, an HC-CDR2 and an HC-CDR3 of the V_{H} comprising the amino acid sequence of SEQ ID NO: 125, and a V_{L} comprising an LC-CDR1, an LC-CDR2 and an LC-CDR3 of the V_{L} comprising the amino acid sequence of SEQ ID NO: 144.

In some embodiments, the anti-CD40 antibody comprises a V_{H} comprising the amino acid sequence of SEQ ID NO: 125, or a variant thereof having at least about 90% (such as at least about any of 91%, 92%, 93%, 94%, 95%, 96%, 97%, 98%, or 99%) sequence identity, and a V_{L} comprising the amino acid sequence of SEQ ID NO: 144, or a variant thereof having at least about 90% (such as at least about any of 91%, 92%, 93%, 94%, 95%, 96%, 97%, 98%, or 99%) sequence identity. In some embodiments, the anti-CD40 antibody comprises a V_{H} comprising the amino acid sequence of SEQ ID NO: 125 and a V_{L} comprising the amino acid sequence of SEQ ID NO: 144.

In some embodiments, the amino acid substitutions described above are limited to "exemplary substitutions" shown in Table 4 of this application.In some embodiments, the amino acid substitutions are limited to "preferred substitutions" shown in Table 4 of this application.

In some embodiments, functional epitopes can be mapped by combinatorial alanine scanning. In this process, a combinatorial alanine-scanning strategy can be used to identify amino acids in the CD40 protein that are necessary for interaction with CD40 antibodies. In some embodiments, the epitope is conformational and crystal structure of anti-CD40 antibodies bound to CD40 may be employed to identify the epitopes.

In some embodiments, the present application provides antibodies which compete with any one of the CD40 antibodies described herein for binding to CD40. In some embodiments, the present application provides antibodies which compete with any one of the anti-CD40 antibodies provided herein for binding to an epitope on the CD40. In some embodiments, an anti-CD40 antibody is provided that binds to the same epitope as an anti-CD40 antibody comprising a V_{H} comprising the amino acid sequence of any one of SEQ ID NOs: 87-125, and a V_{L} comprising the amino acid sequence of any one of SEQ ID NOs: 126-144. In some embodiments, an anti-CD40 antibody is provided that specifically binds to CD40 competitively with an anti-CD40 antibody comprising a V_{H} comprising the amino acid sequence of any one of SEQ ID NOs: 87-125 and a V_{L} comprising the amino acid sequence of any one of SEQ ID NOs: 126-144.

In some embodiments, competition assays may be used to identify a monoclonal antibody that competes with an anti-CD40 antibody described herein for binding to CD40. Competition assays can be used to determine whether two antibodies bind to the same epitope by recognizing identical or sterically overlapping epitopes or one antibody competitively inhibits binding of another antibody to the antigen. In certain embodiments, such a competing antibody binds to the same epitope that is bound by an antibody described herein. Exemplary competition assays include, but are not limited to, routine assays such as those provided in Harlow and Lane (1988) Antibodies: A Laboratory Manual ch.14 (Cold Spring Harbor Laboratory, Cold Spring Harbor, N.Y.). Detailed exemplary methods for mapping an epitope to which an antibody binds are provided in Morris (1996) "Epitope Mapping Protocols", in Methods in Molecular Biology vol. 66 (Humana Press, Totowa, N.J.). In some embodiments, two antibodies are said to bind to the same epitope if each blocks binding of the other by 50% or more. In some embodiments, the antibody that competes with an anti-CD40 antibody described herein is a chimeric, humanized or human antibody.

Exemplary anti-CD40 antibody sequences are shown in Tables 2 and 3, wherein the CDR numbering is according to the EU index of Kabat. Those skilled in the art will recognize that many algorithms are known for prediction of CDR positions and for delimitation of antibody heavy chain and light chain variable regions. Anti-CD40 antibodies comprising CDRs, V_{H} and/or V_{L} sequences from antibodies described herein, but based on prediction algorithms other than those exemplified in the tables below, are within the scope of this invention.

**Table 2. Exemplary anti-CD40 antibody CDR sequences.**

| **Antibody Name** | **HC-CDR1** | **HC-CDR2** | **HC-CDR3** |
|---|---|---|---|
| D1 | GYTVN (SEQ ID NO: 1) | LINPYNGGTTYNQKFKG (SEQ ID NO: 9) | SYYSNPYAMDY (SEQ ID NO: 32) |
| D11 | GYTVN (SEQ ID NO: 1) | LINPYNGGTTYNQKFKG (SEQ ID NO: 9) | SYYSNPYAMDY (SEQ ID NO: 32) |
| D21 | GYTVN (SEQ ID NO: 1) | LINPRNGGTTYNQKFKG (SEQ ID NO: 10) | SYYSNPYAMDY (SEQ ID NO: 32) |
| D22 | GYTVN (SEQ ID NO: 1) | LINPYTGGTTYNQKFKG (SEQ ID NO: 11) | SYYSKPYAMDY (SEQ ID NO: 33) |
| D23 | GYTVN (SEQ ID NO: 1) | LINPYNGGTTYNQKFKG (SEQ ID NO: 9) | SYYSNPYANDY (SEQ ID NO: 34) |
| D24 | GYTVN (SEQ ID NO: 1) | LINPKNGGTTYNQKFKG (SEQ ID NO: 12) | SLYSNPYAMDY (SEQ ID NO: 35) |
| D25 | GYVVN (SEQ ID NO: 2) | LINPYNGGTTYNQKFKG (SEQ ID NO: 9) | SYYSNPYAMDY (SEQ ID NO: 32) |
| D26 | GYTVN (SEQ ID NO: 1) | LINPYNGRTTYNQKFKG (SEQ ID NO: 13) | SYYSNPYANDY (SEQ ID NO: 34) |
| D27 | GYTVN (SEQ ID NO: 1) | LIVPYNGGTTYNQKFKG (SEQ ID NO: 14) | SGYSNPYAMDY (SEQ ID NO: 36) |
| D28 | GYTVN (SEQ ID NO: 1) | LINPYSGGTTYNQKFKG (SEQ ID NO: 15) | SYYSRPYAMDY (SEQ ID NO: 37) |
| D29 | GYTVN (SEQ ID NO: 1) | LINPMNGGTTYNQKFKG (SEQ ID NO: 16) | SYYSNPYAGDY (SEQ ID NO: 38) |
| D30 | GYTVN (SEQ ID NO: 1) | LINPYNGGTWYNQKFKG (SEQ ID NO: 17) | SMYSNPYAMDY (SEQ ID NO: 39) |
| D31 | GYTVN (SEQ ID NO: 1) | LINPYNGGTSYNQKFKG (SEQ ID NO: 18) | SYYSNPYAADY (SEQ ID NO: 40) |
| D32 | GYTVN (SEQ ID NO: 1) | LINPYHGGTTYNQKFKG (SEQ ID NO: 19) | SSYSNPYAMDY (SEQ ID NO: 41) |
| D33 | GYTVN (SEQ ID NO: 1) | LINPLNGGTTYNQKFKG (SEQ ID NO: 20) | SYYSTPYAMDY (SEQ ID NO: 42) |
| D34 | GYTVN (SEQ ID NO: 1) | LINPYNGGTTYNQKFKG (SEQ ID NO: 9) | SYYSRPYAMDY (SEQ ID NO: 37) |
| D35 | GYLVN (SEQ ID NO: 3) | LINPYNGGTTYNQKFKG (SEQ ID NO: 9) | SYYSNPYAMDY (SEQ ID NO: 32) |
| D36 | GYTVN (SEQ ID NO: 1) | LINPYNGGTTYNQKFKG (SEQ ID NO: 9) | SRYSNPYAMDY (SEQ ID NO: 43) |
| D37 | GYTVN (SEQ ID NO: 1) | LIAPYNGGTTYNQKFKG (SEQ ID NO: 21) | SYYSNPYAMDY (SEQ ID NO: 32) |
| D38 | GYTVN (SEQ ID NO: 1) | MINPYNGGTTYNQKFKG (SEQ ID NO: 22) | SYYSVPYAMDY (SEQ ID NO: 44) |
| D39 | GYTVN (SEQ ID NO: 1) | LINPYNGGTTYNQKFKG (SEQ ID NO: 9) | SYYSNPYAQDY (SEQ ID NO: 45) |
| D40 | GYTVN (SEQ ID NO: 1) | LINPYTGGTTYNQKFKG (SEQ ID NO: 11) | SYWSNPYAMDY (SEQ ID NO: 46) |
| D41 | GYTVN (SEQ ID NO: 1) | LINPYSGGTTYNQKFKG (SEQ ID NO: 15) | SPYSNPYAMDY (SEQ ID NO: 47) |
| D42 | GYTVN (SEQ ID NO: 1) | LIVPYNGGTTYNQKFKG (SEQ ID NO: 14) | SRYSNPYAMDY (SEQ ID NO: 43) |
| D43 | GYTVN (SEQ ID NO: 1) | LINPTNGGTSYNQKFKG (SEQ ID NO: 23) | SYYSNPYASDY (SEQ ID NO: 48) |
| D44 | GYTVN (SEQ ID NO: 1) | LINPYNGGTTYNQKFKG (SEQ ID NO: 9) | SYYSLPYAMDY (SEQ ID NO: 49) |
| D45 | GYTVN (SEQ ID NO: 1) | LINPYNGGTSYNQKFKG (SEQ ID NO: 18) | SIYSNPYAMDY (SEQ ID NO: 50) |
| D46 | GYTVN (SEQ ID NO: 1) | LINPYSGGTTYNQKFKG (SEQ ID NO: 15) | SYYVNPYAMDY (SEQ ID NO: 51) |
| D47 | GYTVN (SEQ ID NO: 1) | LINPYTGGTTYNQKFKG (SEQ ID NO: 11) | SYYSNPYASDY (SEQ ID NO: 48) |
| D48 | GYTVN (SEQ ID NO: 1) | LINPSNGGTTYNQKFKG (SEQ ID NO: 24) | SYYSNPYANDY (SEQ ID NO: 34) |
| D49 | GYTVN (SEQ ID NO: 1) | LINPYNGGTSYNQKFKG (SEQ ID NO: 18) | SPYSNPYAMDY (SEQ ID NO: 47) |
| D50 | GYTVN (SEQ ID NO: 1) | LINPYNGGTAYNQKFKG (SEQ ID NO: 25) | SYYSDPYANDY (SEQ ID NO: 52) |
| D51 | GYTVN (SEQ ID NO: 1) | LINPENGGTTYNQKFKG (SEQ ID NO: 26) | SYYSNPYANDY (SEQ ID NO: 34) |
| Composite 1 | GYX₁VN (SEQ ID NO: 81) | X₁IX₂PX₃X₄GX₅TX₆YNQKFKG (SEQ ID NO: 82) | SX₁X₂X₃X₄PYAX₅DY (SEQ ID NO: 83) |
| | | | Wherein, X₁ is G, I, L, M, P, R, S, or Y, X₂ is W or Y, X₃ is S or V, X₄ is D, K, L, N, R, T, or V, and X₅ is A, G, M, N, Q, or S |
| | | Wherein, X₁ is L or M, X₂ is A, N, or V, X₃ is E, K, L, M, R, S, T, or Y, X₄ is H, N, S, or T, X₅ is G or R, and X₆ is A, S, T, or W | |
| | Wherein, X₁ is L, T, or V | | |
| D2 | DAYMH (SEQ ID NO: 4) | RIDPANGNTKYDPKFQG (SEQ ID NO: 27) | NYYGSRYPFAY (SEQ ID NO: 53) |
| D3 | DSYMH (SEQ ID NO: 5) | RIDPANGNTKYDPKFQG (SEQ ID NO: 27) | NYYGAAYPFAY (SEQ ID NO: 54) |
| D4 | DSYIH (SEQ ID NO: 6) | RIDPTNGNTNFDPKFRG (SEQ ID NO: 28) | NYYGSRYPFSY (SEQ ID NO: 55) |
| D5 | DHGMH (SEQ ID NO: 7) | VISTSYGVATYNQKFKG (SEQ ID NO: 29) | RDSKSPFDY (SEQ ID NO: 56) |
| D6 | DHAIH (SEQ ID NO: 8) | AISTNYGAATYNQKFKG (SEQ ID NO: 30) | RGSHGDWFAY (SEQ ID NO: 57) |
| D7 | DAYMH (SEQ ID NO: 4) | RIDPANGYTKSDPKFQG (SEQ ID NO: 31) | NYYGSRYPFAY (SEQ ID NO: 53) |
| | | | |

| **Antibody Name** | **LC-CDR1** | **LC-CDR2** | **LC-CDR3** |
|---|---|---|---|
| D1 | KASQNVHTAVA (SEQ ID NO: 58) | LASDRRT (SEQ ID NO: 65) | LQHWNYPLT (SEQ ID NO: 70) |
| D11 | KASQNVHTAVA (SEQ ID NO: 58) | LASDRRT (SEQ ID NO: 65) | LQHWNYPLT (SEQ ID NO: 70) |
| D21 | KASQNVHTRVA (SEQ ID NO: 59) | LASDRRT (SEQ ID NO: 65) | LQHWNYPLT (SEQ ID NO: 70) |
| D22 | KASQNVHTRVA (SEQ ID NO: 59) | LASDRRT (SEQ ID NO: 65) | LQHWTYPLT (SEQ ID NO: 71) |
| D23 | KASQNVHTAVA (SEQ ID NO: 58) | LRSDRRT (SEQ ID NO: 66) | LQAWNYPLT (SEQ ID NO: 72) |
| D24 | KASQNVHTRVA (SEQ ID NO: 59) | LASDRRT (SEQ ID NO: 65) | LQHWNYPLT (SEQ ID NO: 70) |
| D25 | KASQNVHTAVA (SEQ ID NO: 58) | LASDRRT (SEQ ID NO: 65) | LQHWNYPLT (SEQ ID NO: 70) |
| D26 | KASQNVHTRVA (SEQ ID NO: 59) | LASDRRT (SEQ ID NO: 65) | LQHWNYPLT (SEQ ID NO: 70) |
| D27 | KASQNVHTNVA (SEQ ID NO: 60) | LASDRRT (SEQ ID NO: 65) | LQHWNYPLT (SEQ ID NO: 70) |
| D28 | KASQNVHTRVA (SEQ ID NO: 59) | LASDRRT (SEQ ID NO: 65) | LQHWNSPLT (SEQ ID NO: 73) |
| D29 | KASQNVHTRVA (SEQ ID NO: 59) | LASDRRT (SEQ ID NO: 65) | LQHWNRPLT (SEQ ID NO: 74) |
| D30 | KASQNVHTRVA (SEQ ID NO: 59) | LASDRRT (SEQ ID NO: 65) | LQHWNYPLT (SEQ ID NO: 70) |
| D31 | KASQNVHTRVA (SEQ ID NO: 59) | LASDRRT (SEQ ID NO: 65) | LQHWNYPLT (SEQ ID NO: 70) |
| D32 | KASQNVHTRVA (SEQ ID NO: 59) | LASDRRT (SEQ ID NO: 65) | LQHWNYPLT (SEQ ID NO: 70) |
| D33 | KASQNVHTRVA (SEQ ID NO: 59) | LASDRRT (SEQ ID NO: 65) | LQHWNYPLT (SEQ ID NO: 70) |
| D34 | KASQNVHTRVA (SEQ ID NO: 59) | LASDRRT (SEQ ID NO: 65) | LQHWSYPLT (SEQ ID NO: 75) |
| D35 | KASQNVHTAVA (SEQ ID NO: 58) | LASDRRT (SEQ ID NO: 65) | LQHWNYPLT (SEQ ID NO: 70) |
| D36 | KASQNVHTHVA (SEQ ID NO: 61) | LASDRRT (SEQ ID NO: 65) | LQHWNYPLT (SEQ ID NO: 70) |
| D37 | KASQNVHTRVA (SEQ ID NO: 59) | LASDRRT (SEQ ID NO: 65) | LQHWNYPLT (SEQ ID NO: 70) |
| D38 | KASQNVHTRVA (SEQ ID NO: 59) | LASDRRT (SEQ ID NO: 65) | LQHWNYPLT (SEQ ID NO: 70) |
| D39 | KASQNVHTRVA (SEQ ID NO: 59) | LASDRRT (SEQ ID NO: 65) | LQHWNYPLT (SEQ ID NO: 70) |
| D40 | KASQNVHTRVA (SEQ ID NO: 59) | LASDRRT (SEQ ID NO: 65) | LQHWNYPLT (SEQ ID NO: 70) |
| D41 | KASQNVHTRVA (SEQ ID NO: 59) | LASDRRT (SEQ ID NO: 65) | LQHWNAPLT (SEQ ID NO: 76) |
| D42 | KASQNVHTRVA (SEQ ID NO: 59) | LASDRRT (SEQ ID NO: 65) | LQHWNYPLT (SEQ ID NO: 70) |
| D43 | KASQNVHTRVA (SEQ ID NO: 59) | LASDRRT (SEQ ID NO: 65) | LQHWNYPLT (SEQ ID NO: 70) |
| D44 | KASQNVHTRVA (SEQ ID NO: 59) | LASDRRT (SEQ ID NO: 65) | LQHWNYPLT (SEQ ID NO: 70) |
| D45 | KASQNVHTRVA (SEQ ID NO: 59) | LASDRRT (SEQ ID NO: 65) | LQHWNQPLT (SEQ ID NO: 77) |
| D46 | KASQNVHTRVA (SEQ ID NO: 59) | LASDRRT (SEQ ID NO: 65) | LQHWNYPLT (SEQ ID NO: 70) |
| D47 | KASQNVHTRVA (SEQ ID NO: 59) | LASDRRT (SEQ ID NO: 65) | LQHWTYPLT (SEQ ID NO: 71) |
| D48 | KASQNVHTHVA (SEQ ID NO: 61) | LASDRRT (SEQ ID NO: 65) | LQHWNYPLT (SEQ ID NO: 70) |
| D49 | KASQNVHTRVA (SEQ ID NO: 59) | LASDRRT (SEQ ID NO: 65) | LQHWTYPLT (SEQ ID NO: 71) |
| D50 | KASQNVHTRVA (SEQ ID NO: 59) | LASDRRT (SEQ ID NO: 65) | LQHWNYPLT (SEQ ID NO: 70) |
| D51 | KASQNVHTRVA (SEQ ID NO: 59) | LASDRRT (SEQ ID NO: 65) | LQHWNYPLT (SEQ ID NO: 70) |
| Composite 1 | KASQNVHTX₁V A (SEQ ID NO: 84) | LX₁SDRRT (SEQ ID NO: 85) | LQX₁WX₂X₃PLT (SEQ ID NO: 86) |
| | | | Wherein, X₁ is A or H, X₂ is N, S, or T, and X₃ is A, Q, R, S, or Y |
| | Wherein, X₁ is A, H, N, or R | Wherein, X₁ is A or R | |
| D2 | SASSSVSYMH (SEQ ID NO: 62) | STSNLAS (SEQ ID NO: 67) | QQGSSYPLT (SEQ ID NO: 78) |
| D3 | SASSSVSYMH (SEQ ID NO: 62) | STSNLAS (SEQ ID NO: 67) | QQGSSYPLT (SEQ ID NO: 78) |
| D4 | SGSSSVSYMH (SEQ ID NO: 63) | FTSNLAS (SEQ ID NO: 68) | QQGSTYPLT (SEQ ID NO: 79) |
| D5 | SASSSISSNYLH (SEQ ID NO: 64) | RTSNLAS (SEQ ID NO: 69) | HQYHRSPLT (SEQ ID NO: 80) |
| D6 | SASSSVSYMH (SEQ ID NO: 62) | STSNLAS (SEQ ID NO: 67) | HQYHRSPLT (SEQ ID NO: 80) |
| D7 | SASSSVSYMH (SEQ ID NO: 62) | FTSNLAS (SEQ ID NO: 68) | QQGSTYPLT (SEQ ID NO: 79) |

**Table 3. Exemplary sequences.**

| **SEQ ID NO** | **Descripti on** | **Sequence** |
|---|---|---|
| 87 | D1 V_{H} | EVQLQQSGPDLVKPGASMKISCKASVYSFTGYTVNWVKQSHGKSLEWIGLI NPYNGGTTYNQKFKGKATLTIDKSSTTAYMELLSLTSEDSAVYYCASSYYS NPYAMDYWGQGTSVTVSS |
| 88 | D 11 V_{H} | QVQLVQSGAEVKKPGASVKVSCKASVYSFTGYTVNWVRQAPGQRLEWM GLINPYNGGTTYNQKFKGRVTLTIDKSASTAYMELSSLRSEDTAVYYCASS YYSNPYAMDYWGQGTLVTVSS |
| 89 | D21 V_{H} | QVQLVQSGAEVKKPGASVKVSCKASVYSFTGYTVNWVRQAPGQRLEWM GLINPRNGGTTYNQKFKGRVTLTIDKSASTAYMELSSLRSEDTAVYYCAWS YYSNPYAMDYWGQGTLVTVSS |
| 90 | D22 V_{H} | QVQLVQSGAEVKKPGASVKVSCKASVYSFTGYTVNWVRQAPGQRLEWM GLINPYTGGTTYNQKFKGRVTLTIDKSASTAYMELSSLRSEDTAVYYCASS YYSKPYAMDYWGQGTLVTVSS |
| 91 | D23 V_{H} | QVQLVQSGAEVKKPGASVKVSCKASVYGFTGYTVNWVRQAPGQRLEWM GLINPYNGGTTYNQKFKGRVTLTIDKSASTAYMELSSLRSEDTAVYYCASS YYSNPYANDYWGQGTLVTVSS |
| 92 | D24 V_{H} | QVQLVQSGAEVKKPGASVKVSCKASVYSFTGYTVNWVRQAPGQRLEWM GLINPKNGGTTYNQKFKGRVTLTIDKSASTAYMELSSLRSEDTAVYYCASS |
| | | LYSNPYAMDYWGQGTLVTVSS |
| 93 | D25 V_{H} | QVQLVQSGAEVKKPGASVKVSCKASVYSFTGYVVNWVRQAPGQRLEWM GLINPYNGGTTYNQKFKGRVTLTIDKSASTAYMELSSLRSEDTAVYYCASS YYSNPYAMDYWGQGTLVTVSS |
| 94 | D26 V_{H} | QVQLVQSGAEVKKPGASVKVSCKASVYSFTGYTVNWVRQAPGQRLEWM GLINPYNGRTTYNQKFKGRVTLTIDKSASTAYMELSSLRSEDTAVYYCASS YYSNPYANDYWGQGTLVTVSS |
| 95 | D27 V_{H} | QVQLVQSGAEVKKPGASVKVSCKASVYSFTGYTVNWVRQAPGQRLEWM GLIVPYNGGTTYNQKFKGRVTLTIDKSASTAYMELSSLRSEDTAVYYCASS GYSNPYAMDYWGQGTLVTVSS |
| 96 | D28 V_{H} | QVQLVQSGAEVKKPGASVKVSCKASVYSFTGYTVNWVRQAPGQRLEWM GLINPYSGGTTYNQKFKGRVTLTIDKSASTAYMELSSLRSEDTAVYYCASS YYSRPYAMDYWGQGTLVTVSS |
| 97 | D29 V_{H} | QVQLVQSGAEVKKPGASVKVSCKASVYSFTGYTVNWVRQAPGQRLEWM GLINPMNGGTTYNQKFKGRVTLTIDKSASTAYMELSSLRSEDTAVYYCASS YYSNPYAGDYWGQGTLVTVSS |
| 98 | D30 V_{H} | QVQLVQSGAEVKKPGASVKVSCKASVYSFTGYTVNWVRQAPGQRLEWM GLINPYNGGTWYNQKFKGRVTLTIDKSASTAYMELSSLRSEDTAVYYCASS MYSNPYAMDYWGQGTLVTVSS |
| 99 | D31 V_{H} | QVQLVQSGAEVKKPGASVKVSCKASVYSFTGYTVNWVRQAPGQRLEWM GLINPYNGGTSYNQKFKGRVTLTIDKSASTAYMELSSLRSEDTAVYYCASS YYSNPYAADYWGQGTLVTVSS |
| 100 | D32 V_{H} | QVQLVQSGAEVKKPGASVKVSCKASVYSFTGYTVNWVRQAPGQRLEWM GLINPYHGGTTYNQKFKGRVTLTIDKSASTAYMELSSLRSEDTAVYYCASS SYSNPYAMDYWGQGTLVTVSS |
| 101 | D33 V_{H} | QVQLVQSGAEVKKPGASVKVSCKASVYSFTGYTVNWVRQAPGQRLEWM GLINPLNGGTTYNQKFKGRVTLTIDKSASTAYMELSSLRSEDTAVYYCASS YYSTPYAMDYWGOGTLVTVSS |
| 102 | D34 V_{H} | QVQLVQSGAEVKKPGASVKVSCKASVYSFTGYTVNWVRQAPGQRLEWM GLINPYNGGTTYNQKFKGRVTLTIDKSASTAYMELSSLRSEDTAVYYCASS YYSRPYAMDYWGQGTLVTVSS |
| 103 | D35 V_{H} | QVQLVQSGAEVKKPGASVKVSCKASVYSFTGYLVNWVRQAPGQRLEWM GLINPYNGGTTYNQKFKGRVTLTIDKSASTAYMELSSLRSEDTAVYYCASS YYSNPYAMDYWGQGTLVTVSS |
| 104 | D36 V_{H} | QVQLVQSGAEVKKPGASVKVSCKASVYSFTGYTVNWVRQAPGQRLEWM GLINPYNGGTTYNQKFKGRVTLTIDKSASTAYMELSSLRSEDTAVYYCASS RYSNPYAMDYWGQGTLVTVSS |
| 105 | D37 V_{H} | QVQLVQSGAEVKKPGASVKVSCKASVYSFTGYTVNWVRQAPGQRLEWM GLIAPYNGGTTYNQKFKGRVTLTIDKSASTAYMELSSLRSEDTAVYYCALS YYSNPYAMDYWGQGTLVTVSS |
| 106 | D38 V_{H} | QVQLVQSGAEVKKPGASVKVSCKASVYSFTGYTVNWVRQAPGQRLEWM GMINPYNGGTTYNQKFKGRVTLTIDKSASTAYMELSSLRSEDTAVYYCASS YYSVPYAMDYWGQGTLVTVSS |
| 107 | D39 V_{H} | QVQLVQSGAEVKKPGASVKVSCKASVYSFTGYTVNWVRQAPGQRLEWM GLINPYNGGTTYNQKFKGRVTLTIDKSASTAYMELSSLRSEDTAVYYCASS YYSNPYAQDYWGQGTLVTVSS |
| 108 | D40 V_{H} | QVQLVQSGAEVKKPGASVKVSCKASVYSFTGYTVNWVRQAPGQRLEWM GLINPYTGGTTYNQKFKGRVTLTIDKSASTAYMELSSLRSEDTAVYYCASS YWSNPYAMDYWGQGTLVTVSS |
| 109 | D41 V_{H} | QVQLVQSGAEVKKPGASVKVSCKASVYSFTGYTVNWVRQAPGQRLEWM GLINPYSGGTTYNQKFKGRVTLTIDKSASTAYMELSSLRSEDTAVYYCASSP YSNPYAMDYWGQGTLVTVSS |
| 110 | D42 V_{H} | QVQLVQSGAEVKKPGASVKVSCKASVYSFTGYTVNWVRQAPGQRLEWM GLIVPYNGGTTYNQKFKGRVTLTIDKSASTAYMELSSLRSEDTAVYYCASS RYSNPYAMDYWGQGTLVTVSS |
| 111 | D43 V_{H} | QVQLVQSGAEVKKPGASVKVSCKASVYSFTGYTVNWVRQAPGQRLEWM |
| | | GLINPTNGGTSYNQKFKGRVTLTIDKSASTAYMELSSLRSEDTAVYYCASS YYSNPYASDYWGQGTLVTVSS |
| 112 | D44 V_{H} | QVQLVQSGAEVKKPGASVKVSCKASVYSFTGYTVNWVRQAPGQRLEWM GLINPYNGGTTYNQKFKGRVTLTIDKSASTAYMELSSLRSEDTAVYYCASS YYSLPYAMDYWGQGTLVTVSS |
| 113 | D45 V_{H} | QVQLVQSGAEVKKPGASVKVSCKASVYSFTGYTVNWVRQAPGQRLEWM GLINPYNGGTSYNQKFKGRVTLTIDKSASTAYMELSSLRSEDTAVYYCASSI YSNPYAMDYWGOGTLVTVSS |
| 114 | D46 V_{H} | QVQLVQSGAEVKKPGASVKVSCKASVYSFTGYTVNWVRQAPGQRLEWM GLINPYSGGTTYNQKFKGRVTLTIDKSASTAYMELSSLRSEDTAVYYCASS YYVNPYAMDYWGQGTLVTVSS |
| 115 | D47 V_{H} | QVQLVQSGAEVKKPGASVKVSCKASVYSFTGYTVNWVRQAPGQRLEWM GLINPYTGGTTYNQKFKGRVTLTIDKSASTAYMELSSLRSEDTAVYYCASS YYSNPYASDYWGQGTLVTVSS |
| 116 | D48 V_{H} | QVQLVQSGAEVKKPGASVKVSCKASVYSFTGYTVNWVRQAPGQRLEWM GLINPSNGGTTYNQKFKGRVTLTIDKSASTAYMELSSLRSEDTAVYYCASS YYSNPYANDYWGOGTLVTVSS |
| 117 | D49 V_{H} | QVQLVQSGAEVKKPGASVKVSCKASVYSFTGYTVNWVRQAPGQRLEWM GLINPYNGGTSYNQKFKGRVTLTIDKSASTAYMELSSLRSEDTAVYYCASS PYSNPYAMDYWGQGTLVTVSS |
| 118 | D50 V_{H} | QVQLVQSGAEVKKPGASVKVSCKASVYSFTGYTVNWVRQAPGQRLEWM GLINPYNGGTAYNQKFKGRVTLTIDKSASTAYMELSSLRSEDTAVYYCASS YYSDPYANDYWGQGTLVTVSS |
| 119 | D51 V_{H} | QVQLVQSGAEVKKPGASVKVSCKASVYSFTGYTVNWVRQAPGQRLEWM GLINPENGGTTYNQKFKGRVTLTIDKSASTAYMELSSLRSEDTAVYYCASS YYSNPYANDYWGQGTLVTVSS |
| 120 | D2 V_{H} | EVHVKQSGAELVKPGASVKLSCTASGFNIKDAYMHWVKQRPEQGLEWIG RIDPANGNTKYDPKFQGKASITTDTSSNTAYLQLSSLTSEDTAVYYCTPNYY GSRYPFAYWGQGTTVTVSS |
| 121 | D3 V_{H} | EVQLQQSGAELVKPGASVKLSCTASGFNIKDSYMHWVKQRPEQGLEWIGR IDPANGNTKYDPKFQGKATITADTSSNTAYLQLSSLTSEDTAVYYCVPNYY GAAYPFAYWGQGTTVTVSS |
| 122 | D4 V_{H} | EVMLVESGAELVRPGASVKLSCTASGFKIKDSYIHWMIQRPEQGLEWIGRI DPTNGNTNFDPKFRGKATLTTDTSSNTAYLQLSSLTSEDTAVYYCVPNYYG SRYPFSYWGQGTTVTVSS |
| 123 | D5 V_{H} | EVQLQQSGAELVSPGVSVKISCKGSGYTFTDHGMHWVKQSHAKSLEWIGV ISTSYGVATYNQKFKGKATLSVDKSSSTAYLELARLTSEDSAIYYCARRDSK SPFDYWGQGTTLTVSS |
| 124 | D6 V_{H} | QVQLQQSGAELVRPGVSVKISCKGSGYTFTDHAIHWVKQSHARRLEWIGAI STNYGAATYNQKFKGKATMTVDKSSSTAYMELARLTSEDSAIYYCARRGS HGDWFAYWGQGTLVTVSA |
| 125 | D7 V_{H} | QIQLLQSGAELVKPGASVKLSCTASGFNIKDAYMHWVKQRPEQGLEWIGRI DPANGYTKSDPKFQGKATITSDTSSNTAYLQLSSLTSEDTAVYYCAPNYYG SRYPFAYWGQGTLVTVSS |
| | | |
| 126 | D1 V_{L} | DIVLNQSQKFMSTSVGDRVSITCKASQNVHTAVAWYQQKPGQSPKALIYL ASDRRTGIPDRFTGSGSGTDFTLTIDNVQSEDLADYFCLQHWNYPLTFGAG TKLELK |
| 127 | D11 V_{L} | EIVMTQSPATLSVSPGERATLSCKASQNVHTAVAWYQQKPGQAPRLLIYLA SDRRTGIPARFSGSGSGTEFTLTISSLQSEDFAVYYCLQHWNYPLTFGQGTK VEIK |
| | D25 V_{L} | |
| | D35 V_{L} | |
| 128 | D21 V_{L} | EIVMTQSPATLSVSPGERATLSCKASQNVHTRVAWYQQKPGQAPRLLIYLA SDRRTGIPARFSGSGSGTEFTLTISSLQSEDFAVYYCLQHWNYPLTFGQGTK VEIK |
| | D24 V_{L} | |
| | D26 V_{L} | |
| | D30 V_{L} | |
| | D31 V_{L} | |
| | D32 V_{L} | |
| | D33 V_{L} | |
| | D37 V_{L} | |
| | D38 V_{L} | |
| | D39 V_{L} | |
| | D40 V_{L} | |
| | D42 V_{L} | |
| | D44 V_{L} | |
| | D46 V_{L} | |
| | D50 V_{L} | |
| | D51 V_{L} | |
| 129 | D22 V_{L} | EIVMTQSPATLSVSPGERATLSCKASQNVHTRVAWYQQKPGQAPRLLIYLA SDRRTGIPARFSGSGSGTEFTLTISSLQSEDFAVYYCLQHWTYPLTFGQGTK VEIK |
| | D47 V_{L} | |
| | D49 V_{L} | |
| 130 | D23 V_{L} | EIVMTQSPATLSVSPGERATLSCKASQNVHTAVAWYQQKPGQAPRLLIYLR SDRRTGIPARFSGSGSGTEFTLTISSLQSEDFAVYYCLQAWNYPLTFGQGTK VEIK |
| 131 | D27 V_{L} | EIVMTQSPATLSVSPGERATLSCKASQNVHTNVAWYQQKPGQAPRLLIYLA SDRRTGIPARFSGSGSGTEFTLTISSLQSEDFAVYYCLQHWNYPLTFGQGTK VEIK |
| 132 | D28 V_{L} | EIVMTQSPATLSVSPGERATLSCKASQNVHTRVAWYQQKPGQAPRLLIYLA SDRRTGIPARFSGSGSGTEFTLTISSLQSEDFAVYYCLQHWNSPLTFGQGTK VEIK |
| 133 | D29 V_{L} | EIVMTQSPATLSVSPGERATLSCKASQNVHTRVAWYQQKPGQAPRLLIYLA SDRRTGIPARFSGSGSGTEFTLTISSLQSEDFAVYYCLQHWNRPLTFGQGTK VEIK |
| 134 | D34 V_{L} | EIVMTQSPATLSVSPGERATLSCKASQNVHTRVAWYQQKPGQAPRLLIYLA SDRRTGIPARFSGSGSGTEFTLTISSLQSEDFAVYYCLQHWSYPLTFGQGTK VEIK |
| 135 | D36 V_{L} | EIVMTQSPATLSVSPGERATLSCKASQNVHTHVAWYQQKPGQAPRLLIYLA SDRRTGIPARFSGSGSGTEFTLTISSLQSEDFAVYYCLQHWNYPLTFGQGTK VEIK |
| | D48 V_{L} | |
| 136 | D41 V_{L} | EIVMTQSPATLSVSPGERATLSCKASQNVHTRVAWYQQKPGQAPRLLIYLA SDRRTGIPARFSGSGSGTEFTLTISSLQSEDFAVYYCLQHWNAPLTFGQGTK VEIK |
| 137 | D43 V_{L} | EIVMTQSPATLSVSPGERATMSCKASQNVHTRVAWYQQKPGQAPRLLIYL ASDRRTGIPARFSGSGSGTEFTLTISSLQSEDFAVYYCLQHWNYPLTFGQGT KVEIK |
| 138 | D45 V_{L} | EIVMTQSPATLSVSPGERATLSCKASQNVHTRVAWYQQKPGQAPRLLIYLA SDRRTGIPARFSGSGSGTEFTLTISSLQSEDFAVYYCLQHWNQPLTFGQGTK VEIK |
| 139 | D2 V_{L} | DIVLTQSPAIMSASPGEKVSITCSASSSVSYMHWFQQKPGTSPKLWIYSTSN LASGVPARFSGSGSGTSYSLTISRMEADDAATYYCQQGSSYPLTFGAGTKL ELK |
| 140 | D3 V_{L} | DIVLIQSPAIMSASPGEKVTITCSASSSVSYMHWFQQKPGTSPKVWIYSTSNL ASGVPARFGGSGSGTSYSLTISRMEAEDAATYYCQQGSSYPLTFGAGTKLE LK |
| 141 | D4 V_{L} | QIVLTQSPAIMSASPGEKVTMTCSGSSSVSYMHWFQQKPGNSPKIWIYFTSN LASGVPARFSGSGSGTSYSLTISRMEAGDAATYYCQQGSTYPLTFGAGTKL ELK |
| 142 | D5 V_{L} | QIVLTQSPAIMSASPGEKITITCSASSSISSNYLHWYQQKPGFSPKLLIYRTSN LASGVPARFSGSGSGTSYSLTISSMEAEDAATYYCHQYHRSPLTFGAGTKLE LK |
| 143 | D6 V_{L} | DIELTQSPAIMSASPGEKVTITCSASSSVSYMHWFQQKPGTSPKLWIYSTSNL ASGVPVRFSGSGSGTSYSLTISSMEAEDAATYYCHQYHRSPLTFGAGTKLEL K |
| 144 | D7 V_{L} | QIVLTQSPAIMSASPGEKVTITCSASSSVSYMHWFQQKPGTSPKIWIYFTSNL |
| | | ASGVPARFSGSGSGTSYSLTISRMEAEDAATYYCQQGSTYPLTFGAGTKLE LK |
| 145 | IgG1 heavy chain constant region | ASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSC DKTHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYK CKVSNKALPAPIEKTISKAKGQPREPQVYTLPPSREEMTKNQVSLTCLVKGF YPSDIAVEWESNGQPENNYKTTPPVLDSDGSFFLYSKLTVDKSRWQQGNVF SCSVMHEALHNHYTQKSLSLSPGK |
| 146 | IgG4 heavy chain constant region | ASTKGPSVFPLAPCSRSTSESTAALGCLVKDYFPEPVTVSWNSGALTSGVHT FPAVLQSSGLYSLSSVVTVPSSSLGTKTYTCNVDHKPSNTKVDKRVESKYG PPCPSCPAPEFLGGPSVFLFPPKPKDTLMISRTPEVTCVVVDVSQEDPEVQFN WYVDGVEVHNAKTKPREEQFNSTYRVVSVLTVLHQDWLNGKEYKCKVS NKGLPSSIEKTISKAKGQPREPQVYTLPPSQEEMTKNQVSLTCLVKGFYPSDI AVEWESNGQPENNYKTTPPVLDSDGSFFLYSRLTVDKSRWQEGNVFSCSV MHEALHNHYTQKSLSLSLGK |
| 147 | Light chain constant region (kappa) | |
| 148 | Light chain constant region (lambda) | GQPKAAPSVTLFPPSSEELQANKATLVCLISDFYPGAVTVAWKADSSPVKA GVETTTPSKQSNNKYAASSYLSLTPEQWKSHRSYSCQVTHEGSTVEKTVAP TECS |
| 149 | Native human CD40 | MVRLPLQCVLWGCLLTAVHPEPPTACREKQYLINSQCCSLCQPGQKLVSDC TEFTETECLPCGESEFLDTWNRETHCHQHKYCDPNLGLRVQQKGTSETDTI CTCEEGWHCTSEACESCVLHRSCSPGFGVKQIATGVSDTICEPCPVGFFSNV SSAFEKCHPWTSCETKDLVVQQAGTNKTDVVCGPQDRLRALVVIPIIFGILF AILLVLVFIKKVAKKPTNKAPHPKQEPQEINFPDDLPGSNTAAPVQETLHGC QPVTQEDGKESRISVQERQ |

### CD40

CD40 is a 45 to 50kD type I membrane protein and a member of the TNFR superfamily.CD40 is expressed on APCs, such as B cells, dendritic cells (DCs), macrophages, and monocytes, as well as on non-immune cells such as epithelial, endothelial, and mesenchymal (fbroblasts, myofbroblasts, synoviocytes, stellate cells, etc.) cells, platelets, and tumors (Schönbeck, U, and P Libby. Cellular and molecular life sciences: CMLS vol. 58,1 (2001): 4-43). Since the CD40 molecule acts as a transmembrane signal transducer that leads to the activation of intracellular kinases and transcription factors within the cell, the engagement of CD40 regulates a wide spectrum of molecular and cellular processes, including the initiation and progression of cellular and humoral adaptive immunity. Since CD40 lacks intrinsic kinase activity in the cytoplasmic tail, its signals are transduced largely through the ligand-dependent recruitment of adaptor proteins of the TNF receptor-associated factor (TRAF) family. TRAFs not only couple CD40 to intracellular signalling components but also trigger the release of this signaling complex from CD40 to the cytosol by operating as E3 ubiquitin ligases to activate proximal protein kinases. That allows the activation of mitogen activated protein kinases (MAPKs) pathways, including the extracellular signal-regulated protein kinase (ERK), c-Jun N-terminal kinase (JNK) and p38 MAPK, the phosphatidylinositol 3-kinase (PI3K) cascade, the transcription factors nuclear factor-kB (NF-kB), and the signal transducer and activator of transcription (STAT) (Loskog, Angelica S I, and Aristides G Eliopoulos. Seminars in immunology vol. 21,5 (2009): 301-7; and Matsuzawa, Atsushi et al. Science (New York, N.Y.) vol. 321,5889 (2008): 663-8). These pathways act in concert to regulate many of the reported activities of CD40 in a cell-type and microenvironment-dependent manner.

Interaction between CD40 and CD40L causes several immunological responses including cellular stress-related gene expression and B cell activation (immunoglobulin class switching, germinal center formation, and cytokine production) (Belkhir, R et al. Scandinavian journal of immunology vol. 79,1 (2014): 37-42; and Bensinger, William et al. British journal of haematology vol. 159,1 (2012): 58-66).CD40-CD40L interaction also plays a role in T cell activation, upregulation of surface molecules relevant to inflammatory responses in chronic inflammatory disorders and expression of cytokines. These include interleukin (IL)-2, IL-12, TNF-α, interferon (IFN)-α, as well as IL-1 and IL-6 (Sidiropoulos, P I, and D T Boumpas. Lupus vol. 13,5 (2004): 391-7).

Considering the crucial role of CD40-CD40L as co-stimulatory molecules between B cells and T cells, there is already interest in the effects of CD40-CD40L antagonists in inflammation or autoimmune conditions (e.g. psoriasis, lupus nephritis, systemic lupus erythematosis (SLE), and rheumatoid arthritis (RA), diabetes mellitus, immunological rejection).

### CD40L

CD40L is a 32- to 39-kDa type II transmembrane protein and a member of the TNF ligand superfamily (TNFSF, which consists of such as TNF, lymphotoxin, CD27L, CD30L, and FasL). CD40L is expressed preferentially by activated CD4+ T cells as well as activated B cells and platelets, although it is also variably induced on monocytic cells, natural killer cells, mast cells, and basophils under inflammatory conditions (Elgueta, Raul et al. Immunological reviews vol. 229,1 (2009): 152-72). A soluble form of CD40L (sCD40L) has been reported to express biological activities similar to the transmembrane form, triggering a CD40L/CD40 signaling cascade and also stabilizing the arterial thrombi by a b3 integrin-dependent mechanism as an aIIbb3 ligand (Graf, D et al. European journal of immunology vol. 25,6 (1995): 1749-54; and André, Patrick et al. Nature medicine vol. 8,3 (2002): 247-52). Moreover, the integrins a5b1 and Mac-1 were also recently identified as novel CD154 receptors expressed on many cell types (Léveillé, Claire et al. The Journal of biological chemistry vol. 282,8 (2007): 5143-51; and Zirlik, Andreas et al. Circulation vol. 115,12 (2007): 1571-80).

Neutralization of CD40 binding to CD40L is therefore a therapeutic approach to treating diseases and conditions mediated through CD40-CD40L pathway.

### Full-length anti-CD40 antibody

The anti-CD40 antibody in some embodiments is a full-length anti-CD40 antibody. In some embodiments, the full-length anti-CD40 antibody is an IgA, IgD, IgE, IgG, or IgM. In some embodiments, the full-length anti-CD40 antibody comprises IgG constant domains, such as constant domains of any one of IgG1, IgG2, IgG3, and IgG4 including variants thereof. In some embodiments, the full-length anti-CD40 antibody comprises a lambda light chain constant region. In some embodiments, the full-length anti-CD40 antibody comprises a kappa light chain constant region. In some embodiments, the full-length anti-CD40 antibody is a full-length human anti-CD40 antibody. In some embodiments, the full-length anti-CD40 antibody comprises an Fc sequence of a mouse immunoglobulin. In some embodiments, the full-length anti-CD40 antibody comprises an Fc sequence that has been altered or otherwise changed so that it has enhanced antibody dependent cellular cytotoxicity (ADCC) or complement dependent cytotoxicity (CDC) effector function.

Thus, for example, in some embodiments, there is provided a full-length anti-CD40 antibody comprising IgG1 constant domains, wherein the anti-CD40 antibody specifically binds to CD40. In some embodiments, the IgG1 is human IgG1. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG2 constant domains, wherein the anti-CD40 antibody specifically binds to CD40. In some embodiments, the IgG2 is human IgG2. In some embodiments, the light chain constant region comprises or consists of the amino acid sequence of SEQ ID NO: 147. In some embodiments, the light chain constant region comprises or consists of the amino acid sequence of SEQ ID NO: 148.

In some embodiments, there is provided a full-length anti-CD40 antibody comprising IgG3 constant domains, wherein the anti-CD40 antibody specifically binds to CD40. In some embodiments, the IgG3 is human IgG3. In some embodiments, the light chain constant region comprises or consists of the amino acid sequence of SEQ ID NO: 147. In some embodiments, the light chain constant region comprises or consists of the amino acid sequence of SEQ ID NO: 148.

In some embodiments, there is provided a full-length anti-CD40 antibody comprising IgG4 constant domains, wherein the anti-CD40 antibody specifically binds to CD40. In some embodiments, the IgG4 is human IgG4. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a) a heavy chain variable domain comprising an HC-CDR1 comprising the amino acid sequence of any one of SEQ ID NOs: 1-8, or a variant thereof comprising up to about 3 (such as about any of 1, 2, or 3) amino acid substitutions, an HC-CDR2 comprising the amino acid sequence of any one of SEQ ID NOs: 9-31, or a variant thereof comprising up to about 3 (such as about any of 1, 2, or 3) amino acid substitutions, and an HC-CDR3 comprising the amino acid sequence of any one of SEQ ID NOs: 32-57, or a variant thereof comprising up to about 3 (such as about any of 1, 2, or 3) amino acid substitutions; and b) a light chain variable domain comprising an LC-CDR1 comprising the amino acid sequence of any one of SEQ ID NOs: 58-64, or a variant thereof comprising up to about 3 (such as about any of 1, 2, or 3) amino acid substitutions, an LC-CDR2 comprising the amino acid sequence of any one of SEQ ID NOs: 65-69, or a variant thereof comprising up to about 3 (such as about any of 1, 2, or 3) amino acid substitutions, and an LC-CDR3 comprising the amino acid sequence of any one of SEQ ID NOs: 70-80, or a variant thereof comprising up to about 3 (such as about any of 1, 2, or 3) amino acid substitutions. In some embodiments, the IgG1 is human IgG1. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG2 constant domains, wherein the anti-CD40 antibody comprises a) a heavy chain variable domain comprising an HC-CDR1 comprising the amino acid sequence of any one of SEQ ID NOs: 1-8, or a variant thereof comprising up to about 3 (such as about any of 1, 2, or 3) amino acid substitutions, an HC-CDR2 comprising the amino acid sequence of any one of SEQ ID NOs: 9-31, or a variant thereof comprising up to about 3 (such as about any of 1, 2, or 3) amino acid substitutions, and an HC-CDR3 comprising the amino acid sequence of any one of SEQ ID NOs: 32-57, or a variant thereof comprising up to about 3 (such as about any of 1, 2, or 3) amino acid substitutions; and b) a light chain variable domain comprising an LC-CDR1 comprising the amino acid sequence of any one of SEQ ID NOs: 58-64, or a variant thereof comprising up to about 3 (such as about any of 1, 2, or 3) amino acid substitutions, an LC-CDR2 comprising the amino acid sequence of any one of SEQ ID NOs: 65-69, or a variant thereof comprising up to about 3 (such as about any of 1, 2, or 3) amino acid substitutions, and an LC-CDR3 comprising the amino acid sequence of any one of SEQ ID NOs: 70-80, or a variant thereof comprising up to about 3 (such as about any of 1, 2, or 3) amino acid substitutions. In some embodiments, the IgG2 is human IgG2. In some embodiments, the light chain constant region comprises or consists of the amino acid sequence of SEQ ID NO: 147. In some embodiments, the light chain constant region comprises or consists of the amino acid sequence of SEQ ID NO: 148.

In some embodiments, there is provided a full-length anti-CD40 antibody comprising IgG3 constant domains, wherein the anti-CD40 antibody comprises a) a heavy chain variable domain comprising an HC-CDR1 comprising the amino acid sequence of any one of SEQ ID NOs: 1-8, or a variant thereof comprising up to about 3 (such as about any of 1, 2, or 3) amino acid substitutions, an HC-CDR2 comprising the amino acid sequence of any one of SEQ ID NOs: 9-31, or a variant thereof comprising up to about 3 (such as about any of 1, 2, or 3) amino acid substitutions, and an HC-CDR3 comprising the amino acid sequence of any one of SEQ ID NOs: 32-57, or a variant thereof comprising up to about 3 (such as about any of 1, 2, or 3) amino acid substitutions; and b) a light chain variable domain comprising an LC-CDR1 comprising the amino acid sequence of any one of SEQ ID NOs: 58-64, or a variant thereof comprising up to about 3 (such as about any of 1, 2, or 3) amino acid substitutions, an LC-CDR2 comprising the amino acid sequence of any one of SEQ ID NOs: 65-69, or a variant thereof comprising up to about 3 (such as about any of 1, 2, or 3) amino acid substitutions, and an LC-CDR3 comprising the amino acid sequence of any one of SEQ ID NOs: 70-80, or a variant thereof comprising up to about 3 (such as about any of 1, 2, or 3) amino acid substitutions. In some embodiments, the IgG3 is human IgG3. In some embodiments, the light chain constant region comprises or consists of the amino acid sequence of SEQ ID NO: 147. In some embodiments, the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a) a heavy chain variable domain comprising an HC-CDR1 comprising the amino acid sequence of any one of SEQ ID NOs: 1-8, or a variant thereof comprising up to about 3 (such as about any of 1, 2, or 3) amino acid substitutions, an HC-CDR2 comprising the amino acid sequence of any one of SEQ ID NOs: 9-31, or a variant thereof comprising up to about 3 (such as about any of 1, 2, or 3) amino acid substitutions, and an HC-CDR3 comprising the amino acid sequence of any one of SEQ ID NOs: 32-57, or a variant thereof comprising up to about 3 (such as about any of 1, 2, or 3) amino acid substitutions; and b) a light chain variable domain comprising an LC-CDR1 comprising the amino acid sequence of any one of SEQ ID NOs: 58-64, or a variant thereof comprising up to about 3 (such as about any of 1, 2, or 3) amino acid substitutions, an LC-CDR2 comprising the amino acid sequence of any one of SEQ ID NOs: 65-69, or a variant thereof comprising up to about 3 (such as about any of 1, 2, or 3) amino acid substitutions, and an LC-CDR3 comprising the amino acid sequence of any one of SEQ ID NOs: 70-80, or a variant thereof comprising up to about 3 (such as about any of 1, 2, or 3) amino acid substitutions. In some embodiments, the IgG4 is human IgG4. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG 1 constant domains, wherein the anti-CD40 antibody comprises a) a heavy chain variable domain comprising an HC-CDR1 comprising the amino acid sequence of any one of SEQ ID NOs: 1-8, an HC-CDR2 comprising the amino acid sequence of any one of SEQ ID NOs: 9-31, and an HC-CDR3 comprising the amino acid sequence of any one of SEQ ID NOs: 32-57, or a variant thereof comprising up to about 5 (such as about any of 1, 2, 3, 4, or 5) amino acid substitutions in the HC-CDR sequences; and b) a light chain variable domain comprising an LC-CDR1 comprising the amino acid sequence of any one of SEQ ID NOs: 58-64, an LC-CDR2 comprising the amino acid sequence of any one of SEQ ID NOs: 65-69, and an LC-CDR3 comprising the amino acid sequence of any one of SEQ ID NOs: 70-80, or a variant thereof comprising up to about 5 (such as about any of 1, 2, 3, 4, or 5) amino acid substitutions in the LC-CDR sequences. In some embodiments, the IgG1 is human IgG1. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a) a heavy chain variable domain comprising an HC-CDR1 comprising the amino acid sequence of any one of SEQ ID NOs: 1-8, an HC-CDR2 comprising the amino acid sequence of any one of SEQ ID NOs: 9-31, and an HC-CDR3 comprising the amino acid sequence of any one of SEQ ID NOs: 32-57, or a variant thereof comprising up to about 5 (such as about any of 1, 2, 3, 4, or 5) amino acid substitutions in the HC-CDR sequences; and b) a light chain variable domain comprising an LC-CDR1 comprising the amino acid sequence of any one of SEQ ID NOs: 58-64, an LC-CDR2 comprising the amino acid sequence of any one of SEQ ID NOs: 65-69, and an LC-CDR3 comprising the amino acid sequence of any one of SEQ ID NOs: 70-80, or a variant thereof comprising up to about 5 (such as about any of 1, 2, 3, 4, or 5) amino acid substitutions in the LC-CDR sequences. In some embodiments, the IgG4 is human IgG4. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a) a heavy chain variable domain comprising an HC-CDR1 comprising the amino acid sequence of any one of SEQ ID NOs: 1-8, an HC-CDR2 comprising the amino acid sequence of any one of SEQ ID NOs: 9-31, and an HC-CDR3 comprising the amino acid sequence of any one of SEQ ID NOs: 32-57; and b) a light chain variable domain comprising an LC-CDR1 comprising the amino acid sequence of any one of SEQ ID NOs: 58-64, an LC-CDR2 comprising the amino acid sequence of any one of SEQ ID NOs: 65-69, and an LC-CDR3 comprising the amino acid sequence of any one of SEQ ID NOs: 70-80. In some embodiments, the IgG1 is human IgG1. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a) a heavy chain variable domain comprising an HC-CDR1 comprising the amino acid sequence of any one of SEQ ID NOs: 1-8, an HC-CDR2 comprising the amino acid sequence of any one of SEQ ID NOs: 9-31, and an HC-CDR3 comprising the amino acid sequence of any one of SEQ ID NOs: 32-57; and b) a light chain variable domain comprising an LC-CDR1 comprising the amino acid sequence of any one of SEQ ID NOs: 58-64, an LC-CDR2 comprising the amino acid sequence of any one of SEQ ID NOs: 65-69, and an LC-CDR3 comprising the amino acid sequence of any one of SEQ ID NOs: 70-80. In some embodiments, the IgG4 is human IgG4. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a) a heavy chain variable domain comprising an HC-CDR1 comprising the amino acid sequence of SEQ ID NO: 1, an HC-CDR2 comprising the amino acid sequence of SEQ ID NO: 9, and an HC-CDR3 comprising the amino acid sequence of SEQ ID NO: 32; and b) a light chain variable domain comprising an LC-CDR1 comprising the amino acid sequence of SEQ ID NO: 58, an LC-CDR2 comprising the amino acid sequence of SEQ ID NO: 65, and an LC-CDR3 comprising the amino acid sequence of SEQ ID NO: 70. In some embodiments, the IgG1 is human IgG1. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG 1 constant domains, wherein the anti-CD40 antibody comprises a) a heavy chain variable domain comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 32; and b) a light chain variable domain comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70. In some embodiments, the IgG1 is human IgG1. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a) a heavy chain variable domain comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 34; and b) a light chain variable domain comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70. In some embodiments, the IgG1 is human IgG1. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a) a heavy chain variable domain comprising an HC-CDR1 comprising the amino acid sequence of SEQ ID NO: 1, an HC-CDR2 comprising the amino acid sequence of SEQ ID NO: 15, and an HC-CDR3 comprising the amino acid sequence of SEQ ID NO: 37; and b) a light chain variable domain comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 73. In some embodiments, the IgG1 is human IgG1. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a) a heavy chain variable domain comprising an HC-CDR1 comprising the amino acid sequence of SEQ ID NO: 1, an HC-CDR2 comprising the amino acid sequence of SEQ ID NO: 20, and an HC-CDR3 comprising the amino acid sequence of SEQ ID NO: 42; and b) a light chain variable domain comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70. In some embodiments, the IgG1 is human IgG1. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a) a heavy chain variable domain comprising an HC-CDR1 comprising the amino acid sequence of SEQ ID NO: 1, an HC-CDR2 comprising the amino acid sequence of SEQ ID NO: 14, and an HC-CDR3 comprising the amino acid sequence of SEQ ID NO: 43; and b) a light chain variable domain comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70. In some embodiments, the IgG1 is human IgG1. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a) a heavy chain variable domain comprising an HC-CDR1 comprising the amino acid sequence of SEQ ID NO: 1, an HC-CDR2 comprising the amino acid sequence of SEQ ID NO: 15, and an HC-CDR3 comprising the amino acid sequence of SEQ ID NO: 51; and b) a light chain variable domain comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70. In some embodiments, the IgG1 is human IgG1. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a) a heavy chain variable domain comprising an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 48; and b) a light chain variable domain comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 71. In some embodiments, the IgG1 is human IgG1. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a) a heavy chain variable domain comprising an HC-CDR1 comprising the amino acid sequence of SEQ ID NO: 1, an HC-CDR2 comprising the amino acid sequence of SEQ ID NO: 18, and an HC-CDR3 comprising the amino acid sequence of SEQ ID NO: 47; and b) a light chain variable domain comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 71. In some embodiments, the IgG1 is human IgG1. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG 1 constant domains, wherein the anti-CD40 antibody comprises a) a heavy chain variable domain comprising an HC-CDR1 comprising the amino acid sequence of SEQ ID NO: 4, an HC-CDR2 comprising the amino acid sequence of SEQ ID NO: 27, and an HC-CDR3 comprising the amino acid sequence of SEQ ID NO: 53; and b) a light chain variable domain comprising an LC-CDR1 comprising the amino acid sequence of SEQ ID NO: 62, an LC-CDR2 comprising the amino acid sequence of SEQ ID NO: 67, and an LC-CDR3 comprising the amino acid sequence of SEQ ID NO: 78. In some embodiments, the IgG1 is human IgG1. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG 1 constant domains, wherein the anti-CD40 antibody comprises a) a heavy chain variable domain comprising an HC-CDR1 comprising the amino acid sequence of SEQ ID NO: 5, an HC-CDR2 comprising the amino acid sequence of SEQ ID NO: 27, and an HC-CDR3 comprising the amino acid sequence of SEQ ID NO: 54; and b) a light chain variable domain comprising an LC-CDR1 comprising the amino acid sequence of SEQ ID NO: 62, an LC-CDR2 comprising the amino acid sequence of SEQ ID NO: 67, and an LC-CDR3 comprising the amino acid sequence of SEQ ID NO: 78. In some embodiments, the IgG1 is human IgG1. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a) a heavy chain variable domain comprising an HC-CDR1 comprising the amino acid sequence of SEQ ID NO: 6, an HC-CDR2 comprising the amino acid sequence of SEQ ID NO: 28, and an HC-CDR3 comprising the amino acid sequence of SEQ ID NO: 55; and b) a light chain variable domain comprising an LC-CDR1 comprising the amino acid sequence of SEQ ID NO: 63, an LC-CDR2 comprising the amino acid sequence of SEQ ID NO: 68, and an LC-CDR3 comprising the amino acid sequence of SEQ ID NO: 79. In some embodiments, the IgG1 is human IgG1. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a) a heavy chain variable domain comprising an HC-CDR1 comprising the amino acid sequence of SEQ ID NO: 7, an HC-CDR2 comprising the amino acid sequence of SEQ ID NO: 29, and an HC-CDR3 comprising the amino acid sequence of SEQ ID NO: 56; and b) a light chain variable domain comprising an LC-CDR1 comprising the amino acid sequence of SEQ ID NO: 64, an LC-CDR2 comprising the amino acid sequence of SEQ ID NO: 69, and an LC-CDR3 comprising the amino acid sequence of SEQ ID NO: 80. In some embodiments, the IgG1 is human IgG1. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a) a heavy chain variable domain comprising an HC-CDR1 comprising the amino acid sequence of SEQ ID NO: 8, an HC-CDR2 comprising the amino acid sequence of SEQ ID NO: 30, and an HC-CDR3 comprising the amino acid sequence of SEQ ID NO: 57; and b) a light chain variable domain comprising an LC-CDR1 comprising the amino acid sequence of SEQ ID NO: 62, an LC-CDR2 comprising the amino acid sequence of SEQ ID NO: 67, and an LC-CDR3 comprising the amino acid sequence of SEQ ID NO: 80. In some embodiments, the IgG1 is human IgG1. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG 1 constant domains, wherein the anti-CD40 antibody comprises a) a heavy chain variable domain comprising an HC-CDR1 comprising the amino acid sequence of SEQ ID NO: 4, an HC-CDR2 comprising the amino acid sequence of SEQ ID NO: 31, and an HC-CDR3 comprising the amino acid sequence of SEQ ID NO: 53; and b) a light chain variable domain comprising an LC-CDR1 comprising the amino acid sequence of SEQ ID NO: 62, an LC-CDR2 comprising the amino acid sequence of SEQ ID NO: 68, and an LC-CDR3 comprising the amino acid sequence of SEQ ID NO: 79. In some embodiments, the IgG1 is human IgG1. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a) a heavy chain variable domain comprising an HC-CDR1 comprising the amino acid sequence of SEQ ID NO: 1, an HC-CDR2 comprising the amino acid sequence of SEQ ID NO: 9, and an HC-CDR3 comprising the amino acid sequence of SEQ ID NO: 32; and b) a light chain variable domain comprising an LC-CDR1 comprising the amino acid sequence of SEQ ID NO: 58, an LC-CDR2 comprising the amino acid sequence of SEQ ID NO: 65, and an LC-CDR3 comprising the amino acid sequence of SEQ ID NO: 70. In some embodiments, the IgG4 is human IgG4. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a) a heavy chain variable domain comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 32; and b) a light chain variable domain comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70. In some embodiments, the IgG4 is human IgG4. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a) a heavy chain variable domain comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 34; and b) a light chain variable domain comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70. In some embodiments, the IgG4 is human IgG4. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a) a heavy chain variable domain comprising an HC-CDR1 comprising the amino acid sequence of SEQ ID NO: 1, an HC-CDR2 comprising the amino acid sequence of SEQ ID NO: 15, and an HC-CDR3 comprising the amino acid sequence of SEQ ID NO: 37; and b) a light chain variable domain comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 73. In some embodiments, the IgG4 is human IgG4. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a) a heavy chain variable domain comprising an HC-CDR1 comprising the amino acid sequence of SEQ ID NO: 1, an HC-CDR2 comprising the amino acid sequence of SEQ ID NO: 20, and an HC-CDR3 comprising the amino acid sequence of SEQ ID NO: 42; and b) a light chain variable domain comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70. In some embodiments, the IgG4 is human IgG4. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a) a heavy chain variable domain comprising an HC-CDR1 comprising the amino acid sequence of SEQ ID NO: 1, an HC-CDR2 comprising the amino acid sequence of SEQ ID NO: 14, and an HC-CDR3 comprising the amino acid sequence of SEQ ID NO: 43; and b) a light chain variable domain comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70. In some embodiments, the IgG4 is human IgG4. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a) a heavy chain variable domain comprising an HC-CDR1 comprising the amino acid sequence of SEQ ID NO: 1, an HC-CDR2 comprising the amino acid sequence of SEQ ID NO: 15, and an HC-CDR3 comprising the amino acid sequence of SEQ ID NO: 51; and b) a light chain variable domain comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70. In some embodiments, the IgG4 is human IgG4. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a) a heavy chain variable domain comprising an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 48; and b) a light chain variable domain comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 71. In some embodiments, the IgG4 is human IgG4. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a) a heavy chain variable domain comprising an HC-CDR1 comprising the amino acid sequence of SEQ ID NO: 1, an HC-CDR2 comprising the amino acid sequence of SEQ ID NO: 18, and an HC-CDR3 comprising the amino acid sequence of SEQ ID NO: 47; and b) a light chain variable domain comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 71. In some embodiments, the IgG4 is human IgG4. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a) a heavy chain variable domain comprising an HC-CDR1 comprising the amino acid sequence of SEQ ID NO: 4, an HC-CDR2 comprising the amino acid sequence of SEQ ID NO: 27, and an HC-CDR3 comprising the amino acid sequence of SEQ ID NO: 53; and b) a light chain variable domain comprising an LC-CDR1 comprising the amino acid sequence of SEQ ID NO: 62, an LC-CDR2 comprising the amino acid sequence of SEQ ID NO: 67, and an LC-CDR3 comprising the amino acid sequence of SEQ ID NO: 78. In some embodiments, the IgG4 is human IgG4. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a) a heavy chain variable domain comprising an HC-CDR1 comprising the amino acid sequence of SEQ ID NO: 5, an HC-CDR2 comprising the amino acid sequence of SEQ ID NO: 27, and an HC-CDR3 comprising the amino acid sequence of SEQ ID NO: 54; and b) a light chain variable domain comprising an LC-CDR1 comprising the amino acid sequence of SEQ ID NO: 62, an LC-CDR2 comprising the amino acid sequence of SEQ ID NO: 67, and an LC-CDR3 comprising the amino acid sequence of SEQ ID NO: 78. In some embodiments, the IgG4 is human IgG4. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a) a heavy chain variable domain comprising an HC-CDR1 comprising the amino acid sequence of SEQ ID NO: 6, an HC-CDR2 comprising the amino acid sequence of SEQ ID NO: 28, and an HC-CDR3 comprising the amino acid sequence of SEQ ID NO: 55; and b) a light chain variable domain comprising an LC-CDR1 comprising the amino acid sequence of SEQ ID NO: 63, an LC-CDR2 comprising the amino acid sequence of SEQ ID NO: 68, and an LC-CDR3 comprising the amino acid sequence of SEQ ID NO: 79. In some embodiments, the IgG4 is human IgG4. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a) a heavy chain variable domain comprising an HC-CDR1 comprising the amino acid sequence of SEQ ID NO: 7, an HC-CDR2 comprising the amino acid sequence of SEQ ID NO: 29, and an HC-CDR3 comprising the amino acid sequence of SEQ ID NO: 56; and b) a light chain variable domain comprising an LC-CDR1 comprising the amino acid sequence of SEQ ID NO: 64, an LC-CDR2 comprising the amino acid sequence of SEQ ID NO: 69, and an LC-CDR3 comprising the amino acid sequence of SEQ ID NO: 80. In some embodiments, the IgG4 is human IgG4. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a) a heavy chain variable domain comprising an HC-CDR1 comprising the amino acid sequence of SEQ ID NO: 8, an HC-CDR2 comprising the amino acid sequence of SEQ ID NO: 30, and an HC-CDR3 comprising the amino acid sequence of SEQ ID NO: 57; and b) a light chain variable domain comprising an LC-CDR1 comprising the amino acid sequence of SEQ ID NO: 62, an LC-CDR2 comprising the amino acid sequence of SEQ ID NO: 67, and an LC-CDR3 comprising the amino acid sequence of SEQ ID NO: 80. In some embodiments, the IgG4 is human IgG4. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a) a heavy chain variable domain comprising an HC-CDR1 comprising the amino acid sequence of SEQ ID NO: 4, an HC-CDR2 comprising the amino acid sequence of SEQ ID NO: 31, and an HC-CDR3 comprising the amino acid sequence of SEQ ID NO: 53; and b) a light chain variable domain comprising an LC-CDR1 comprising the amino acid sequence of SEQ ID NO: 62, an LC-CDR2 comprising the amino acid sequence of SEQ ID NO: 68, and an LC-CDR3 comprising the amino acid sequence of SEQ ID NO: 79. In some embodiments, the IgG4 is human IgG4. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a heavy chain variable domain comprising the amino acid sequence of any one of SEQ ID NOs: 87-125, or a variant thereof having at least about 90% (such as at least about any of 91%, 92%, 93%, 94%, 95%, 96%, 97%, 98%, or 99%) sequence identity, and a light chain variable domain comprising the amino acid sequence of any one of SEQ ID NOs: 126-144, or a variant thereof having at least about 90% (such as at least about any of 91%, 92%, 93%, 94%, 95%, 96%, 97%, 98%, or 99%) sequence identity. In some embodiments, the IgG1 is human IgG1. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG2 constant domains, wherein the anti-CD40 antibody comprises a heavy chain variable domain comprising the amino acid sequence of any one of SEQ ID NOs: 87-125, or a variant thereof having at least about 90% (such as at least about any of 91%, 92%, 93%, 94%, 95%, 96%, 97%, 98%, or 99%) sequence identity, and a light chain variable domain comprising the amino acid sequence of any one of SEQ ID NOs: 126-144, or a variant thereof having at least about 90% (such as at least about any of 91%, 92%, 93%, 94%, 95%, 96%, 97%, 98%, or 99%) sequence identity. In some embodiments, the IgG2 is human IgG2. In some embodiments, the light chain constant region comprises or consists of the amino acid sequence of SEQ ID NO: 147. In some embodiments, the light chain constant region comprises or consists of the amino acid sequence of SEQ ID NO: 148.

In some embodiments, there is provided a full-length anti-CD40 antibody comprising IgG3 constant domains, wherein the anti-CD40 antibody comprises a heavy chain variable domain comprising the amino acid sequence of any one of SEQ ID NOs: 87-125, or a variant thereof having at least about 90% (such as at least about any of 91%, 92%, 93%, 94%, 95%, 96%, 97%, 98%, or 99%) sequence identity, and a light chain variable domain comprising the amino acid sequence of any one of SEQ ID NOs: 126-144, or a variant thereof having at least about 90% (such as at least about any of 91%, 92%, 93%, 94%, 95%, 96%, 97%, 98%, or 99%) sequence identity. In some embodiments, the IgG3 is human IgG3. In some embodiments, the light chain constant region comprises or consists of the amino acid sequence of SEQ ID NO: 147. In some embodiments, the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a heavy chain variable domain comprising the amino acid sequence of any one of SEQ ID NOs: 87-125, or a variant thereof having at least about 90% (such as at least about any of 91%, 92%, 93%, 94%, 95%, 96%, 97%, 98%, or 99%) sequence identity, and a light chain variable domain comprising the amino acid sequence of any one of SEQ ID NOs: 126-144, or a variant thereof having at least about 90% (such as at least about any of 91%, 92%, 93%, 94%, 95%, 96%, 97%, 98%, or 99%) sequence identity. In some embodiments, the IgG4 is human IgG4. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a heavy chain variable domain comprising the amino acid sequence of any one of SEQ ID NOs: 87-125, and a light chain variable domain comprising the amino acid sequence of any one of SEQ ID NOs: 126-144. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a heavy chain variable domain comprising the amino acid sequence of any one of SEQ ID NOs: 87-125, and a light chain variable domain comprising the amino acid sequence of any one of SEQ ID NOs: 126-144. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a heavy chain variable domain comprising the amino acid sequence of SEQ ID NO: 87, or a variant thereof having at least about 90% sequence identity to the amino acid sequence of SEQ ID NO: 87; and a light chain variable domain comprising the amino acid sequence of SEQ ID NO: 126, or a variant thereof having at least about 90% sequence identity to the amino acid sequence of SEQ ID NO: 126. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a heavy chain variable domain comprising the amino acid sequence of SEQ ID NO: 88 , or a variant thereof having at least about 90% sequence identity to the amino acid sequence of SEQ ID NO: 88; and a light chain variable domain comprising the amino acid sequence of SEQ ID NO: 127, or a variant thereof having at least about 90% sequence identity to the amino acid sequence of SEQ ID NO: 127. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a heavy chain variable domain comprising the amino acid sequence of SEQ ID NO: 89, or a variant thereof having at least about 90% sequence identity to the amino acid sequence of SEQ ID NO: 89; and a light chain variable domain comprising the amino acid sequence of SEQ ID NO: 128, or a variant thereof having at least about 90% sequence identity to the amino acid sequence of SEQ ID NO:128. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a heavy chain variable domain comprising the amino acid sequence of SEQ ID NO: 94, or a variant thereof having at least about 90% sequence identity to the amino acid sequence of SEQ ID NO: 94; and a light chain variable domain comprising the amino acid sequence of SEQ ID NO: 128, or a variant thereof having at least about 90% sequence identity to the amino acid sequence of SEQ ID NO: 128. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a heavy chain variable domain comprising the amino acid sequence of SEQ ID NO: 96, or a variant thereof having at least about 90% sequence identity to the amino acid sequence of SEQ ID NO: 96; and a light chain variable domain comprising the amino acid sequence of SEQ ID NO: 132, or a variant thereof having at least about 90% sequence identity to the amino acid sequence of SEQ ID NO: 132. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a heavy chain variable domain comprising the amino acid sequence of SEQ ID NO: 101, or a variant thereof having at least about 90% sequence identity to the amino acid sequence of SEQ ID NO: 101; and a light chain variable domain comprising the amino acid sequence of SEQ ID NO: 128, or a variant thereof having at least about 90% sequence identity to the amino acid sequence of SEQ ID NO: 128. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a heavy chain variable domain comprising the amino acid sequence of SEQ ID NO: 110, or a variant thereof having at least about 90% sequence identity to the amino acid sequence of SEQ ID NO: 110; and a light chain variable domain comprising the amino acid sequence of SEQ ID NO: 128, or a variant thereof having at least about 90% sequence identity to the amino acid sequence of SEQ ID NO: 128. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a heavy chain variable domain comprising the amino acid sequence of SEQ ID NO: 114, or a variant thereof having at least about 90% sequence identity to the amino acid sequence of SEQ ID NO:114; and a light chain variable domain comprising the amino acid sequence of SEQ ID NO: 128, or a variant thereof having at least about 90% sequence identity to the amino acid sequence of SEQ ID NO: 128. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a heavy chain variable domain comprising the amino acid sequence of SEQ ID NO: 115, or a variant thereof having at least about 90% sequence identity to the amino acid sequence of SEQ ID NO: 115; and a light chain variable domain comprising the amino acid sequence of SEQ ID NO: 129, or a variant thereof having at least about 90% sequence identity to the amino acid sequence of SEQ ID NO: 129. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a heavy chain variable domain comprising the amino acid sequence of SEQ ID NO: 117, or a variant thereof having at least about 90% sequence identity to the amino acid sequence of SEQ ID NO: 117; and a light chain variable domain comprising the amino acid sequence of SEQ ID NO: 129, or a variant thereof having at least about 90% sequence identity to the amino acid sequence of SEQ ID NO: 129. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a heavy chain variable domain comprising the amino acid sequence of SEQ ID NO: 120, or a variant thereof having at least about 90% sequence identity to the amino acid sequence of SEQ ID NO: 120; and a light chain variable domain comprising the amino acid sequence of SEQ ID NO: 139, or a variant thereof having at least about 90% sequence identity to the amino acid sequence of SEQ ID NO: 139. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a heavy chain variable domain comprising the amino acid sequence of SEQ ID NO: 121, or a variant thereof having at least about 90% sequence identity to the amino acid sequence of SEQ ID NO: 121; and a light chain variable domain comprising the amino acid sequence of SEQ ID NO: 140, or a variant thereof having at least about 90% sequence identity to the amino acid sequence of SEQ ID NO: 140. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a heavy chain variable domain comprising the amino acid sequence of SEQ ID NO: 122, or a variant thereof having at least about 90% sequence identity to the amino acid sequence of SEQ ID NO:122; and a light chain variable domain comprising the amino acid sequence of SEQ ID NO: 141, or a variant thereof having at least about 90% sequence identity to the amino acid sequence of SEQ ID NO: 141. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a heavy chain variable domain comprising the amino acid sequence of SEQ ID NO: 123, or a variant thereof having at least about 90% sequence identity to the amino acid sequence of SEQ ID NO: 123; and a light chain variable domain comprising the amino acid sequence of SEQ ID NO: 142, or a variant thereof having at least about 90% sequence identity to the amino acid sequence of SEQ ID NO: 142. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG 1 constant domains, wherein the anti-CD40 antibody comprises a heavy chain variable domain comprising the amino acid sequence of SEQ ID NO: 124, or a variant thereof having at least about 90% sequence identity to the amino acid sequence of SEQ ID NO: 124; and a light chain variable domain comprising the amino acid sequence of SEQ ID NO: 143, or a variant thereof having at least about 90% sequence identity to the amino acid sequence of SEQ ID NO: 143. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG1 constant domains, wherein the anti-CD40 antibody comprises a heavy chain variable domain comprising the amino acid sequence of SEQ ID NO: 125, or a variant thereof having at least about 90% sequence identity to the amino acid sequence of SEQ ID NO: 125; and a light chain variable domain comprising the amino acid sequence of SEQ ID NO: 144, or a variant thereof having at least about 90% sequence identity to the amino acid sequence of SEQ ID NO: 144. In some embodiments, the heavy chain constant region comprises or consists of the amino acid sequence of SEQ ID NO: 145. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 145 and the light chain constant region comprises or consists of the amino acid sequence of SEQ ID NO: 147. In some embodiments, the heavy chain constant region comprises or consists of the amino acid sequence of SEQ ID NO: 145 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a heavy chain variable domain comprising the amino acid sequence of SEQ ID NO: 87, or a variant thereof having at least about 90% sequence identity to the amino acid sequence of SEQ ID NO: 87; and a light chain variable domain comprising the amino acid sequence of SEQ ID NO: 126, or a variant thereof having at least about 90% sequence identity to the amino acid sequence of SEQ ID NO: 126. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a heavy chain variable domain comprising the amino acid sequence of SEQ ID NO: 88, or a variant thereof having at least about 90% sequence identity to the amino acid sequence of SEQ ID NO: 88; and a light chain variable domain comprising the amino acid sequence of SEQ ID NO: 127, or a variant thereof having at least about 90% sequence identity to the amino acid sequence of SEQ ID NO: 127. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a heavy chain variable domain comprising the amino acid sequence of SEQ ID NO: 89, or a variant thereof having at least about 90% sequence identity to the amino acid sequence of SEQ ID NO: 89; and a light chain variable domain comprising the amino acid sequence of SEQ ID NO: 128, or a variant thereof having at least about 90% sequence identity to the amino acid sequence of SEQ ID NO: 128. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a heavy chain variable domain comprising the amino acid sequence of SEQ ID NO: 94, or a variant thereof having at least about 90% sequence identity to the amino acid sequence of SEQ ID NO: 94; and a light chain variable domain comprising the amino acid sequence of SEQ ID NO: 128 or a variant thereof having at least about 90% sequence identity to the amino acid sequence of SEQ ID NO: 128. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a heavy chain variable domain comprising the amino acid sequence of SEQ ID NO: 96, or a variant thereof having at least about 90% sequence identity to the amino acid sequence of SEQ ID NO: 96; and a light chain variable domain comprising the amino acid sequence of SEQ ID NO: 132, or a variant thereof having at least about 90% sequence identity to the amino acid sequence of SEQ ID NO: 132. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a heavy chain variable domain comprising the amino acid sequence of SEQ ID NO: 101, or a variant thereof having at least about 90% sequence identity to the amino acid sequence of SEQ ID NO: 101; and a light chain variable domain comprising the amino acid sequence of SEQ ID NO: 128, or a variant thereof having at least about 90% sequence identity to the amino acid sequence of SEQ ID NO: 128. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a heavy chain variable domain comprising the amino acid sequence of SEQ ID NO: 110, or a variant thereof having at least about 90% sequence identity to the amino acid sequence of SEQ ID NO: 110; and a light chain variable domain comprising the amino acid sequence of SEQ ID NO: 128, or a variant thereof having at least about 90% sequence identity to the amino acid sequence of SEQ ID NO: 128. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a heavy chain variable domain comprising the amino acid sequence of SEQ ID NO: 114, or a variant thereof having at least about 90% sequence identity to the amino acid sequence of SEQ ID NO: 114; and a light chain variable domain comprising the amino acid sequence of SEQ ID NO: 128, or a variant thereof having at least about 90% sequence identity to the amino acid sequence of SEQ ID NO: 128. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a heavy chain variable domain comprising the amino acid sequence of SEQ ID NO: 115, or a variant thereof having at least about 90% sequence identity to the amino acid sequence of SEQ ID NO: 115; and a light chain variable domain comprising the amino acid sequence of SEQ ID NO: 129, or a variant thereof having at least about 90% sequence identity to the amino acid sequence of SEQ ID NO: 129. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a heavy chain variable domain comprising the amino acid sequence of SEQ ID NO: 117, or a variant thereof having at least about 90% sequence identity to the amino acid sequence of SEQ ID NO: 117; and a light chain variable domain comprising the amino acid sequence of SEQ ID NO: 129, or a variant thereof having at least about 90% sequence identity to the amino acid sequence of SEQ ID NO: 129. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a heavy chain variable domain comprising the amino acid sequence of SEQ ID NO: 120, or a variant thereof having at least about 90% sequence identity to the amino acid sequence of SEQ ID NO: 120; and a light chain variable domain comprising the amino acid sequence of SEQ ID NO: 139, or a variant thereof having at least about 90% sequence identity to the amino acid sequence of SEQ ID NO: 139. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a heavy chain variable domain comprising the amino acid sequence of SEQ ID NO: 121, or a variant thereof having at least about 90% sequence identity to the amino acid sequence of SEQ ID NO: 121; and a light chain variable domain comprising the amino acid sequence of SEQ ID NO: 140, or a variant thereof having at least about 90% sequence identity to the amino acid sequence of SEQ ID NO: 140. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a heavy chain variable domain comprising the amino acid sequence of SEQ ID NO: 122, or a variant thereof having at least about 90% sequence identity to the amino acid sequence of SEQ ID NO: 122; and a light chain variable domain comprising the amino acid sequence of SEQ ID NO: 141, or a variant thereof having at least about 90% sequence identity to the amino acid sequence of SEQ ID NO: 141. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a heavy chain variable domain comprising the amino acid sequence of SEQ ID NO: 123, or a variant thereof having at least about 90% sequence identity to the amino acid sequence of SEQ ID NO: 123; and a light chain variable domain comprising the amino acid sequence of SEQ ID NO: 142, or a variant thereof having at least about 90% sequence identity to the amino acid sequence of SEQ ID NO: 142. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a heavy chain variable domain comprising the amino acid sequence of SEQ ID NO: 124, or a variant thereof having at least about 90% sequence identity to the amino acid sequence of SEQ ID NO: 124; and a light chain variable domain comprising the amino acid sequence of SEQ ID NO: 143, or a variant thereof having at least about 90% sequence identity to the amino acid sequence of SEQ ID NO: 143. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

In some embodiments, there is provided a full-length anti-CD40 antibody comprising IgG4 constant domains, wherein the anti-CD40 antibody comprises a heavy chain variable domain comprising the amino acid sequence of SEQ ID NO: 125, or a variant thereof having at least about 90% sequence identity to the amino acid sequence of SEQ ID NO: 125; and a light chain variable domain comprising the amino acid sequence of SEQ ID NO: 144, or a variant thereof having at least about 90% sequence identity to the amino acid sequence of SEQ ID NO: 144. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148. In some embodiments, the heavy chain constant region comprises or consists of the amino acid sequence of SEQ ID NO: 146 and the light chain constant region comprises or consists of the amino acid sequence of SEQ ID NO: 147. In some embodiments, the heavy chain constant region comprises or consists of the amino acid sequence of SEQ ID NO: 146 and the light chain constant region comprises or consists of the amino acid sequence of SEQ ID NO: 148.

### Binding affinity

Binding affinity can be indicated by Kd, Koff, Kon, or Ka. The term "Koff', as used herein, is intended to refer to the off-rate constant for dissociation of an antibody from the antibody /antigen complex, as determined from a kinetic selection set up. The term "Kon", as used herein, is intended to refer to the on-rate constant for association of an antibody to the antigen to form the antibody/antigen complex. The term dissociation constant "Kd", as used herein, refers to the dissociation constant of a particular antibody-antigen interaction, and describes the concentration of antigen required to occupy one half of all of the antibody-binding domains present in a solution of antibody molecules at equilibrium, and is equal to Koff/Kon. The measurement of Kd presupposes that all binding agents are in solution. In the case where the antibody is tethered to a cell wall, *e.g.*, in a yeast expression system, the corresponding equilibrium rate constant is expressed as EC₅₀, which gives a good approximation of Kd. The affinity constant, Ka, is the inverse of the dissociation constant, Kd.

The dissociation constant (Kd) is used as an indicator showing affinity of antibody moieties to antigens. For example, easy analysis is possible by the Scatchard method using antibodies marked with a variety of marker agents, as well as by using Biacore (made by Amersham Biosciences), analysis of biomolecular interactions by surface plasmon resonance, according to the user's manual and attached kit. The Kd value that can be derived using these methods is expressed in units of M. An antibody that specifically binds to a target may have a Kd of, for example, ≤ 10⁻⁷ M, ≤ 10⁻⁸ M, ≤ 10⁻⁹ M, ≤ 10⁻¹⁰ M, ≤ 10⁻¹¹ M ≤ 10⁻¹² M, or ≤ 10⁻¹³ M.

Binding specificity of the antibody can be determined experimentally by methods known in the art. Such methods comprise, but are not limited to, Western blots, ELISA-, RIA-, ECL-, IRMA-, EIA-, BIAcore-tests and peptide scans.

In some embodiments, the anti-CD40 antibody specifically binds to a target CD40 with a Kd of about 10⁻⁷ M to about 10⁻¹¹ M (such as about 10⁻⁷ M to about 10⁻¹³ M, about 10⁻⁸ M to about 10⁻¹¹ M, about 10⁻⁹ M to about 10⁻¹³ M, or about 10⁻¹⁰ M to about 10⁻¹² M). Thus in some embodiments, the Kd of the binding between the anti-CD40 antibody and CD40, is about 10⁻⁷ M to about 10⁻¹³ M, about 1×10⁻⁷ M to about 5×10⁻¹³ M, about 10⁻⁷ M to about 10⁻¹² M, about 10⁻⁷ M to about 10⁻¹¹ M, about 10⁻⁷ M to about 10⁻¹⁰ M, about 10⁻⁷ M to about 10⁻⁹ M, about 10⁻⁸ M to about 10⁻¹³ M, about 1×10⁻⁸ M to about 5×10⁻¹³ M, about 10⁻⁸ M to about 10⁻¹² M, about 10⁻⁸ M to about 10⁻¹¹ M, about 10⁻⁸ M to about 10⁻¹⁰ M, about 10⁻⁸ M to about 10⁻⁹ M, about 5×10⁻⁹ M to about 1×10⁻¹³ M, about 5×10⁻⁹ M to about 1×10⁻¹¹ M, about 5×10⁻⁹ M to about 1×10⁻¹¹ M, about 5×10⁻⁹ M to about 1×10⁻¹⁰ M, about 10⁻⁹ M to about 10⁻¹¹ M, about 10⁻⁹ M to about 10⁻¹² M, about 10⁻⁹ M to about 10⁻¹¹ M, about 10⁻⁹ M to about 10⁻¹⁰ M, about 5×10⁻¹⁰ M to about 1×10⁻¹³ M, about 5×10⁻¹⁰ M to about 1×10⁻¹² M, about 5×10⁻¹⁰ M to about 1×10⁻¹¹ M, about 10⁻¹⁰ M to about10⁻¹³ M, about 1×10⁻¹⁰ M to about 5×10⁻¹³ M, about 1×10⁻¹⁰ M to about 1×10⁻¹² M, about 1×10⁻¹⁰ M to about 5×10⁻¹² M, about 1×10⁻¹⁰ M to about 1×10⁻¹¹ M, about 10⁻¹¹ M to about 10⁻¹³ M, about 1× 10⁻¹¹ M to about 5×10⁻¹³ M, about 10⁻¹¹ M to about 10⁻¹² M, or about 10⁻¹² M to about 10⁻¹³ M. In some embodiments, the Kd of the binding between the anti-CD40 antibody and a CD40 is about 10⁻⁷ M to about 10⁻¹³ M.

In some embodiments, the Kd of the binding between the anti-CD40 antibody and a non-target is more than the Kd of the binding between the anti-CD40 antibody and the target, and is herein referred to in some embodiments as the binding affinity, of the anti-CD40 antibody to the target (*e.g.*, CD40) is higher than that to a non-target. In some embodiments, the non-target is an antigen that is not CD40. In some embodiments, the Kd of the binding between the anti-CD40 antibody (against CD40) and a non-CD40 target can be at least about 10 times, such as about 10-100 times, about 100-1000 times, about 10³-10⁴ times, about 10⁴-10⁵ times, about 10⁵-10⁶ times, about 10⁶-10⁷ times, about 10⁷-10⁸ times, about 10⁸-10⁹ times, about 10⁹-10¹⁰ times, about 10¹⁰-10¹¹ times, or about 10¹¹-10¹² times of the Kd of the binding between the anti-CD40 antibody and a target CD40.

In some embodiments, the anti-CD40 antibody binds to a non-target with a Kd of about 10⁻¹ M to about 10⁻⁶ M (such as about 10⁻¹ M to about 10⁻⁶ M, about 10⁻¹ M to about 10⁻⁵ M, or about 10⁻² M to about 10⁻⁴ M). In some embodiments, the non-target is an antigen that is not CD40. Thus in some embodiments, the Kd of the binding between the anti-CD40 antibody and a non-CD40 target is about 10⁻¹ M to about 10⁻⁶ M, about 1×10⁻¹ M to about 5×10⁻⁶ M, about 10⁻¹ M to about 10⁻⁵ M, about 1×10⁻¹ M to about 5×10⁻⁵ M, about 10⁻¹ M to about 10⁻⁴ M, about 1×10⁻¹ M to about 5×10⁻⁴ M, about 10⁻¹ M to about 10⁻³ M, about 1×10⁻¹ M to about 5×10⁻³ M, about 10⁻¹ M to about 10⁻² M, about 10⁻² M to about 10⁻⁶ M, about 1×10⁻² M to about 5×10⁻⁶ M, about 10⁻² M to about 10⁻⁵ M, about 1× 10⁻² M to about 5×10⁻⁵ M, about 10⁻² M to about 10⁻⁴ M, about 1×10⁻¹ M to about 5×10⁻⁴ M, about 10⁻² M to about 10⁻³ M, about 10⁻³ M to about 10⁻⁶ M, about 1×10⁻³ M to about 5×10⁻⁶ M, about 10⁻³ M to about 10⁻⁵ M, about 1×10⁻³ M to about 5×10⁻⁵ M, about 10⁻³ M to about 10⁻⁴ M, about 10⁻⁴ M to about 10⁻⁶ M, about 1×10⁻⁴ M to about 5×10⁻⁶ M, about 10⁻⁴ M to about 10⁻⁵ M, or about 10⁻⁵ M to about 10⁻⁶ M.

In some embodiments, when referring to that the anti-CD40 antibody specifically recognizes a target CD40 at a high binding affinity, and binds to a non-target at a low binding affinity, the anti-CD40 antibody will bind to the target CD40 with a Kd of about 10⁻⁷ M to about 10⁻¹³ M (such as about 10⁻⁷ M to about 10⁻¹³ M, about 10⁻⁸ M to about 10⁻¹³ M, about 10⁻⁹ M to about 10⁻¹³ M, or about 10⁻¹¹ M to about 10⁻¹² M), and will bind to the non-target with a Kd of about 10⁻¹ M to about 10⁻⁶ M (such as about 10⁻¹ M to about 10⁻⁶ M, about 10⁻¹ M to about 10⁻⁵ M, or about 10⁻³ M to about 10⁻⁴ M).

In some embodiments, when referring to that the anti-CD40 antibody specifically recognizes CD40, the binding affinity of the anti-CD40 antibody is compared to that of a control anti-CD40 antibody (such as ASKP1240). In some embodiments, the Kd of the binding between the control anti-CD40 antibody and CD40 can be at least about 2 times, such as about 2 times, about 3 times, about 4 times, about 5 times, about 6 times, about 7 times, about 8 times, about 9 times, about 10 times, about 10-100 times, about 100-1000 times, about 10³-10⁴ times of the Kd of the binding between the anti-CD40 antibody described herein and CD40.

### Nucleic Acids

Nucleic acid molecules encoding the anti-CD40 antibodies are also contemplated. In some embodiments, there is provided a nucleic acid (or a set of nucleic acids) encoding a full-length anti-CD40 antibody, including any of the full-length anti-CD40 antibodies described herein. In some embodiments, the nucleic acid (or a set of nucleic acids) encoding the anti-CD40 antibody described herein may further comprises a nucleic acid sequence encoding a peptide tag (such as protein purification tag, e.g., His-tag, HA tag).

Also contemplated here are isolated host cells comprising an anti-CD40 antibody, an isolated nucleic acid encoding the polypeptide components of the anti-CD40 antibody, or a vector comprising a nucleic acid encoding the polypeptide components of the anti-CD40 antibody described herein.

The present application also includes variants to these nucleic acid sequences. For example, the variants include nucleotide sequences that hybridize to the nucleic acid sequences encoding the anti-CD40 antibodies of the present application under at least moderately stringent hybridization conditions.

The present application also provides vectors in which a nucleic acid of the present application is inserted.

In brief summary, the expression of an anti-CD40 antibody (e.g., full-length anti-CD40 antibody) by a natural or synthetic nucleic acid encoding the anti-CD40 antibody can be achieved by inserting the nucleic acid into an appropriate expression vector, such that the nucleic acid is operably linked to 5' and 3' regulatory elements, including for example a promoter (e.g., a lymphocyte-specific promoter) and a 3' untranslated region (UTR). The vectors can be suitable for replication and integration in eukaryotic host cells. Typical cloning and expression vectors contain transcription and translation terminators, initiation sequences, and promoters useful for regulation of the expression of the desired nucleic acid sequences.

The nucleic acids of the present application may also be used for nucleic acid immunization and gene therapy, using standard gene delivery protocols. Methods for gene delivery are known in the art. See, *e.g.,* U.S. Pat. Nos. 5,399,346, 5,580,859, 5,589,466, incorporated by reference herein in their entireties. In some embodiments, the application provides a gene therapy vector.

The nucleic acid can be cloned into a number of types of vectors. For example, the nucleic acid can be cloned into a vector including, but not limited to a plasmid, a phagemid, a phage derivative, an animal virus, and a cosmid. Vectors of particular interest include expression vectors, replication vectors, probe generation vectors, and sequencing vectors.

Further, the expression vector may be provided to a cell in the form of a viral vector. Viral vector technology is well known in the art and is described, for example, in Green and Sambrook (2013, Molecular Cloning: A Laboratory Manual, Cold Spring Harbor Laboratory, New York), and in other virology and molecular biology manuals. Viruses which are useful as vectors include, but are not limited to, retroviruses, adenoviruses, adeno-associated viruses, herpes viruses, and lentiviruses. In general, a suitable vector contains an origin of replication functional in at least one organism, a promoter sequence, convenient restriction endonuclease sites, and one or more selectable markers *(see, e.g.,* WO 01/96584; WO 01/29058; and U.S. Pat. No. 6,326,193).

A number of viral based systems have been developed for gene transfer into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either *in vivo* or *ex vivo.* A number of retroviral systems are known in the art. In some embodiments, adenovirus vectors are used. A number of adenovirus vectors are known in the art. In some embodiments, lentivirus vectors are used. Vectors derived from retroviruses such as the lentivirus are suitable tools to achieve long-term gene transfer since they allow long-term, stable integration of a transgene and its propagation in daughter cells. Lentiviral vectors have the added advantage over vectors derived from onco-retroviruses such as murine leukemia viruses in that they can transduce non-proliferating cells, such as hepatocytes. They also have the added advantage of low immunogenicity.

Additional promoter elements, e.g., enhancers, regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline.

One example of a suitable promoter is the immediate early cytomegalovirus (CMV) promoter sequence. This promoter sequence is a strong constitutive promoter sequence capable of driving high levels of expression of any polynucleotide sequence operatively linked thereto. Another example of a suitable promoter is Elongation Factor-1α (EF-1α). However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the hemoglobin promoter, and the creatine kinase promoter. Further, the application should not be limited to the use of constitutive promoters. Inducible promoters are also contemplated as part of the application. The use of an inducible promoter provides a molecular switch capable of turning on expression of the polynucleotide sequence to which it is operatively linked when such expression is desired, or turning off the expression when expression is not desired. Examples of inducible promoters include, but are not limited to a metallothionine promoter, a glucocorticoid promoter, a progesterone promoter, and a tetracycline promoter.

In some embodiments, the expression of the anti-CD40 antibody is inducible. In some embodiments, a nucleic acid sequence encoding the anti-CD40 antibody is operably linked to an inducible promoter, including any inducible promoter described herein.

### Inducible promoters

The use of an inducible promoter provides a molecular switch capable of turning on expression of the polynucleotide sequence to which it is operatively linked when such expression is desired, or turning off the expression when expression is not desired. Exemplary inducible promoter systems for use in eukaryotic cells include, but are not limited to, hormone-regulated elements (*e.g., see* Mader, S. and White, J. H. (1993) Proc. Natl. Acad. Sci. USA 90:5603-5607), synthetic ligand-regulated elements (*see, e.g.,* Spencer, D. M. et al. (1993) Science 262: 1019-1024) and ionizing radiation-regulated elements *(e.g.,* see Manome, Y. et al. (1993) Biochemistry 32: 10607-10613; Datta, R. et al. (1992) Proc. Natl. Acad. Sci. USA 89: 1014- 10153). Further exemplary inducible promoter systems for use in in vitro or in vivo mammalian systems are reviewed in Gingrich et al. (1998) Annual Rev. Neurosci 21 :377-405. In some embodiments, the inducible promoter system for use to express the anti-CD40 antibody is the Tet system. In some embodiments, the inducible promoter system for use to express the anti-CD40 antibody is the lac repressor system from E. *coli.*

An exemplary inducible promoter system for use in the present application is the Tet system. Such systems are based on the Tet system described by Gossen *et al.* (1993). In an exemplary embodiment, a polynucleotide of interest is under the control of a promoter that comprises one or more Tet operator (TetO) sites. In the inactive state, Tet repressor (TetR) will bind to the TetO sites and repress transcription from the promoter. In the active state, *e.g.,* in the presence of an inducing agent such as tetracycline (Tc), anhydrotetracycline, doxycycline (Dox), or an active analog thereof, the inducing agent causes release of TetR from TetO, thereby allowing transcription to take place. Doxycycline is a member of the tetracycline family of antibiotics having the chemical name of 1-dimethylamino-2,4a,5,7,12-pentahydroxy-11-methyl-4,6-dioxo-1,4a,11,11a,12,12a-hexahydrotetracene-3-carboxamide.

In one embodiment, a TetR is codon-optimized for expression in mammalian cells, *e.g.,* murine or human cells. Most amino acids are encoded by more than one codon due to the degeneracy of the genetic code, allowing for substantial variations in the nucleotide sequence of a given nucleic acid without any alteration in the amino acid sequence encoded by the nucleic acid. However, many organisms display differences in codon usage, also known as "codon bias" (*i.e.,* bias for use of a particular codon(s) for a given amino acid). Codon bias often correlates with the presence of a predominant species of tRNA for a particular codon, which in turn increases efficiency of mRNA translation. Accordingly, a coding sequence derived from a particular organism (e.g., a prokaryote) may be tailored for improved expression in a different organism (*e.g*., a eukaryote) through codon optimization.

Other specific variations of the Tet system include the following "Tet-Off" and "Tet-On" systems. In the Tet-Off system, transcription is inactive in the presence of Tc or Dox. In that system, a tetracycline-controlled transactivator protein (tTA), which is composed of TetR fused to the strong transactivating domain of VP16 from Herpes simplex virus, regulates expression of a target nucleic acid that is under transcriptional control of a tetracycline-responsive promoter element (TRE). The TRE is made up of TetO sequence concatamers fused to a promoter (commonly the minimal promoter sequence derived from the human cytomegalovirus (hCMV) immediate-early promoter). In the absence of Tc or Dox, tTA binds to the TRE and activates transcription of the target gene. In the presence of Tc or Dox, tTA cannot bind to the TRE, and expression from the target gene remains inactive.

Conversely, in the Tet-On system, transcription is active in the presence of Tc or Dox. The Tet-On system is based on a reverse tetracycline-controlled transactivator, rtTA. Like tTA, rtTA is a fusion protein comprised of the TetR repressor and the VP16 transactivation domain. However, a four amino acid change in the TetR DNA binding moiety alters rtTA's binding characteristics such that it can only recognize the tetO sequences in the TRE of the target transgene in the presence of Dox. Thus, in the Tet-On system, transcription of the TRE-regulated target gene is stimulated by rtTA only in the presence of Dox.

Another inducible promoter system is the lac repressor system from *E. coli* (*See* Brown et al., Cell 49:603-612 (1987)). The lac repressor system functions by regulating transcription of a polynucleotide of interest operably linked to a promoter comprising the lac operator (lacO). The lac repressor (lacR) binds to LacO, thus preventing transcription of the polynucleotide of interest. Expression of the polynucleotide of interest is induced by a suitable inducing agent, e.g., isopropyl-(3-D-thiogalactopyranoside (IPTG).

In order to assess the expression of a polypeptide or portions thereof, the expression vector to be introduced into a cell can also contain either a selectable marker gene or a reporter gene or both to facilitate identification and selection of expressing cells from the population of cells sought to be transfected or infected through viral vectors. In other aspects, the selectable marker may be carried on a separate piece of DNA and used in a co-transfection procedure. Both selectable markers and reporter genes may be flanked with appropriate regulatory sequences to enable expression in the host cells. Useful selectable markers include, for example, antibiotic-resistance genes, such as neo and the like.

Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, β-galactosidase, chloramphenicol acetyl transferase, secreted alkaline phosphatase, or the green fluorescent protein gene *(e.g.,* Ui-Tel et al., 2000 FEBS Letters 479: 79-82). Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter-driven transcription.

In some embodiments, there is provided nucleic acid encoding a full-length anti-CD40 antibody according to any of the full-length anti-CD40 antibodies described herein. In some embodiments, the nucleic acid comprises one or more nucleic acid sequences encoding the heavy and light chains of the full-length anti-CD40 antibody. In some embodiments, each of the one or more nucleic acid sequences are contained in separate vectors. In some embodiments, at least some of the nucleic acid sequences are contained in the same vector. In some embodiments, all of the nucleic acid sequences are contained in the same vector. Vectors may be selected, for example, from the group consisting of mammalian expression vectors and viral vectors (such as those derived from retroviruses, adenoviruses, adeno-associated viruses, herpes viruses, and lentiviruses).

Methods of introducing and expressing genes into a cell are known in the art. In the context of an expression vector, the vector can be readily introduced into a host cell, *e.g*., mammalian, bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Green and Sambrook (2013, Molecular Cloning: A Laboratory Manual, Cold Spring Harbor Laboratory, New York). In some embodiments, the introduction of a polynucleotide into a host cell is carried out by calcium phosphate transfection.

Biological methods for introducing a polynucleotide of interest into a host cell include the use of DNA and RNA vectors. Viral vectors, and especially retroviral vectors, have become the most widely used method of inserting genes into mammalian, *e.g*., human cells. Other viral vectors can be derived from lentivirus, poxviruses, herpes simplex virus 1, adenoviruses and adeno-associated viruses, and the like. *See,* for example, U.S. Pat. Nos. 5,350,674 and 5,585,362.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle *in vitro* and *in vivo* is a liposome *(e.g.,* an artificial membrane vesicle).

In the case where a non-viral delivery system is utilized, an exemplary delivery vehicle is a liposome. The use of lipid formulations is contemplated for the introduction of the nucleic acids into a host cell (*in vitro, ex vivo* or *in vivo*)*.* In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

Regardless of the method used to introduce exogenous nucleic acids into a host cell or otherwise expose a cell to the inhibitor of the present application, in order to confirm the presence of the recombinant DNA sequence in the host cell, a variety of assays may be performed. Such assays include, for example, "molecular biological" assays well known to those of skill in the art, such as Southern and Northern blotting, RT-PCR and PCR; "biochemical" assays, such as detecting the presence or absence of a particular peptide, *e.g.,* by immunological means (ELISAs and Western blots) or by assays described herein to identify agents falling within the scope of the application.

### Preparation of anti-CD40 antibodies

In some embodiments, the anti-CD40 antibody is a monoclonal antibody or derived from a monoclonal antibody. In some embodiments, the anti-CD40 antibody comprises V_{H} and V_{L} domains, or variants thereof, from the monoclonal antibody. In some embodiments, the anti-CD40 antibody further comprises C_{H}1 and C_{L} domains, or variants thereof, from the monoclonal antibody. Monoclonal antibodies can be prepared, *e.g*., using known methods in the art, including hybridoma methods, phage display methods, or using recombinant DNA methods. Additionally, exemplary phage display methods are described herein and in the Examples below.

In a hybridoma method, a hamster, mouse, or other appropriate host animal is typically immunized with an immunizing agent to elicit lymphocytes that produce or are capable of producing antibodies that will specifically bind to the immunizing agent. Alternatively, the lymphocytes can be immunized *in vitro.* The immunizing agent can include a polypeptide or a fusion protein of the protein of interest. Generally, peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell. Immortalized cell lines are usually transformed mammalian cells, particularly myeloma cells of rodent, bovine, and human origin. Usually, rat or mouse myeloma cell lines are employed. The hybridoma cells can be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which prevents the growth of HGPRT-deficient cells.

In some embodiments, the immortalized cell lines fuse efficiently, support stable high-level expression of antibody by the selected antibody-producing cells, and are sensitive to a medium such as HAT medium. In some embodiments, the immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies.

The culture medium in which the hybridoma cells are cultured can then be assayed for the presence of monoclonal antibodies directed against the polypeptide. The binding specificity of monoclonal antibodies produced by the hybridoma cells can be determined by immunoprecipitation or by an *in vitro* binding assay, such as radioimmunoassay (RIA) or enzyme-linked immunosorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones can be sub-cloned by limiting dilution procedures and grown by standard methods. Goding, *supra.* Suitable culture media for this purpose include, for example, Dulbecco's Modified Eagle's Medium and RPMI-1640 medium. Alternatively, the hybridoma cells can be grown *in vivo* as ascites in a mammal.

The monoclonal antibodies secreted by the sub-clones can be isolated or purified from the culture medium or ascites fluid by conventional immunoglobulin purification procedures such as, for example, protein A-Sepharose, hydroxylapatite chromatography, gel electrophoresis, dialysis, or affinity chromatography.

In some embodiments, according to any of the anti-CD40 antibodies described herein, the anti-CD40 antibody comprises sequences from a clone selected from an antibody library (such as a phage library presenting scFv or Fab fragments). The clone may be identified by screening combinatorial libraries for antibody fragments with the desired activity or activities. For example, a variety of methods are known in the art for generating phage display libraries and screening such libraries for antibodies possessing the desired binding characteristics. Such methods are reviewed, *e.g.,* in Hoogenboom et al., Methods in Molecular Biology 178:1-37 (O'Brien et al., ed., Human Press, Totowa, N.J., 2001) and further described, *e.g.,* in McCafferty et al., Nature 348:552-554; Clackson et al., Nature 352: 624-628 (1991); Marks et al., J. Mol. Biol. 222: 581-597 (1992); Marks and Bradbury, Methods in Molecular Biology 248:161-175 (Lo, ed., Human Press, Totowa, N.J., 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of V_{H} and V_{L} genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as scFv fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self-antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter, J. Mol. Biol., 227: 381-388 (1992). Patent publications describing human antibody phage libraries include, for example: U.S. Pat. No. 5,750,373, and US Patent Publication Nos. 2005/0079574, 2005/0119455, 2005/0266000, 2007/0117126, 2007/0160598, 2007/0237764, 2007/0292936, and 2009/0002360.

The anti-CD40 antibodies can be prepared using phage display to screen libraries for anti-CD40 antibody moieties specific to the target CD40. The library can be a human scFv phage display library having a diversity of at least 1 × 10⁹ (such as at least about any of 1 × 10⁹, 2.5 × 10⁹, 5 × 10⁹, 7.5 × 10⁹, 1 × 10¹⁰, 2.5 × 10¹⁰, 5 × 10¹⁰, 7.5 × 10¹⁰, or 1 × 10¹¹) unique human antibody fragments. In some embodiments, the library is a naive human library constructed from DNA extracted from human PMBCs and spleens from healthy donors, encompassing all human heavy and light chain subfamilies. In some embodiments, the library is a naïve human library constructed from DNA extracted from PBMCs isolated from patients with various diseases, such as patients with autoimmune diseases, cancer patients, and patients with infectious diseases. In some embodiments, the library is a semi-synthetic human library, wherein heavy chain CDR3 is completely randomized, with all amino acids (with the exception of cysteine) equally likely to be present at any given position (*see, e.g.,* Hoet, R.M. et al., Nat. Biotechnol. 23(3):344-348, 2005). In some embodiments, the heavy chain CDR3 of the semi-synthetic human library has a length from about 5 to about 24 (such as about any of 5, 6, 7, 8, 9, 10, 11, 12, 13, 14, 15, 16, 17, 18, 19, 20, 21, 22, 23, or 24) amino acids. In some embodiments, the library is a fully-synthetic phage display library. In some embodiments, the library is a non-human phage display library.

Phage clones that bind to the target CD40 with high affinity can be selected by iterative binding of phage to the target CD40, which is bound to a solid support (such as, for example, beads for solution panning or mammalian cells for cell panning), followed by removal of non-bound phage and by elution of specifically bound phage. The bound phage clones are then eluted and used to infect an appropriate host cell, such as *E. coli* XL1-Blue, for expression and purification. The panning can be performed for multiple (such as about any of 2, 3, 4, 5, 6 or more) rounds with solution panning, cell panning, or a combination of both, to enrich for phage clones binding specifically to the target CD40. Enriched phage clones can be tested for specific binding to the target CD40 by any methods known in the art, including for example ELISA and FACS.

An alternative method for screening antibody libraries is to display the protein on the surface of yeast cells. Wittrup et al. (US Patent Nos. 6,699,658 and 6,696,25 1) have developed a method for a yeast cell display library. In this yeast display system, a component involves the yeast agglutinin protein (Agal), which is anchored to the yeast cell wall. Another component involves a second subunit of the agglutinin protein Aga2, which can display on the surface yeast cells through disulfide bonds to Agal protein. The protein Agal is expressed from a yeast chromosome after the Agal gene integration. A library of single chain variable fragments (scFv) is fused genetically to Aga2 sequence in the yeast display plasmid, which, after transformation, is maintained in yeast episomally with a nutritional marker. Both of the Agal and Aga2 proteins were expressed under the control of the galactose-inducible promoter.

Human antibody V gene repertoire (V_{H} and V_{K} fragments) are obtained by PCR method using a pool of degenerate primers (Sblattero, D. & Bradbury, A. Immunotechnology 3, 271-278 1998). The PCR templates are from the commercially available RNAs or cDNAs, including PBMC, spleen, lymph nodes, bone marrow and tonsils. Separate V_{H} and V_{K} PCR libraries were combined, then assembled together in the scFv format by overlap extension PCR ( Sheets, M.D. et al., Proc. Natl. Acad. Sci. USA 95, 6157-6162 1998.). To construct the yeast scFv display library, the resultant scFv PCR products are cloned into the yeast display plasmid in the yeasts by homologous recombination. (Chao, G, et al., Nat Protoc. 2006;1(2):755-68. Miller KD, et al., Current Protocols in Cytometry 4.7.1-4.7.30, 2008).

The anti-CD40 antibodies can be discovered using mammalian cell display systems in which antibody moieties are displayed on the cell surface and those specific to the target CD40 are isolated by the antigen-guided screening method, as described in U.S. patent No. 7,732,195B2. A Chinese hamster ovary (CHO) cell library representing a large set of human IgG antibody genes can be established and used to discover the clones expressing high-affinity antibody genes. Another display system has been developed to enable simultaneous high-level cell surface display and secretion of the same protein through alternate splicing, where the displayed protein phenotype remains linked to genotype, allowing soluble secreted antibody to be simultaneously characterized in biophysical and cell-based functional assays. This approach overcomes many limitations of previous mammalian cell display, enabling direct selection and maturation of antibodies in the form of full-length, glycosylated IgGs (Peter M. Bowers, et al., Methods 2014,65:44-56). Transient expression systems are suitable for a single round of antigen selection before recovery of the antibody genes and therefore most useful for the selection of antibodies from smaller libraries. Stable episomal vectors offer an attractive alternative. Episomal vectors can be transfected at high efficiency and stably maintained at low copy number, permitting multiple rounds of panning and the resolution of more complex antibody libraries.

The IgG library is based on germline sequence V-gene segments joined to rearranged (D)J regions isolated from a panel of human donors. RNA collected from 2000 human blood samples was reverse-transcribed into cDNA, and the V_{H} and V_{K} fragments were amplified using V_{H}- and V_{K}-specific primers and purified by gel extraction. IgG libraries were generated by sub-cloning the V_{H} and V_{K} fragments into the display vectors containing IgG 1 or K constant regions respectively and then electroporating into or transducing 293T cells. To generate the scFv antibody display library, scFvs were generated by linking V_{H} and V_{K}, and then sub-cloned into the display vector, which were then electroporated into or transduce 293T cells. As we known, the IgG library is based on germline sequence V-gene segments joined to rearranged (D)J regions isolated from a panel of donors, the donor can be a mouse, rat, rabbit, or monkey.

Monoclonal antibodies can also be made by recombinant DNA methods, such as those described in U.S. Patent No. 4,816,567. DNA encoding the monoclonal antibodies of the application can be readily isolated and sequenced using conventional procedures (*e.g*., by using oligonucleotide probes that are capable of binding specifically to genes encoding the heavy and light chains of murine antibodies). Hybridoma cells as described above or CD40-specific phage clones of the application can serve as a source of such DNA. Once isolated, the DNA can be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also can be modified, for example, by substituting the coding sequence for human heavy- and light-chain constant domains and/or framework regions in place of the homologous non-human sequences (U.S. Patent No. 4,816,567; Morrison *et al., supra*) or by covalently joining to the immunoglobulin coding sequence all or part of the coding sequence for a non-immunoglobulin polypeptide. Such a non-immunoglobulin polypeptide can be substituted for the constant domains of an antibody of the application, or can be substituted for the variable domains of one antigen-combining site of an antibody of the application to create a chimeric bivalent antibody.

The antibodies can be monovalent antibodies. Methods for preparing monovalent antibodies are known in the art. For example, one method involves recombinant expression of immunoglobulin light chain and modified heavy chain. The heavy chain is truncated generally at any point in the Fc region so as to prevent heavy-chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly Fab fragments, can be accomplished using any method known in the art.

Antibody variable domains with the desired binding specificities (antibody-antigen combining sites) can be fused to immunoglobulin constant-domain sequences. The fusion preferably is with an immunoglobulin heavy-chain constant domain, comprising at least part of the hinge, CH2, and CH3 regions. In some embodiments, the first heavy-chain constant region (CH1) containing the site necessary for light-chain binding is present in at least one of the fusions. DNAs encoding the immunoglobulin heavy-chain fusions and, if desired, the immunoglobulin light chain, are inserted into separate expression vectors, and are co-transfected into a suitable host organism.

### Human and Humanized Antibodies

The anti-CD40 antibodies (e.g., full-length anti-CD40 antibodies) can be humanized antibodies or human antibodies. Humanized forms of non-human (e.g., murine) antibody moieties are chimeric immunoglobulins, immunoglobulin chains, or fragments thereof (such as Fv, Fab, Fab', F(ab')₂, scFv, or other antigen-binding subsequences of antibodies) that typically contain minimal sequence derived from non-human immunoglobulin. Humanized antibody moieties include human immunoglobulins, immunoglobulin chains, or fragments thereof (recipient antibody) in which residues from a CDR of the recipient are replaced by residues from a CDR of a non-human species (donor antibody) such as mouse, rat, or rabbit having the desired specificity, affinity, and capacity. In some instances, Fv framework residues of the human immunoglobulin are replaced by corresponding non-human residues. Humanized antibody moieties can also comprise residues that are found neither in the recipient antibody nor in the imported CDR or framework sequences. In general, the humanized antibody can comprise substantially at least one, and typically two, variable domains, in which all or substantially all of the CDR regions correspond to those of a non-human immunoglobulin, and all or substantially all of the FR regions are those of a human immunoglobulin consensus sequence.

Generally, a humanized antibody has one or more amino acid residues introduced into it from a source that is non-human. These non-human amino acid residues are often referred to as "import" residues, which are typically taken from an "import" variable domain. According to some embodiments, humanization can be essentially performed following the method of Winter and co-workers (Jones et al., Nature, 321: 522-525 (1986); Riechmann et al., Nature, 332: 323-327 (1988); Verhoeyen et al., Science, 239: 1534-1536 (1988)), by substituting rodent CDRs or CDR sequences for the corresponding sequences of a human antibody. Accordingly, such "humanized" antibody moieties are antibody moieties (U.S. Patent No. 4,816,567), wherein substantially less than an intact human variable domain has been substituted by the corresponding sequence from a non-human species. In practice, humanized antibody moieties are typically human antibody moieties in which some CDR residues and possibly some FR residues are substituted by residues from analogous sites in rodent antibodies.

As an alternative to humanization, human antibody moieties can be generated. For example, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region (JH) gene in chimeric and germ-line mutant mice results in complete inhibition of endogenous antibody production. Transfer of the human germ-line immunoglobulin gene array into such germ-line mutant mice will result in the production of human antibodies upon antigen challenge. See, *e.g.,* Jakobovits et al., PNAS USA, 90:2551 (1993); Jakobovits et al., Nature, 362:255-258 (1993); Bruggemann et al., Year in Immunol., 7:33 (1993); U.S. Patent Nos. 5,545,806, 5,569,825, 5,591,669; 5,545,807; and WO 97/17852. Alternatively, human antibodies can be made by introducing human immunoglobulin loci into transgenic animals, *e.g.,* mice in which the endogenous immunoglobulin genes have been partially or completely inactivated. Upon challenge, human antibody production is observed that closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; and 5,661,016, and Marks et al., BiolTechnology, 10: 779-783 (1992); Lonberg et al., Nature, 368: 856-859 (1994); Morrison, Nature, 368: 812-813 (1994); Fishwild et al., Nature Biotechnology, 14: 845-851 (1996); Neuberger, Nature Biotechnology, 14: 826 (1996); Lonberg and Huszar, Intern. Rev. Immunol., 13: 65-93 (1995).

Human antibodies may also be generated by *in vitro* activated B cells (see U.S. Patents 5,567,610 and 5,229,275) or by using various techniques known in the art, including phage display libraries. Hoogenboom and Winter, J. Mol. Biol., 227:381 (1991); Marks et al., J. Mol. Biol., 222:581 (1991). The techniques of Cole *et al.* and Boerner *et al.* are also available for the preparation of human monoclonal antibodies. Cole et al., Monoclonal Antibodies and Cancer Therapy, Alan R. Liss, p.77 (1985) and Boerner et al., J. Immunol., 147(1): 86-95 (1991).

### Anti-CD40 antibody variants

In some embodiments, amino acid sequences of the anti-CD40 antibody variants (e.g., full-length anti-CD40 antibody) provided herein are contemplated. For example, it may be desirable to improve the binding affinity and/or other biological properties of the antibody. Amino acid sequences of an antibody variant may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, *e.g*., antigen-binding.

In some embodiments, anti-CD40 antibody variants having one or more amino acid substitutions are provided. Sites of interest for substitutional mutagenesis include the HVRs and FRs. Amino acid substitutions may be introduced into an antibody of interest and the products screened for a desired activity, *e.g*., improved bioactivity, retained/improved antigen binding, decreased immunogenicity, or improved ADCC or CDC.

Conservative substitutions are shown in Table 4 below.

**TABLE 4: CONSERVATIVE SUBSTITUTIONS**

| **Original** | **Exemplary** | **Preferred** |
|---|---|---|
| **Residue** | **Substitutions** | **Substitutions** |
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped into different classes according to common side-chain properties:
a. hydrophobic: Norleucine, Met, Ala, Val, Leu, Ile;
b. neutral hydrophilic: Cys, Ser, Thr, Asn, Gln;
c. acidic: Asp, Glu;
d. basic: His, Lys, Arg;
e. residues that influence chain orientation: Gly, Pro;
f. aromatic: Trp, Tyr, Phe.

Non-conservative substitutions will entail exchanging a member of one of these classes for another class.

An exemplary substitutional variant is an affinity matured antibody, which may be conveniently generated, *e.g*., using phage display-based affinity maturation techniques. Briefly, one or more CDR residues are mutated and the variant antibody moieties displayed on phage and screened for a particular biological activity (e.g., bioactivity based on HEK-Blue^{™} CD40L NF-κB activation assay or binding affinity). Alterations (e.g., substitutions) may be made in HVRs, *e.g.,* to improve bioactivity based on HEK-Blue^{™} CD40L NF-κB activation assay or binding affinity. Such alterations may be made in HVR "hotspots", *e.g*., residues encoded by codons that undergo mutation at high frequency during the somatic maturation process (see, *e.g*., Chowdhury, Methods Mol. Biol. 207:179-196 (2008)), and/or specificity determining residues (SDRs), with the resulting variant V_{H} and V_{L} being tested for binding affinity. Affinity maturation by constructing and reselecting from secondary libraries has been described, *e.g.,* in Hoogenboom et al., in Methods in Molecular Biology 178:1-37 (O'Brien et al., ed., Human Press, Totowa, NJ, (2001)).

In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, *e.g.,* using alanine scanning mutagenesis or modeling. CDR-H3 and CDR-L3 in particular are often targeted.

In some embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may be outside of HVR "hotspots" or SDRs. In some embodiments of the variant V_{H} and V_{L} sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (*e.g*., charged residues such as Arg, Asp, His, Lys, and Glu) are identified and replaced by a neutral or negatively charged amino acid (e.g., Ala or Glu) to determine whether the interaction of the antibody with antigen is affected. Further substitutions may be introduced at the amino acid locations to demonstrate functional sensitivity to the initial substitutions. Alternatively, or additionally, a crystal structure of an antigen-antibody complex can be determined to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (*e.g*., for ADEPT) or a polypeptide which increases the serum half-life of the antibody.

### Fc Region Variants

In some embodiments, one or more amino acid modifications may be introduced into the Fc region of an antibody (e.g., a full-length anti-CD40 antibody or anti-CD40 Fc fusion protein) provided herein, thereby generating an Fc region variant. In some embodiments, the Fc region variant has enhanced ADCC effector function, often related to binding to Fc receptors (FcRs). In some embodiments, the Fc region variant has decreased ADCC effector function. There are many examples of changes or mutations to Fc sequences that can alter effector function. For example, WO 00/42072 and Shields et al., J Biol. Chem. 9(2): 6591-6604 (2001) describe antibody variants with improved or diminished binding to FcRs. The contents of those publications are specifically incorporated herein by reference.

Antibody-Dependent Cell-Mediated Cytotoxicity (ADCC) is a mechanism of action of therapeutic antibodies against tumor cells. ADCC is a cell-mediated immune defense whereby an effector cell of the immune system actively lyses a target cell (e.g., a cancer cell), whose membrane-surface antigens have been bound by specific antibodies (e.g., an anti-CD40 antibody). The typical ADCC involves activation of NK cells by antibodies. An NK cell expresses CD 16 which is an Fc receptor. This receptor recognizes, and binds to, the Fc portion of an antibody bound to the surface of a target cell. The most common Fc receptor on the surface of an NK cell is called CD16 or FcγRIII. Binding of the Fc receptor to the Fc region of an antibody results in NK cell activation, release of cytolytic granules and consequent target cell apoptosis. The contribution of ADCC to tumor cell killing can be measured with a specific test that uses NK-92 cells that have been transfected with a high-affinity FcR. Results are compared to wild-type NK-92 cells that do not express the FcR.

In some embodiments, the application contemplates an anti-CD40 antibody variant (such as a full-length anti-CD40 antibody variant) comprising an Fc region that possesses some but not all effector functions, which makes it a desirable candidate for applications in which the half-life of the anti-CD40 antibody *in vivo* is important yet certain effector functions (such as CDC and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express FcyRIII only, whereas monocytes express FcγRI, FcγRII and FcyRIII. FcR expression on hematopoietic cells is summarized in Table 3 on page 464 of Ravetch and Kinet, Annu. Rev. Immunol. 9:457-492 (1991). Non-limiting examples of in vitro assays to assess ADCC activity of a molecule of interest is described in U.S. Pat. No. 5,500,362 (see, *e.g.,* Hellstrom, I. et al., Proc. Nat'l Acad. Sci. USA 83:7059-7063 (1986)) and Hellstrom, I. et al., Proc. Nat'l Acad. Sci. USA 82:1499-1502 (1985); U.S. Pat. No. 5,821,337 (see Bruggemann, M. et al., J. Exp. Med. 166:1351-1361 (1987)). Alternatively, non-radioactive assay methods may be employed (see, for example, ACTI^{™} non-radioactive cytotoxicity assay for flow cytometry (CellTechnology, Inc. Mountain View, Calif.; and CYTOTOX 96^{™} non-radioactive cytotoxicity assay (Promega, Madison, Wis.). Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes et al., Proc. Nat'l Acad. Sci. USA 95:652-656 (1998). Clq binding assays may also be carried out to confirm that the antibody is unable to bind Clq and hence lacks CDC activity. See, *e.g.,* Clq and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M. S. et al., Blood 101:1045-1052 (2003); and Cragg, M. S. and M. J. Glennie, Blood 103:2738-2743 (2004)). FcRn binding and *in vivo* clearance/half-life determinations can also be performed using methods known in the art (see, *e.g.,* Petkova, S. B. et al., Int'l. Immunol. 18(12):1759-1769 (2006)).

Antibodies with reduced effector function include those with substitution of one or more of Fc region residues 238, 265, 269, 270, 297, 327 and 329 (U.S. Pat. No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (U.S. Pat. No. 7,332,581).

Certain antibody variants with improved or diminished binding to FcRs are described. (See, *e.g.,* U.S. Pat. No. 6,737,056; WO 2004/056312, and Shields et al., J. Biol. Chem. 9(2): 6591-6604 (2001).)

In some embodiments, there is provided an anti-CD40 antibody (such as a full-length anti-CD40 antibody) variant comprising a variant Fc region comprising one or more amino acid substitutions which improve ADCC. In some embodiments, the variant Fc region comprises one or more amino acid substitutions which improve ADCC, wherein the substitutions are at positions 298, 333, and/or 334 of the variant Fc region (EU numbering of residues). In some embodiments, the anti-CD40 antibody (e.g., full-length anti-CD40 antibody) variant comprises the following amino acid substitution in its variant Fc region: S298A, E333A, and K334A.

In some embodiments, alterations are made in the Fc region that result in altered *(i.e.,* either improved or diminished) Clq binding and/or Complement Dependent Cytotoxicity (CDC), *e.g.,* as described in U.S. Pat. No. 6,194,551, WO 99/51642, and Idusogie et al., J. Immunol. 164: 4178-4184 (2000).

In some embodiments, there is provided an anti-CD40 antibody (such as a full-length anti-CD40 antibody) variant comprising a variant Fc region comprising one or more amino acid substitutions which increase half-life and/or improve binding to the neonatal Fc receptor (FcRn). Antibodies with increased half-lives and improved binding to FcRn are described in US2005/0014934A1 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or *434, e.g.,* substitution of Fc region residue 434 (U.S. Pat. No. 7,371,826).

*See also* Duncan & Winter, Nature 322:738-40 (1988); U.S. Pat. No. 5,648,260; U.S. Pat. No. 5,624,821; and WO 94/29351 concerning other examples of Fc region variants.

Anti-CD40 antibodies (such as full-length anti-CD40 antibodies) comprising any of the Fc variants described herein, or combinations thereof, are contemplated.

### Glycosylation Variants

In some embodiments, an anti-CD40 antibody (such as a full-length anti-CD40 antibody) provided herein is altered to increase or decrease the extent to which the anti-CD40 antibody is glycosylated. Addition or deletion of glycosylation sites to an anti-CD40 antibody may be conveniently accomplished by altering the amino acid sequence of the anti-CD40 antibody or polypeptide portion thereof such that one or more glycosylation sites are created or removed.

Wherein the anti-CD40 antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, *e.g.,* Wright et al., TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an anti-CD40 antibody of the application may be made in order to create anti-CD40 antibody variants with certain improved properties.

The N-glycans attached to the CH2 domain of Fc is heterogeneous. Antibodies or Fc fusion proteins generated in CHO cells are fucosylated by fucosyltransferase activity. See Shoji-Hosaka et al., J. Biochem. 2006, 140:777- 83. Normally, a small percentage of naturally occurring afucosylated IgGs may be detected in human serum. N-glycosylation of the Fc is important for binding to FcγR; and afucosylation of the N-glycan increases Fc's binding capacity to FcyRIIIa. Increased FcyRIIIa binding can enhance ADCC, which can be advantageous in certain antibody therapeutic applications in which cytotoxicity is desirable.

In some embodiments, an enhanced effector function can be detrimental when Fc-mediated cytotoxicity is undesirable. In some embodiments, the Fc fragment or CH2 domain is not glycosylated. In some embodiments, the N-glycosylation site in the CH2 domain is mutated to prevent from glycosylation.

In some embodiments, anti-CD40 antibody (such as a full-length anti-CD40 antibody) variants are provided comprising an Fc region wherein a carbohydrate structure attached to the Fc region has reduced fucose or lacks fucose, which may improve ADCC function. Specifically, anti-CD40 antibodies are contemplated herein that have reduced fucose relative to the amount of fucose on the same anti-CD40 antibody produced in a wild-type CHO cell. That is, they are characterized by having a lower amount of fucose than they would otherwise have if produced by native CHO cells (e.g., a CHO cell that produce a native glycosylation pattern, such as, a CHO cell containing a native FUT8 gene). In some embodiments, the anti-CD40 antibody is one wherein less than about 50%, 40%, 30%, 20%, 10%, or 5% of the N-linked glycans thereon comprise fucose. For example, the amount of fucose in such an anti-CD40 antibody may be from 1% to 80%, from 1% to 65%, from 5% to 65% or from 20% to 40%. In some embodiments, the anti-CD40 antibody is one wherein none of the N-linked glycans thereon comprise fucose, *i.e.,* wherein the anti-CD40 antibody is completely without fucose, or has no fucose or is afucosylated. The amount of fucose is determined by calculating the average amount of fucose within the sugar chain at Asn297, relative to the sum of all glycostructures attached to Asn 297 (e.g., complex, hybrid and high mannose structures) as measured by MALDI-TOF mass spectrometry, as described in WO 2008/077546, for example. Asn297 refers to the asparagine residue located at about position 297 in the Fc region (EU numbering of Fc region residues); however, Asn297 may also be located about ±3 amino acids upstream or downstream of position 297, *i.e.,* between positions 294 and 300, due to minor sequence variations in antibodies. Such fucosylation variants may have improved ADCC function. See, *e.g.,* US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031 140; Okazaki et al., J. Mol. Biol. 336:1239-1249 (2004); Yamane-Ohnuki et al., Biotech. Bioeng. 87: 614 (2004). Examples of cell lines capable of producing defucosylated antibodies include Lec13 CHO cells deficient in protein fucosylation (Ripka et al., Arch. Biochem. Biophys. 249:533-545 (1986); US Pat Appl No US 2003/0157108 A1, Presta, L; and WO 2004/056312 A1, Adams et al., especially at Example 11), and knockout cell lines, such asα-1,6-fucosyltransferase gene, FUT8, knockout CHO cells (see, *e.g.,* Yamane-Ohnuki et al., Biotech. Bioeng. 87: 614 (2004); Kanda, Y. et al., Biotechnol. Bioeng. 94(4):680-688 (2006); and WO2003/085107).

Anti-CD40 antibody (such as a full-length anti-CD40 antibody) variants are further provided with bisected oligosaccharides, e.g., in which a biantennary oligosaccharide attached to the Fc region of the anti-CD40 antibody is bisected by GlcNAc. Such anti-CD40 antibody (such as a full-length anti-CD40 antibody) variants may have reduced fucosylation and/or improved ADCC function. Examples of such antibody variants are described, *e.g.,* in WO 2003/011878 (Jean-Mairet et al.); U.S. Pat. No. 6,602,684 (Umana et al.); US 2005/0123546 (Umana et al.), and Ferrara et al., Biotechnology and Bioengineering, 93(5): 851-861 (2006). Anti-CD40 antibody (such as full-length anti-CD40 antibody) variants with at least one galactose residue in the oligosaccharide attached to the Fc region are also provided. Such anti-CD40 antibody variants may have improved CDC function. Such antibody variants are described, *e.g.,* in WO 1997/30087 (Patel et al.); WO 1998/58964 (Raju, S.); and WO 1999/22764 (Raju, S.).

In some embodiments, the anti-CD40 antibody (such as a full-length anti-CD40 antibody) variants comprising an Fc region are capable of binding to an FcγRIII. In some embodiments, the anti-CD40 antibody (such as a full-length anti-CD40 antibody) variants comprising an Fc region have ADCC activity in the presence of human effector cells (*e.g*., T cell) or have increased ADCC activity in the presence of human effector cells compared to the otherwise same anti-CD40 antibody (such as a full-length anti-CD40 antibody) comprising a human wild-type IgG1Fc region.

### Cysteine Engineered Variants

In some embodiments, it may be desirable to create cysteine engineered anti-CD40 antibodies (such as a full-length anti-CD40 antibody) in which one or more amino acid residues are substituted with cysteine residues. In some embodiments, the substituted residues occur at accessible sites of the anti-CD40 antibody. By substituting those residues with cysteine, reactive thiol groups are thereby positioned at accessible sites of the anti-CD40 antibody and may be used to conjugate the anti-CD40 antibody to other moieties, such as drug moieties or linker-drug moieties, to create an anti-CD40 immunoconjugate, as described further herein. Cysteine engineered anti-CD40 antibodies (*e.g*., full-length anti-CD40 antibodies) may be generated as described, *e.g*., in U.S. Pat. No. 7,521,541.

### Derivatives

In some embodiments, an anti-CD40 antibody (such as a full-length anti-CD40 antibody) provided herein may be further modified to contain additional non-proteinaceous moieties that are known in the art and readily available. The moieties suitable for derivatization of the anti-CD40 antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1,3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the anti-CD40 antibody may vary, and if more than one polymer are attached, they can be the same or different molecules. In general, the number and/or type of polymers used for derivatization can be determined based on considerations including, but not limited to, the particular properties or functions of anti-CD40 antibody to be improved, whether the anti-CD40 antibody derivative will be used in a therapy under defined conditions, *etc.*

### Pharmaceutical Compositions

Also provided herein are compositions (such as pharmaceutical compositions, also referred to herein as formulations) comprising any of the anti-CD40 antibodies (such as a full-length anti-CD40 antibody), nucleic acids encoding the antibodies, vectors comprising the nucleic acids encoding the antibodies, or host cells comprising the nucleic acids or vectors described herein. In some embodiments, there is provided a pharmaceutical composition comprising any one of the anti-CD40 antibodies described herein and a pharmaceutically acceptable carrier.

Suitable formulations of the anti-CD40 antibodies are obtained by mixing an anti-CD40 antibody having the desired degree of purity with optional pharmaceutically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propylparaben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as olyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN^{™}, PLURONICS^{™} or polyethylene glycol (PEG). Exemplary formulations are described in WO98/56418, expressly incorporated herein by reference. Lyophilized formulations adapted for subcutaneous administration are described in WO97/04801. Such lyophilized formulations may be reconstituted with a suitable diluent to a high protein concentration and the reconstituted formulation may be administered subcutaneously to the individual to be treated herein. Lipofectins or liposomes can be used to deliver the anti-CD40 antibodies of this application into cells.

The formulation herein may also contain one or more active compounds in addition to the anti-CD40 antibody (such as a full-length anti-CD40 antibody) as necessary for the particular indication being treated, preferably those with complementary activities that do not adversely affect each other. For example, it may be desirable to further provide an anti-neoplastic agent, a growth inhibitory agent, a cytotoxic agent, or a chemotherapeutic agent in addition to the anti-CD40 antibody. Such molecules are suitably present in combination in amounts that are effective for the purpose intended. The effective amount of such other agents depends on the amount of anti-CD40 antibody present in the formulation, the type of disease or disorder or treatment, and other factors discussed above. These are generally used in the same dosages and with administration routes as described herein or about from 1 to 99% of the heretofore employed dosages.

The anti-CD40 antibodies (e.g., full-length anti-CD40 antibodies) may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Sustained-release preparations may be prepared.

Sustained-release preparations of the anti-CD40 antibodies (e.g., full-length anti-CD40 antibodies) can be prepared. Suitable examples of sustained-release preparations include semipermeable matrices of solid hydrophobic polymers containing the antibody (or fragment thereof), which matrices are in the form of shaped articles, *e.g*., films, or microcapsules. Examples of sustained-release matrices include polyesters, hydrogels (for example, poly(2-hydroxyethyl-methacrylate ), or poly(vinylalcohol)), polylactides (U.S. Pat. No. 3,773,919), copolymers of L-glutamic acid and ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D (-)-3-hydroxybutyric acid. While polymers such as ethylene-vinyl acetate and lactic acid-glycolic acid enable release of molecules for over 100 days, certain hydro gels release proteins for shorter time periods. When encapsulated antibody remain in the body for a long time, they can denature or aggregate as a result of exposure to moisture at 37°C, resulting in a loss of biological activity and possible changes in immunogenicity. Rational strategies can be devised for stabilization of anti-CD40 antibodies depending on the mechanism involved. For example, if the aggregation mechanism is discovered to be intermolecular S-S bond formation through thio-disulfide interchange, stabilization can be achieved by modifying sulfhydryl residues, lyophilizing from acidic solutions, controlling moisture content, using appropriate additives, and developing specific polymer matrix compositions.

In some embodiments, the anti-CD40 antibody (such as a full-length anti-CD40 antibody) is formulated in a buffer comprising a citrate, NaCl, acetate, succinate, glycine, polysorbate 80 (Tween 80), or any combination of the foregoing.

The formulations to be used for in vivo administration must be sterile. This is readily accomplished by, *e.g.,* filtration through sterile filtration membranes.

### Methods of treatment using anti-CD40 antibodies

The anti-CD40 antibodies (e.g., full-length anti-CD40 antibodies) and/or compositions of the application can be administered to individuals (e.g., mammals such as humans) to treat a disease and/or disorder associated with CD40 signaling (e.g., autoimmune disease and/or inflammatory disease and/or cancer and/or transplantation-related disease). These diseases include, but are not limited to, Systemic Lupus Erythematosus (SLE), discoid lupus, lupus nephritis, inflammatory arthritis: including but not limited to juvenile arthritis, rheumatoid arthritis, psoriatic arthritis, Reiter's syndrome, ankylosing spondylitis and gouty arthritis, organ or tissue transplant rejection: hyperacute, acute or chronic rejection and/or graft-versus-host disease, multiple sclerosis, hyper IgE syndrome, polyarteritis nodosa, primary biliary cirrhosis, inflammatory bowel disease, Crohn's disease, celiac disease (gluten-sensitive bowel disease), autoimmune hepatitis, pernicious anemia, autoimmune hemolytic anemia, scleroderma, myasthenia gravis, idiopathic thrombocytopenic purpura, autoimmune thyroiditis, Grave's disease, Hasimoto's thyroiditis, immune complex diseases, Chronic Fatigue Immune Dysfunction Syndrome (CFIDS), polymyositis, dermatomyositis, cryoglobulinemia, thrombolysis, cardiomyopathy, pemphigus vulgaris, interstitial pulmonary fibrosis, type I and type II diabetes, type 1, 2, 3 and 4 delayed-type hypersensitivity, allergic or allergic disorders, unwanted/unintended immune reactions to therapeutic proteins(e.g., factor VII in hemophilia), asthma, Rich-Strauss syndrome (allergic granulomatosis), atopic dermatitis, allergic and irritant contact dermatitis, urticaria, IgE-mediated allergy, atherosclerosis, vasculitis, idiopathic inflammatory myopathy, hemolytic diseases, Alzheimer's disease, chronic inflammatory demyelinating polyneuropathy, Sjogren's disease, psoriasis, hidradenitis suppurativa, suppurative urethritis, sarcoidosis, ulcerative colitis, cancer: including but not limited to Non-Hodgkins lymphoma, chronic lymphocytic leukemia, multiple myeloma, B-cell lymphoma, high-level/mid-level/lower B-cell lymphoma, acute B-cell lymphoblastic leukemia, myeloblastic leukemia and Hodgkin's disease, nephritis, respiratory distress syndrome, epididymitis, glomerulonephritis, hyperthyroidism. The present application thus in some embodiments provides a method of treating a disease or condition associated with CD40 signaling (e.g., autoimmune disease and/or inflammatory disease and/or cancer and/or transplantation-related disease) in an individual comprising administering to the individual an effective amount of a composition (such as a pharmaceutical composition) comprising an anti-CD40 antibody *(e.g.,* a full-length anti-CD40 antibody), such as any one of the anti-CD40 antibodies (*e.g*., full-length anti-CD40 antibodies) described herein. In some embodiments, the individual is human.

For example, in some embodiments, there is provided a method of treating an individual having a disease or condition associated with CD40 signaling (e.g., autoimmune disease and/or inflammatory disease and/or cancer and/or transplantation-related disease) comprising administering to the individual an effective amount of a pharmaceutical composition comprising an anti-CD40 antibody (e.g., full-length anti-CD40 antibody) specifically binding to an epitope on human CD40 , wherein the epitope comprises amino acid residues of human CD40. In some embodiments, the anti-CD40 antibody is a full-length antibody. In some embodiments, the full-length anti-CD40 antibody is an IgG1 or IgG4 antibody. In some embodiments, the disease or condition is selected from the group consisting of Systemic Lupus Erythematosus (SLE), discoid lupus, lupus nephritis, inflammatory arthritis: including but not limited to juvenile arthritis, rheumatoid arthritis, psoriatic arthritis, Reiter's syndrome, ankylosing spondylitis and gouty arthritis, organ or tissue transplant rejection: hyperacute, acute or chronic rejection and/or graft-versus-host disease, multiple sclerosis, hyper IgE syndrome, polyarteritis nodosa, primary biliary cirrhosis, inflammatory bowel disease, Crohn's disease, celiac disease (gluten-sensitive bowel disease), autoimmune hepatitis, pernicious anemia, autoimmune hemolytic anemia, scleroderma, myasthenia gravis, idiopathic thrombocytopenic purpura, autoimmune thyroiditis, Grave's disease, Hasimoto's thyroiditis, immune complex diseases, Chronic Fatigue Immune Dysfunction Syndrome (CFIDS), polymyositis, dermatomyositis, cryoglobulinemia, thrombolysis, cardiomyopathy, pemphigus vulgaris, interstitial pulmonary fibrosis, type I and type II diabetes, type 1, 2, 3 and 4 delayed-type hypersensitivity, allergic or allergic disorders, unwanted/unintended immune reactions to therapeutic proteins(e.g., factor VII in hemophilia), asthma, Rich-Strauss syndrome (allergic granulomatosis), atopic dermatitis, allergic and irritant contact dermatitis, urticaria, IgE-mediated allergy, atherosclerosis, vasculitis, idiopathic inflammatory myopathy, hemolytic diseases, Alzheimer's disease, chronic inflammatory demyelinating polyneuropathy, Sjogren's disease, psoriasis, hidradenitis suppurativa, suppurative urethritis, sarcoidosis, ulcerative colitis, cancer: including but not limited to Non-Hodgkins lymphoma, chronic lymphocytic leukemia, multiple myeloma, B-cell lymphoma, high-level/mid-level/lower B-cell lymphoma, acute B-cell lymphoblastic leukemia, myeloblastic leukemia and Hodgkin's disease, nephritis, respiratory distress syndrome, epididymitis, glomerulonephritis, hyperthyroidism. In some embodiments, the individual is human.

For example, in some embodiments, there is provided a method of treating an individual having a disease or condition associated with CD40 signaling (e.g., autoimmune disease and/or inflammatory disease and/or cancer and/or transplantation-related disease) comprising administering to the individual an effective amount of a pharmaceutical composition comprising an anti-CD40 antibody (e.g., full-length anti-CD40 antibody) comprising a heavy chain variable domain (V_{H}) comprising a heavy chain complementarity determining region (HC-CDR) 1 comprising GYX₁VN (SEQ ID NO: 81), wherein X₁ is L, T, or V; an HC-CDR2 comprising X₁IX₂PX₃X₄GX₅TX₆YNQKFKG (SEQ ID NO: 82), wherein X₁ is L or M, X₂ is A, N, or V, X₃ is E, K, L, M, R, S, T, or Y, X₄ is H, N, S, or T, X₅ is G or R, and X₆ is A, S, T, or W; and an HC-CDR3 comprising SX₁X₂X₃X₄PYAX₅DY (SEQ ID NO: 83), wherein X₁ is G, I, L, M, P, R, S, or Y, X₂ is W or Y, X₃ is S or V, X₄ is D, K, L, N, R, T, or V, and X₅ is A, G, M, N, Q, or S; and a light chain variable domain (V_{L}) comprising a light chain complementarity determining region (LC-CDR) 1 comprising KASQNVHTX₁VA (SEQ ID NO: 84), wherein X₁ is A, H, N, or R; an LC-CDR2 comprising LX₁SDRRT (SEQ ID NO: 85), wherein X₁ is A or R; and an LC-CDR3 comprising LQX₁ WX₂X₃PLT (SEQ ID NO: 86), wherein X₁ is A or H, X₂ is N, S, or T, and X₃ is A, Q, R, S, or Y. In some embodiments, the anti-CD40 antibody is a full-length antibody. In some embodiments, the full-length anti-CD40 antibody is an IgG1 or IgG4 antibody. In some embodiments, the disease or condition is selected from the group consisting of Systemic Lupus Erythematosus (SLE), discoid lupus, lupus nephritis, inflammatory arthritis: including but not limited to juvenile arthritis, rheumatoid arthritis, psoriatic arthritis, Reiter's syndrome, ankylosing spondylitis and gouty arthritis, organ or tissue transplant rejection: hyperacute, acute or chronic rejection and/or graft-versus-host disease, multiple sclerosis, hyper IgE syndrome, polyarteritis nodosa, primary biliary cirrhosis, inflammatory bowel disease, Crohn's disease, celiac disease (gluten-sensitive bowel disease), autoimmune hepatitis, pernicious anemia, autoimmune hemolytic anemia, scleroderma, myasthenia gravis, idiopathic thrombocytopenic purpura, autoimmune thyroiditis, Grave's disease, Hasimoto's thyroiditis, immune complex diseases, Chronic Fatigue Immune Dysfunction Syndrome (CFIDS), polymyositis, dermatomyositis, cryoglobulinemia, thrombolysis, cardiomyopathy, pemphigus vulgaris, interstitial pulmonary fibrosis, type I and type II diabetes, type 1, 2, 3 and 4 delayed-type hypersensitivity, allergic or allergic disorders, unwanted/unintended immune reactions to therapeutic proteins(e.g., factor VII in hemophilia), asthma, Rich-Strauss syndrome (allergic granulomatosis), atopic dermatitis, allergic and irritant contact dermatitis, urticaria, IgE-mediated allergy, atherosclerosis, vasculitis, idiopathic inflammatory myopathy, hemolytic diseases, Alzheimer's disease, chronic inflammatory demyelinating polyneuropathy, Sjogren's disease, psoriasis, hidradenitis suppurativa, suppurative urethritis, sarcoidosis, ulcerative colitis, cancer: including but not limited to Non-Hodgkins lymphoma, chronic lymphocytic leukemia, multiple myeloma, B-cell lymphoma, high-level/mid-level/lower B-cell lymphoma, acute B-cell lymphoblastic leukemia, myeloblastic leukemia and Hodgkin's disease, nephritis, respiratory distress syndrome, epididymitis, glomerulonephritis, hyperthyroidism. In some embodiments, the individual is human.

In some embodiments, there is provided a method of treating an individual having a disease or condition associated with CD40 signaling (e.g., autoimmune disease and/or inflammatory disease and/or cancer and/or transplantation-related disease) comprising administering to the individual an effective amount of a pharmaceutical composition comprising an anti-CD40 antibody (e.g., full-length anti-CD40 antibody) comprising: a V_{H} comprising an HC-CDR1 comprising the amino acid sequence of any one of SEQ ID NOs: 1-3, an HC-CDR2 comprising the amino acid sequence of any one of SEQ ID NOs: 9-26, and an HC-CDR3 comprising the amino acid sequence of any one of SEQ ID NOs: 32-52, or a variant thereof comprising up to 5 amino acid substitutions in the HC-CDRs; and a V_{L} comprising an LC-CDR1 comprising the amino acid sequence of any one of SEQ ID NOs: 58-61, an LC-CDR2 comprising the amino acid sequence of any one of SEQ ID NOs: 65-66, and an LC-CDR3 comprising the amino acid sequence of any one of SEQ ID NOs: 70-77, or a variant thereof comprising up to 5 amino acid substitutions in the LC-CDRs.

In some embodiments, there is provided a method of treating an individual having a disease or condition associated with CD40 signaling (*e.g*., autoimmune disease and/or inflammatory disease and/or cancer and/or transplantation-related disease) comprising administering to the individual an effective amount of a pharmaceutical composition comprising an anti-CD40 antibody (e.g., full-length anti-CD40 antibody) comprising a V_{H} comprising the amino acid sequence of any one of SEQ ID NOs: 87-119 or a variant thereof having at least about 90% sequence identity to the amino acid sequence of any one of SEQ ID NOs: 87-119, and a V_{L} comprising the amino acid sequence of any one of SEQ ID NOs: 126-138, or a variant thereof having at least about 90% sequence identity to the amino acid sequence of any one of SEQ ID NOs: 126-138.

In some embodiments, the anti-CD40 antibody provided herein is a full-length anti-CD40 antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 145. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148.

In some embodiments, there is provided a method of treating an individual having a disease or condition associated with CD40 signaling (e.g., autoimmune disease and/or inflammatory disease and/or cancer and/or transplantation-related disease) comprising administering to the individual an effective amount of a pharmaceutical composition comprising an anti-CD40 antibody (e.g., full-length anti-CD40 antibody) comprising: a V_{H} comprising an HC-CDR1 comprising the amino acid sequence of SEQ ID NO: 1, an HC-CDR2 comprising the amino acid sequence of SEQ ID NO: 9, and an HC-CDR3 comprising the amino acid sequence of SEQ ID NO: 32, or a variant thereof comprising up to 5 amino acid substitutions in the HC-CDRs; and a V_{L} comprising an LC-CDR1 comprising the amino acid sequence of SEQ ID NO: 58, an LC-CDR2 comprising the amino acid sequence of SEQ ID NO: 65, and an LC-CDR3 comprising the amino acid sequence of SEQ ID NO: 70, or a variant thereof comprising up to 5 amino acid substitutions in the LC-CDRs.

In some embodiments, the anti-CD40 antibody provided herein comprises a V_{H} comprising the amino acid sequence of SEQ ID NO: 87, or a variant thereof having at least about 90% sequence identity to the amino acid sequence of SEQ ID NO: 87; and a V_{L} comprising the amino acid sequence of SEQ ID NO: 126, or a variant thereof having at least about 90% sequence identity to the amino acid sequence of SEQ ID NO: 126. In some embodiments, the anti-CD40 antibody provided herein is a full-length anti-CD40 antibody comprising IgG 1 or IgG4 constant domains. In some embodiments, the IgG 1 is human IgG 1. In some embodiments, the IgG4 is human IgG4. In some embodiments, the heavy chain constant region comprises or consists of the amino acid sequence of SEQ ID NO: 145. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148.

In some embodiments, the anti-CD40 antibody provided herein comprises a V_{H} comprising the amino acid sequence of SEQ ID NO: 88, or a variant thereof having at least about 90% sequence identity to the amino acid sequence of SEQ ID NO: 87; and a V_{L} comprising the amino acid sequence of SEQ ID NO: 127, or a variant thereof having at least about 90% sequence identity to the amino acid sequence of SEQ ID NO: 127. In some embodiments, the anti-CD40 antibody provided herein is a full-length anti-CD40 antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 145. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148.

In some embodiments, there is provided a method of treating an individual having a disease or condition associated with CD40 signaling (e.g., autoimmune disease and/or inflammatory disease and/or cancer and/or transplantation-related disease) comprising administering to the individual an effective amount of a pharmaceutical composition comprising an anti-CD40 antibody (*e.g*., full-length anti-CD40 antibody) comprising: a V_{H} comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 32, or a variant thereof comprising up to 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70, or a variant thereof comprising up to 5 amino acid substitutions in the LC-CDRs.

In some embodiments, the anti-CD40 antibody provided herein comprises a V_{H} comprising the amino acid sequence of SEQ ID NO: 89, or a variant thereof having at least about 90% sequence identity to the amino acid sequence of SEQ ID NO: 89; and a V_{L} comprising the amino acid sequence of SEQ ID NO: 128, or a variant thereof having at least about 90% sequence identity to the amino acid sequence of SEQ ID NO: 128. In some embodiments, the anti-CD40 antibody provided herein is a full-length anti-CD40 antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 145. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148.

In some embodiments, there is provided a method of treating an individual having a disease or condition associated with CD40 signaling (*e.g*., autoimmune disease and/or inflammatory disease and/or cancer and/or transplantation-related disease) comprising administering to the individual an effective amount of a pharmaceutical composition comprising an anti-CD40 antibody (e.g., full-length anti-CD40 antibody) comprising: a V_{H} comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 34, or a variant thereof comprising up to 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70, or a variant thereof comprising up to 5 amino acid substitutions in the LC-CDRs.

In some embodiments, the anti-CD40 antibody provided herein comprises a V_{H} comprising the amino acid sequence of SEQ ID NO: 94, or a variant thereof having at least about 90% sequence identity to the amino acid sequence of SEQ ID NO: 94; and a V_{L} comprising the amino acid sequence of SEQ ID NO: 128, or a variant thereof having at least about 90% sequence identity to the amino acid sequence of SEQ ID NO: 128. In some embodiments, the anti-CD40 antibody provided herein is a full-length anti-CD40 antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 145. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148.

In some embodiments, there is provided a method of treating an individual having a disease or condition associated with CD40 signaling (e.g., autoimmune disease and/or inflammatory disease and/or cancer and/or transplantation-related disease) comprising administering to the individual an effective amount of a pharmaceutical composition comprising an anti-CD40 antibody (e.g., full-length anti-CD40 antibody) comprising: a V_{H} comprising an HC-CDR1 comprising the amino acid sequence of SEQ ID NO: 1, an HC-CDR2 comprising the amino acid sequence of SEQ ID NO: 15, and an HC-CDR3 comprising the amino acid sequence of SEQ ID NO: 37, or a variant thereof comprising up to 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 73, or a variant thereof comprising up to 5 amino acid substitutions in the LC-CDRs.

In some embodiments, the anti-CD40 antibody provided herein comprises a V_{H} comprising the amino acid sequence of SEQ ID NO: 96, or a variant thereof having at least about 90% sequence identity to the amino acid sequence of SEQ ID NO: 96; and a V_{L} comprising the amino acid sequence of SEQ ID NO: 132, or a variant thereof having at least about 90% sequence identity to the amino acid sequence of SEQ ID NO: 132. In some embodiments, the anti-CD40 antibody provided herein is a full-length anti-CD40 antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 145. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148.

In some embodiments, there is provided a method of treating an individual having a disease or condition associated with CD40 signaling (e.g., autoimmune disease and/or inflammatory disease and/or cancer and/or transplantation-related disease) comprising administering to the individual an effective amount of a pharmaceutical composition comprising an anti-CD40 antibody (e.g., full-length anti-CD40 antibody) comprising: a V_{H} comprising an HC-CDR1 comprising the amino acid sequence of SEQ ID NO: 1, an HC-CDR2 comprising the amino acid sequence of SEQ ID NO: 20, and an HC-CDR3 comprising the amino acid sequence of SEQ ID NO: 42, or a variant thereof comprising up to 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70, or a variant thereof comprising up to 5 amino acid substitutions in the LC-CDRs.

In some embodiments, the anti-CD40 antibody provided herein comprises a V_{H} comprising the amino acid sequence of SEQ ID NO: 101, or a variant thereof having at least about 90% sequence identity to the amino acid sequence of SEQ ID NO: 101; and a V_{L} comprising the amino acid sequence of SEQ ID NO: 128, or a variant thereof having at least about 90% sequence identity to the amino acid sequence of SEQ ID NO: 128. In some embodiments, the anti-CD40 antibody provided herein is a full-length anti-CD40 antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 145. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148.

In some embodiments, there is provided a method of treating an individual having a disease or condition associated with CD40 signaling (e.g., autoimmune disease and/or inflammatory disease and/or cancer and/or transplantation-related disease) comprising administering to the individual an effective amount of a pharmaceutical composition comprising an anti-CD40 antibody (*e.g*., full-length anti-CD40 antibody) comprising: a V_{H} comprising an HC-CDR1 comprising the amino acid sequence of SEQ ID NO: 1, an HC-CDR2 comprising the amino acid sequence of SEQ ID NO: 14, and an HC-CDR3 comprising the amino acid sequence of SEQ ID NO: 43, or a variant thereof comprising up to 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70, or a variant thereof comprising up to 5 amino acid substitutions in the LC-CDRs.

In some embodiments, the anti-CD40 antibody provided herein comprises a V_{H} comprising the amino acid sequence of SEQ ID NO: 110, or a variant thereof having at least about 90% sequence identity to the amino acid sequence of SEQ ID NO: 110; and a V_{L} comprising the amino acid sequence of SEQ ID NO: 128, or a variant thereof having at least about 90% sequence identity to the amino acid sequence of SEQ ID NO: 128. In some embodiments, the anti-CD40 antibody provided herein is a full-length anti-CD40 antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 145. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148.

In some embodiments, there is provided a method of treating an individual having a disease or condition associated with CD40 signaling (e.g., autoimmune disease and/or inflammatory disease and/or cancer and/or transplantation-related disease) comprising administering to the individual an effective amount of a pharmaceutical composition comprising an anti-CD40 antibody (*e.g*., full-length anti-CD40 antibody) comprising: a V_{H} comprising an HC-CDR1 comprising the amino acid sequence of SEQ ID NO: 1, an HC-CDR2 comprising the amino acid sequence of SEQ ID NO: 15, and an HC-CDR3 comprising the amino acid sequence of SEQ ID NO: 51, or a variant thereof comprising up to 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70, or a variant thereof comprising up to 5 amino acid substitutions in the LC-CDRs.

In some embodiments, the anti-CD40 antibody provided herein comprises a V_{H} comprising the amino acid sequence of SEQ ID NO: 114, or a variant thereof having at least about 90% sequence identity to the amino acid sequence of SEQ ID NO: 114; and a V_{L} comprising the amino acid sequence of SEQ ID NO: 128, or a variant thereof having at least about 90% sequence identity to the amino acid sequence of SEQ ID NO: 128. In some embodiments, the anti-CD40 antibody provided herein is a full-length anti-CD40 antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 145. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148.

In some embodiments, there is provided a method of treating an individual having a disease or condition associated with CD40 signaling (e.g., autoimmune disease and/or inflammatory disease and/or cancer and/or transplantation-related disease) comprising administering to the individual an effective amount of a pharmaceutical composition comprising an anti-CD40 antibody (e.g., full-length anti-CD40 antibody) comprising: a V_{H} comprising an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 48, or a variant thereof comprising up to 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 71, or a variant thereof comprising up to 5 amino acid substitutions in the LC-CDRs.

In some embodiments, the anti-CD40 antibody provided herein comprises a V_{H} comprising the amino acid sequence of SEQ ID NO: 115, or a variant thereof having at least about 90% sequence identity to the amino acid sequence of SEQ ID NO: 115; and a V_{L} comprising the amino acid sequence of SEQ ID NO: 129, or a variant thereof having at least about 90% sequence identity to the amino acid sequence of SEQ ID NO: 129. In some embodiments, the anti-CD40 antibody provided herein is a full-length anti-CD40 antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 145. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148.

In some embodiments, there is provided a method of treating an individual having a disease or condition associated with CD40 signaling (e.g., autoimmune disease and/or inflammatory disease and/or cancer and/or transplantation-related disease) comprising administering to the individual an effective amount of a pharmaceutical composition comprising an anti-CD40 antibody (e.g., full-length anti-CD40 antibody) comprising: a V_{H} comprising an HC-CDR1 comprising the amino acid sequence of SEQ ID NO: 1, an HC-CDR2 comprising the amino acid sequence of SEQ ID NO: 18, and an HC-CDR3 comprising the amino acid sequence of SEQ ID NO: 47, or a variant thereof comprising up to 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 71, or a variant thereof comprising up to 5 amino acid substitutions in the LC-CDRs.

In some embodiments, the anti-CD40 antibody provided herein comprises a V_{H} comprising the amino acid sequence of SEQ ID NO: 117, or a variant thereof having at least about 90% sequence identity to the amino acid sequence of SEQ ID NO: 117; and a V_{L} comprising the amino acid sequence of SEQ ID NO: 129, or a variant thereof having at least about 90% sequence identity to the amino acid sequence of SEQ ID NO: 129. In some embodiments, the anti-CD40 antibody provided herein is a full-length anti-CD40 antibody comprising IgG1 or IgG4 constant domains. In some embodiments, the IgG1 is human IgG 1. In some embodiments, the IgG4 is human IgG4. In some embodiments, the heavy chain constant region comprises or consists of the amino acid sequence of SEQ ID NO: 145. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148.

For example, in some embodiments, there is provided a method of treating an individual having a disease or condition associated with CD40 signaling (e.g., autoimmune disease and/or inflammatory disease and/or cancer and/or transplantation-related disease) comprising administering to the individual an effective amount of a pharmaceutical composition comprising an anti-CD40 antibody (*e.g*., full-length anti-CD40 antibody) comprising a V_{H} comprising an HC-CDR1 comprising the amino acid sequence of SEQ ID NO: 4, an HC-CDR2 comprising the amino acid sequence of SEQ ID NO: 27, and an HC-CDR3 comprising the amino acid sequence of SEQ ID NO: 53, or a variant thereof comprising up to 5 amino acid substitutions in the HC-CDRs; and a V_{L} comprising an LC-CDR1 comprising the amino acid sequence of SEQ ID NO: 62, an LC-CDR2 comprising the amino acid sequence of SEQ ID NO: 67, and an LC-CDR3 comprising the amino acid sequence of SEQ ID NO: 78, or a variant thereof comprising up to 5 amino acid substitutions in the LC-CDRs.In some embodiments, the anti-CD40 antibody is a full-length antibody. In some embodiments, the full-length anti-CD40 antibody is an IgG1 or IgG4 antibody. In some embodiments, the disease or condition is selected from the group consisting of Systemic Lupus Erythematosus (SLE), discoid lupus, lupus nephritis, inflammatory arthritis: including but not limited to juvenile arthritis, rheumatoid arthritis, psoriatic arthritis, Reiter's syndrome, ankylosing spondylitis and gouty arthritis, organ or tissue transplant rejection: hyperacute, acute or chronic rejection and/or graft-versus-host disease, multiple sclerosis, hyper IgE syndrome, polyarteritis nodosa, primary biliary cirrhosis, inflammatory bowel disease, Crohn's disease, celiac disease (gluten-sensitive bowel disease), autoimmune hepatitis, pernicious anemia, autoimmune hemolytic anemia, scleroderma, myasthenia gravis, idiopathic thrombocytopenic purpura, autoimmune thyroiditis, Grave's disease, Hasimoto's thyroiditis, immune complex diseases, Chronic Fatigue Immune Dysfunction Syndrome (CFIDS), polymyositis, dermatomyositis, cryoglobulinemia, thrombolysis, cardiomyopathy, pemphigus vulgaris, interstitial pulmonary fibrosis, type I and type II diabetes, type 1, 2, 3 and 4 delayed-type hypersensitivity, allergic or allergic disorders, unwanted/unintended immune reactions to therapeutic proteins(e.g., factor VII in hemophilia), asthma, Rich-Strauss syndrome (allergic granulomatosis), atopic dermatitis, allergic and irritant contact dermatitis, urticaria, IgE-mediated allergy, atherosclerosis, vasculitis, idiopathic inflammatory myopathy, hemolytic diseases, Alzheimer's disease, chronic inflammatory demyelinating polyneuropathy, Sjogren's disease, psoriasis, hidradenitis suppurativa, suppurative urethritis, sarcoidosis, ulcerative colitis, cancer: including but not limited to Non-Hodgkins lymphoma, chronic lymphocytic leukemia, multiple myeloma, B-cell lymphoma, high-level/mid-level/lower B-cell lymphoma, acute B-cell lymphoblastic leukemia, myeloblastic leukemia and Hodgkin's disease, nephritis, respiratory distress syndrome, epididymitis, glomerulonephritis, hyperthyroidism. In some embodiments, the individual is human.

In some embodiments, there is provided a method of treating an individual having a disease or condition associated with CD40 signaling (e.g., autoimmune disease and/or inflammatory disease and/or cancer and/or transplantation-related disease) comprising administering to the individual an effective amount of a pharmaceutical composition comprising an anti-CD40 antibody (e.g., full-length anti-CD40 antibody) comprising a V_{H} comprising the amino acid sequence of SEQ ID NO: 120 or a variant thereof having at least about 90% sequence identity to the amino acid sequence of SEQ ID NO: 120, and a V_{L} comprising the amino acid sequence of SEQ ID NO: 139, or a variant thereof having at least about 90% sequence identity to the amino acid sequence of SEQ ID NO: 139.

In some embodiments, the anti-CD40 antibody provided herein is a full-length anti-CD40 antibody comprising IgG1 or IgG4 constant domains. In some embodiments, the IgG 1 is human IgG 1. In some embodiments, the IgG4 is human IgG4. In some embodiments, the heavy chain constant region comprises or consists of the amino acid sequence of SEQ ID NO: 145. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148.

For example, in some embodiments, there is provided a method of treating an individual having a disease or condition associated with CD40 signaling (e.g., autoimmune disease and/or inflammatory disease and/or cancer and/or transplantation-related disease) comprising administering to the individual an effective amount of a pharmaceutical composition comprising an anti-CD40 antibody (e.g., full-length anti-CD40 antibody) comprising a V_{H} comprising an HC-CDR1 comprising the amino acid sequence of SEQ ID NO: 5, an HC-CDR2 comprising the amino acid sequence of SEQ ID NO: 27, and an HC-CDR3 comprising the amino acid sequence of SEQ ID NO: 54, or a variant thereof comprising up to 5 amino acid substitutions in the HC-CDRs; and a V_{L} comprising an LC-CDR1 comprising the amino acid sequence of SEQ ID NO: 62, an LC-CDR2 comprising the amino acid sequence of SEQ ID NO: 67, and an LC-CDR3 comprising the amino acid sequence of SEQ ID NO: 78, or a variant thereof comprising up to 5 amino acid substitutions in the LC-CDRs.In some embodiments, the anti-CD40 antibody is a full-length antibody. In some embodiments, the full-length anti-CD40 antibody is an IgG1 or IgG4 antibody. In some embodiments, the disease or condition is selected from the group consisting of Systemic Lupus Erythematosus (SLE), discoid lupus, lupus nephritis, inflammatory arthritis: including but not limited to juvenile arthritis, rheumatoid arthritis, psoriatic arthritis, Reiter's syndrome, ankylosing spondylitis and gouty arthritis, organ or tissue transplant rejection: hyperacute, acute or chronic rejection and/or graft-versus-host disease, multiple sclerosis, hyper IgE syndrome, polyarteritis nodosa, primary biliary cirrhosis, inflammatory bowel disease, Crohn's disease, celiac disease (gluten-sensitive bowel disease), autoimmune hepatitis, pernicious anemia, autoimmune hemolytic anemia, scleroderma, myasthenia gravis, idiopathic thrombocytopenic purpura, autoimmune thyroiditis, Grave's disease, Hasimoto's thyroiditis, immune complex diseases, Chronic Fatigue Immune Dysfunction Syndrome (CFIDS), polymyositis, dermatomyositis, cryoglobulinemia, thrombolysis, cardiomyopathy, pemphigus vulgaris, interstitial pulmonary fibrosis, type I and type II diabetes, type 1, 2, 3 and 4 delayed-type hypersensitivity, allergic or allergic disorders, unwanted/unintended immune reactions to therapeutic proteins(e.g., factor VII in hemophilia), asthma, Rich-Strauss syndrome (allergic granulomatosis), atopic dermatitis, allergic and irritant contact dermatitis, urticaria, IgE-mediated allergy, atherosclerosis, vasculitis, idiopathic inflammatory myopathy, hemolytic diseases, Alzheimer's disease, chronic inflammatory demyelinating polyneuropathy, Sjogren's disease, psoriasis, hidradenitis suppurativa, suppurative urethritis, sarcoidosis, ulcerative colitis, cancer: including but not limited to Non-Hodgkins lymphoma, chronic lymphocytic leukemia, multiple myeloma, B-cell lymphoma, high-level/mid-level/lower B-cell lymphoma, acute B-cell lymphoblastic leukemia, myeloblastic leukemia and Hodgkin's disease, nephritis, respiratory distress syndrome, epididymitis, glomerulonephritis, hyperthyroidism. In some embodiments, the individual is human.

In some embodiments, there is provided a method of treating an individual having a disease or condition associated with CD40 signaling (e.g., autoimmune disease and/or inflammatory disease and/or cancer and/or transplantation-related disease) comprising administering to the individual an effective amount of a pharmaceutical composition comprising an anti-CD40 antibody (e.g., full-length anti-CD40 antibody) comprising a V_{H} comprising the amino acid sequence of SEQ ID NO: 121 or a variant thereof having at least about 90% sequence identity to the amino acid sequence of SEQ ID NO: 121, and a V_{L} comprising the amino acid sequence of SEQ ID NO: 140, or a variant thereof having at least about 90% sequence identity to the amino acid sequence of SEQ ID NO: 140.

In some embodiments, the anti-CD40 antibody provided herein is a full-length anti-CD40 antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 145. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148.

For example, in some embodiments, there is provided a method of treating an individual having a disease or condition associated with CD40 signaling (e.g., autoimmune disease and/or inflammatory disease and/or cancer and/or transplantation-related disease) comprising administering to the individual an effective amount of a pharmaceutical composition comprising an anti-CD40 antibody (*e.g*., full-length anti-CD40 antibody) comprising a V_{H} comprising an HC-CDR1 comprising the amino acid sequence of SEQ ID NO: 6, an HC-CDR2 comprising the amino acid sequence of SEQ ID NO: 28, and an HC-CDR3 comprising the amino acid sequence of SEQ ID NO: 55, or a variant thereof comprising up to 5 amino acid substitutions in the HC-CDRs; and a V_{L} comprising an LC-CDR1 comprising the amino acid sequence of SEQ ID NO: 63, an LC-CDR2 comprising the amino acid sequence of SEQ ID NO: 68, and an LC-CDR3 comprising the amino acid sequence of SEQ ID NO: 79, or a variant thereof comprising up to 5 amino acid substitutions in the LC-CDRs.In some embodiments, the anti-CD40 antibody is a full-length antibody. In some embodiments, the full-length anti-CD40 antibody is an IgG1 or IgG4 antibody. In some embodiments, the disease or condition is selected from the group consisting of Systemic Lupus Erythematosus (SLE), discoid lupus, lupus nephritis, inflammatory arthritis: including but not limited to juvenile arthritis, rheumatoid arthritis, psoriatic arthritis, Reiter's syndrome, ankylosing spondylitis and gouty arthritis, organ or tissue transplant rejection: hyperacute, acute or chronic rejection and/or graft-versus-host disease, multiple sclerosis, hyper IgE syndrome, polyarteritis nodosa, primary biliary cirrhosis, inflammatory bowel disease, Crohn's disease, celiac disease (gluten-sensitive bowel disease), autoimmune hepatitis, pernicious anemia, autoimmune hemolytic anemia, scleroderma, myasthenia gravis, idiopathic thrombocytopenic purpura, autoimmune thyroiditis, Grave's disease, Hasimoto's thyroiditis, immune complex diseases, Chronic Fatigue Immune Dysfunction Syndrome (CFIDS), polymyositis, dermatomyositis, cryoglobulinemia, thrombolysis, cardiomyopathy, pemphigus vulgaris, interstitial pulmonary fibrosis, type I and type II diabetes, type 1, 2, 3 and 4 delayed-type hypersensitivity, allergic or allergic disorders, unwanted/unintended immune reactions to therapeutic proteins(e.g., factor VII in hemophilia), asthma, Rich-Strauss syndrome (allergic granulomatosis), atopic dermatitis, allergic and irritant contact dermatitis, urticaria, IgE-mediated allergy, atherosclerosis, vasculitis, idiopathic inflammatory myopathy, hemolytic diseases, Alzheimer's disease, chronic inflammatory demyelinating polyneuropathy, Sjogren's disease, psoriasis, hidradenitis suppurativa, suppurative urethritis, sarcoidosis, ulcerative colitis, cancer: including but not limited to Non-Hodgkins lymphoma, chronic lymphocytic leukemia, multiple myeloma, B-cell lymphoma, high-level/mid-level/lower B-cell lymphoma, acute B-cell lymphoblastic leukemia, myeloblastic leukemia and Hodgkin's disease, nephritis, respiratory distress syndrome, epididymitis, glomerulonephritis, hyperthyroidism. In some embodiments, the individual is human.

In some embodiments, there is provided a method of treating an individual having a disease or condition associated with CD40 signaling (*e.g*., autoimmune disease and/or inflammatory disease and/or cancer and/or transplantation-related disease) comprising administering to the individual an effective amount of a pharmaceutical composition comprising an anti-CD40 antibody (*e.g*., full-length anti-CD40 antibody) comprising a V_{H} comprising the amino acid sequence of SEQ ID NO: 122 or a variant thereof having at least about 90% sequence identity to the amino acid sequence of SEQ ID NO: 122, and a V_{L} comprising the amino acid sequence of SEQ ID NO: 141, or a variant thereof having at least about 90% sequence identity to the amino acid sequence of SEQ ID NO: 141.

In some embodiments, the anti-CD40 antibody provided herein is a full-length anti-CD40 antibody comprising IgG1 or IgG4 constant domains. In some embodiments, the IgG1 is human IgG 1. In some embodiments, the IgG4 is human IgG4. In some embodiments, the heavy chain constant region comprises or consists of the amino acid sequence of SEQ ID NO: 145. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148.

For example, in some embodiments, there is provided a method of treating an individual having a disease or condition associated with CD40 signaling (e.g., autoimmune disease and/or inflammatory disease and/or cancer and/or transplantation-related disease) comprising administering to the individual an effective amount of a pharmaceutical composition comprising an anti-CD40 antibody (e.g., full-length anti-CD40 antibody) comprising a V_{H} comprising an HC-CDR1 comprising the amino acid sequence of SEQ ID NO: 7, an HC-CDR2 comprising the amino acid sequence of SEQ ID NO: 29, and an HC-CDR3 comprising the amino acid sequence of SEQ ID NO: 56, or a variant thereof comprising up to 5 amino acid substitutions in the HC-CDRs; and a V_{L} comprising an LC-CDR1 comprising the amino acid sequence of SEQ ID NO: 64, an LC-CDR2 comprising the amino acid sequence of SEQ ID NO: 69, and an LC-CDR3 comprising the amino acid sequence of SEQ ID NO: 80, or a variant thereof comprising up to 5 amino acid substitutions in the LC-CDRs.In some embodiments, the anti-CD40 antibody is a full-length antibody. In some embodiments, the full-length anti-CD40 antibody is an IgG1 or IgG4 antibody. In some embodiments, the disease or condition is selected from the group consisting of Systemic Lupus Erythematosus (SLE), discoid lupus, lupus nephritis, inflammatory arthritis: including but not limited to juvenile arthritis, rheumatoid arthritis, psoriatic arthritis, Reiter's syndrome, ankylosing spondylitis and gouty arthritis, organ or tissue transplant rejection: hyperacute, acute or chronic rejection and/or graft-versus-host disease, multiple sclerosis, hyper IgE syndrome, polyarteritis nodosa, primary biliary cirrhosis, inflammatory bowel disease, Crohn's disease, celiac disease (gluten-sensitive bowel disease), autoimmune hepatitis, pernicious anemia, autoimmune hemolytic anemia, scleroderma, myasthenia gravis, idiopathic thrombocytopenic purpura, autoimmune thyroiditis, Grave's disease, Hasimoto's thyroiditis, immune complex diseases, Chronic Fatigue Immune Dysfunction Syndrome (CFIDS), polymyositis, dermatomyositis, cryoglobulinemia, thrombolysis, cardiomyopathy, pemphigus vulgaris, interstitial pulmonary fibrosis, type I and type II diabetes, type 1, 2, 3 and 4 delayed-type hypersensitivity, allergic or allergic disorders, unwanted/unintended immune reactions to therapeutic proteins(e.g., factor VII in hemophilia), asthma, Rich-Strauss syndrome (allergic granulomatosis), atopic dermatitis, allergic and irritant contact dermatitis, urticaria, IgE-mediated allergy, atherosclerosis, vasculitis, idiopathic inflammatory myopathy, hemolytic diseases, Alzheimer's disease, chronic inflammatory demyelinating polyneuropathy, Sjogren's disease, psoriasis, hidradenitis suppurativa, suppurative urethritis, sarcoidosis, ulcerative colitis, cancer: including but not limited to Non-Hodgkins lymphoma, chronic lymphocytic leukemia, multiple myeloma, B-cell lymphoma, high-level/mid-level/lower B-cell lymphoma, acute B-cell lymphoblastic leukemia, myeloblastic leukemia and Hodgkin's disease, nephritis, respiratory distress syndrome, epididymitis, glomerulonephritis, hyperthyroidism. In some embodiments, the individual is human.

In some embodiments, there is provided a method of treating an individual having a disease or condition associated with CD40 signaling (e.g., autoimmune disease and/or inflammatory disease and/or cancer and/or transplantation-related disease) comprising administering to the individual an effective amount of a pharmaceutical composition comprising an anti-CD40 antibody (e.g., full-length anti-CD40 antibody) comprising a V_{H} comprising the amino acid sequence of SEQ ID NO: 123 or a variant thereof having at least about 90% sequence identity to the amino acid sequence of SEQ ID NO: 123, and a V_{L} comprising the amino acid sequence of SEQ ID NO: 142, or a variant thereof having at least about 90% sequence identity to the amino acid sequence of SEQ ID NO: 142.

In some embodiments, the anti-CD40 antibody provided herein is a full-length anti-CD40 antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 145. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148.

For example, in some embodiments, there is provided a method of treating an individual having a disease or condition associated with CD40 signaling (e.g., autoimmune disease and/or inflammatory disease and/or cancer and/or transplantation-related disease) comprising administering to the individual an effective amount of a pharmaceutical composition comprising an anti-CD40 antibody (e.g., full-length anti-CD40 antibody) comprising a V_{H} comprising an HC-CDR1 comprising the amino acid sequence of SEQ ID NO: 8, an HC-CDR2 comprising the amino acid sequence of SEQ ID NO: 30, and an HC-CDR3 comprising the amino acid sequence of SEQ ID NO: 57, or a variant thereof comprising up to 5 amino acid substitutions in the HC-CDRs; and a V_{L} comprising an LC-CDR1 comprising the amino acid sequence of SEQ ID NO: 62, an LC-CDR2 comprising the amino acid sequence of SEQ ID NO: 67, and an LC-CDR3 comprising the amino acid sequence of SEQ ID NO: 80, or a variant thereof comprising up to 5 amino acid substitutions in the LC-CDRs.In some embodiments, the anti-CD40 antibody is a full-length antibody. In some embodiments, the full-length anti-CD40 antibody is an IgG 1 or IgG4 antibody. In some embodiments, the disease or condition is selected from the group consisting of Systemic Lupus Erythematosus (SLE), discoid lupus, lupus nephritis, inflammatory arthritis: including but not limited to juvenile arthritis, rheumatoid arthritis, psoriatic arthritis, Reiter's syndrome, ankylosing spondylitis and gouty arthritis, organ or tissue transplant rejection: hyperacute, acute or chronic rejection and/or graft-versus-host disease, multiple sclerosis, hyper IgE syndrome, polyarteritis nodosa, primary biliary cirrhosis, inflammatory bowel disease, Crohn's disease, celiac disease (gluten-sensitive bowel disease), autoimmune hepatitis, pernicious anemia, autoimmune hemolytic anemia, scleroderma, myasthenia gravis, idiopathic thrombocytopenic purpura, autoimmune thyroiditis, Grave's disease, Hasimoto's thyroiditis, immune complex diseases, Chronic Fatigue Immune Dysfunction Syndrome (CFIDS), polymyositis, dermatomyositis, cryoglobulinemia, thrombolysis, cardiomyopathy, pemphigus vulgaris, interstitial pulmonary fibrosis, type I and type II diabetes, type 1, 2, 3 and 4 delayed-type hypersensitivity, allergic or allergic disorders, unwanted/unintended immune reactions to therapeutic proteins(e.g., factor VII in hemophilia), asthma, Rich-Strauss syndrome (allergic granulomatosis), atopic dermatitis, allergic and irritant contact dermatitis, urticaria, IgE-mediated allergy, atherosclerosis, vasculitis, idiopathic inflammatory myopathy, hemolytic diseases, Alzheimer's disease, chronic inflammatory demyelinating polyneuropathy, Sjogren's disease, psoriasis, hidradenitis suppurativa, suppurative urethritis, sarcoidosis, ulcerative colitis, cancer: including but not limited to Non-Hodgkins lymphoma, chronic lymphocytic leukemia, multiple myeloma, B-cell lymphoma, high-level/mid-level/lower B-cell lymphoma, acute B-cell lymphoblastic leukemia, myeloblastic leukemia and Hodgkin's disease, nephritis, respiratory distress syndrome, epididymitis, glomerulonephritis, hyperthyroidism. In some embodiments, the individual is human.

In some embodiments, there is provided a method of treating an individual having a disease or condition associated with CD40 signaling (*e.g*., autoimmune disease and/or inflammatory disease and/or cancer and/or transplantation-related disease) comprising administering to the individual an effective amount of a pharmaceutical composition comprising an anti-CD40 antibody (e.g., full-length anti-CD40 antibody) comprising a V_{H} comprising the amino acid sequence of SEQ ID NO: 124 or a variant thereof having at least about 90% sequence identity to the amino acid sequence of SEQ ID NO: 124, and a V_{L} comprising the amino acid sequence of SEQ ID NO: 143, or a variant thereof having at least about 90% sequence identity to the amino acid sequence of SEQ ID NO: 143.

In some embodiments, the anti-CD40 antibody provided herein is a full-length anti-CD40 antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 145. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148.

For example, in some embodiments, there is provided a method of treating an individual having a disease or condition associated with CD40 signaling (*e.g*., autoimmune disease and/or inflammatory disease and/or cancer and/or transplantation-related disease) comprising administering to the individual an effective amount of a pharmaceutical composition comprising an anti-CD40 antibody (e.g., full-length anti-CD40 antibody) comprising a V_{H} comprising an HC-CDR1 comprising the amino acid sequence of SEQ ID NO: 4, an HC-CDR2 comprising the amino acid sequence of SEQ ID NO: 31, and an HC-CDR3 comprising the amino acid sequence of SEQ ID NO: 53, or a variant thereof comprising up to 5 amino acid substitutions in the HC-CDRs; and a V_{L} comprising an LC-CDR1 comprising the amino acid sequence of SEQ ID NO: 62, an LC-CDR2 comprising the amino acid sequence of SEQ ID NO: 68, and an LC-CDR3 comprising the amino acid sequence of SEQ ID NO: 79, or a variant thereof comprising up to 5 amino acid substitutions in the LC-CDRs.In some embodiments, the anti-CD40 antibody is a full-length antibody. In some embodiments, the full-length anti-CD40 antibody is an IgG 1 or IgG4 antibody. In some embodiments, the disease or condition is selected from the group consisting of Systemic Lupus Erythematosus (SLE), discoid lupus, lupus nephritis, inflammatory arthritis: including but not limited to juvenile arthritis, rheumatoid arthritis, psoriatic arthritis, Reiter's syndrome, ankylosing spondylitis and gouty arthritis, organ or tissue transplant rejection: hyperacute, acute or chronic rejection and/or graft-versus-host disease, multiple sclerosis, hyper IgE syndrome, polyarteritis nodosa, primary biliary cirrhosis, inflammatory bowel disease, Crohn's disease, celiac disease (gluten-sensitive bowel disease), autoimmune hepatitis, pernicious anemia, autoimmune hemolytic anemia, scleroderma, myasthenia gravis, idiopathic thrombocytopenic purpura, autoimmune thyroiditis, Grave's disease, Hasimoto's thyroiditis, immune complex diseases, Chronic Fatigue Immune Dysfunction Syndrome (CFIDS), polymyositis, dermatomyositis, cryoglobulinemia, thrombolysis, cardiomyopathy, pemphigus vulgaris, interstitial pulmonary fibrosis, type I and type II diabetes, type 1, 2, 3 and 4 delayed-type hypersensitivity, allergic or allergic disorders, unwanted/unintended immune reactions to therapeutic proteins(e.g., factor VII in hemophilia), asthma, Rich-Strauss syndrome (allergic granulomatosis), atopic dermatitis, allergic and irritant contact dermatitis, urticaria, IgE-mediated allergy, atherosclerosis, vasculitis, idiopathic inflammatory myopathy, hemolytic diseases, Alzheimer's disease, chronic inflammatory demyelinating polyneuropathy, Sjogren's disease, psoriasis, hidradenitis suppurativa, suppurative urethritis, sarcoidosis, ulcerative colitis, cancer: including but not limited to Non-Hodgkins lymphoma, chronic lymphocytic leukemia, multiple myeloma, B-cell lymphoma, high-level/mid-level/lower B-cell lymphoma, acute B-cell lymphoblastic leukemia, myeloblastic leukemia and Hodgkin's disease, nephritis, respiratory distress syndrome, epididymitis, glomerulonephritis, hyperthyroidism. In some embodiments, the individual is human.

In some embodiments, there is provided a method of treating an individual having a disease or condition associated with CD40 signaling (e.g., autoimmune disease and/or inflammatory disease and/or cancer and/or transplantation-related disease) comprising administering to the individual an effective amount of a pharmaceutical composition comprising an anti-CD40 antibody (e.g., full-length anti-CD40 antibody) comprising a V_{H} comprising the amino acid sequence of SEQ ID NO: 125 or a variant thereof having at least about 90% sequence identity to the amino acid sequence of SEQ ID NO: 125, and a V_{L} comprising the amino acid sequence of SEQ ID NO: 144, or a variant thereof having at least about 90% sequence identity to the amino acid sequence of SEQ ID NO: 144.

In some embodiments, the anti-CD40 antibody provided herein is a full-length anti-CD40 antibody comprising IgG1 or IgG4 constant domains. In some embodiments, the IgG1 is human IgG1. In some embodiments, the IgG4 is human IgG4. In some embodiments, the heavy chain constant region comprises or consists of the amino acid sequence of SEQ ID NO: 145. In some embodiments, the heavy chain constant region comprises or consists of the amino acid sequence of SEQ ID NO: 146. In some embodiments, the light chain constant region comprises or consists of the amino acid sequence of SEQ ID NO: 147. In some embodiments, the light chain constant region comprises or consists of the amino acid sequence of SEQ ID NO: 148.

In some embodiments, the individual is a mammal (*e.g*., human, non-human primate, rat, mouse, cow, horse, pig, sheep, goat, dog, cat, *etc*.)*.* In some embodiments, the individual is a human. In some embodiments, the individual is a clinical patient, a clinical trial volunteer, an experimental animal, *etc.* In some embodiments, the individual is younger than about 60 years old (including for example younger than about any of 50, 40, 30, 25, 20, 15, or 10 years old). In some embodiments, the individual is older than about 60 years old (including for example older than about any of 70, 80, 90, or 100 years old). In some embodiments, the individual is diagnosed with or genetically prone to one or more of the diseases or disorders described herein (such as autoimmune disease and/or inflammatory disease and/or cancer and/or transplantation-related disease). In some embodiments, the individual has one or more risk factors associated with one or more diseases or disorders described herein.

The present application in some embodiments provides a method of delivering an anti-CD40 antibody (such as any one of the anti-CD40 antibodies described herein, *e.g*., an isolated anti-CD40 antibody) to a cell producing CD40 in an individual, the method comprising administering to the individual a composition comprising the anti-CD40 antibody.

Many diagnostic methods for autoimmune disease and/or inflammatory disease and/or cancer and/or transplantation-related disease or any other disease associated with CD40 abnormal expression and the clinical delineation of those diseases are known in the art. Such methods include, but are not limited to, *e.g*., immunohistochemistry, PCR, and fluorescent in situ hybridization (FISH).

In some embodiments, the anti-CD40 antibodies (e.g., full-length anti-CD40 antibodies) and/or compositions of the application are administered in combination with a second, third, or fourth agent (including, *e.g*., immunosuppressive and/or immunomodulatory and/or anti-inflammatory agent) to treat diseases or disorders associated with CD40 signaling.

Methods for assessing immunosuppressive activity of agents are well known in the art. For example, the length of time a transplanted organ survives in vivo with and without pharmacological intervention is used as a quantitative indicator of immune response suppression. In vitro assays, such as Mixed Lymphocyte Reaction (MLR) assays (see, *e.g*., Fathman et al, J.Immunol. 118:1232-8,1977), can also be used; CD3 assay (specific activation of immune cells by anti-CD 3 antibodies (e.g., OKT 3)) (see, e.g., Khanna et al, Transplantation 67:882-9, 1999; Khanna et al, (1999) Transplantation 67: S58); and IL-2R assay (specific activation of immune cells with exogenously added cytokine IL-2) (see, e.g., Farrar et al, J.Immuno1.126:1120-5, 1981).

Methods for assessing therapeutic response of autoimmune and/or inflammatory diseases are well known in the art. Beneficial results that can be achieved by administering the pharmaceutical composition of the invention to a subject suffering from an inflammatory or autoimmune disease include a reduction in secretion of inflammatory cytokines, adhesion molecules, proteases, immunoglobulins, or a combination thereof, and the like, an increase in production of anti-inflammatory proteins, a reduction in the number of autoreactive cells, an increase in immune tolerance, inhibition of survival of autoreactive cells, and/or a reduction in one or more symptoms mediated by cells expressing CD40. Such positive therapeutic response is not limited to the route of administration and can include administration to a donor, administration to a tissue of a donor (e.g., organ perfusion), administration to a host, any combination thereof, and the like.

Clinical responses can be assessed using screening techniques such as Magnetic Resonance Imaging (MRI) scans, radiographic imaging, Computed Tomography (CT) scans, flow cytometry or Fluorescence Activated Cell Sorter (FACS) analysis, histology, gross pathology, and blood chemistry, including but not limited to changes detectable by ELISA, RIA, chromatography, and the like.

### Dosing and method of administering the anti-CD40 antibodies

The dose of the anti-CD40 antibody (such as isolated anti-CD40 antibody) compositions administered to an individual (such as a human) may vary with the particular composition, the mode of administration, and the type of disease being treated. In some embodiments, the amount of the composition (such as composition comprising isolated anti-CD40 antibody) is effective to result in an objective response (such as a partial response or a complete response) in the treatment of autoimmune disease and/or inflammatory disease and/or cancer and/or transplantation-related disease. In some embodiments, the amount of the anti-CD40 antibody composition is sufficient to result in a complete response in the individual. In some embodiments, the amount of the anti-CD40 antibody composition is sufficient to result in a partial response in the individual. In some embodiments, the amount of the anti-CD40 antibody composition administered (for example when administered alone) is sufficient to produce an overall response rate of more than about any of 20%, 25%, 30%, 35%, 40%, 45%, 50%, 55%, 60%, 64%, 65%, 70%, 75%, 80%, 85%, or 90% among a population of individuals treated with the anti-CD40 antibody composition. Responses of an individual to the treatment of the methods described herein can be determined, for example, based on the reduction of inflammatory cytokines.

In some embodiments, the amount of the composition (such as composition comprising isolated anti-CD40 antibody) is sufficient to control symptoms and reduce the risk of exacerbations of the individual. In some embodiments, the amount of the composition is sufficient to control symptoms and reduce the risk of exacerbations of the individual. In some embodiments, the amount of the composition (for example when administered along) is sufficient to produce clinical benefit of more than about any of 50%, 60%, 70%, or 77% among a population of individuals treated with the anti-CD40 antibody composition.

In some embodiments, the amount of the composition (such as composition comprising isolated anti-CD40 antibody), alone or in combination with a second, third, and/or fourth agent, is an amount sufficient to alleviate or reduce the severity, number of symptoms and reduce the frequency of exacerbations by at least about any of 10%, 20%, 30%, 40%, 50%, 60%, 70%, 80%, 90%, 95% or 100% in the same subject prior to treatment or compared to the corresponding activity in other subjects not receiving the treatment. Standard methods can be used to measure the magnitude of this effect, such as *in vitro* assays with purified enzyme, cell-based assays, animal models, or human testing.

In some embodiments, the amount of the anti-CD40 antibody (such as a full-length anti-CD40 antibody) in the composition is below the level that induces a toxicological effect (*i.e.,* an effect above a clinically acceptable level of toxicity) or is at a level where a potential side effect can be controlled or tolerated when the composition is administered to the individual.

In some embodiments, the amount of the composition is close to a maximum tolerated dose (MTD) of the composition following the same dosing regimen. In some embodiments, the amount of the composition is more than about any of 80%, 90%, 95%, or 98% of the MTD.

In some embodiments, the amount of an anti-CD40 antibody (such as a full-length anti-CD40 antibody) in the composition is included in a range of about 0.001 µg to about 1000 µg.

In some embodiments of any of the above aspects, the effective amount of anti-CD40 antibody (such as a full-length anti-CD40 antibody) in the composition is in the range of about 0.1 µg/kg to about 100 mg/kg of total body weight.

The anti-CD40 antibody compositions can be administered to an individual (such as human) via various routes, including, for example, intravenous, intra-arterial, intraperitoneal, intrapulmonary, oral, inhalation, intravesicular, intramuscular, intra-tracheal, subcutaneous, intraocular, intrathecal, transmucosal or transdermal. In some embodiments, sustained continuous release formulation of the composition may be used. In some embodiments, the composition is administered inhaled. In some embodiments, the composition is administered intravenously. In some embodiments, the composition is administered intraportally. In some embodiments, the composition is administered intraarterially. In some embodiments, the composition is administered intraperitoneally. In some embodiments, the composition is administered intrahepatically. In some embodiments, the composition is administered by hepatic arterial infusion. In some embodiments, the administration is to an injection site distal to a first disease site.

### Articles of Manufacture and Kits

In some embodiments of the application, there is provided an article of manufacture containing materials useful for the treatment of disease or condition associated with CD40 signaling, (e.g., autoimmune disease and/or inflammatory disease and/or cancer and/or transplantation-related disease) or for delivering an anti-CD40 antibody (such as a full-length anti-CD40 antibody) to a cell producing CD40 of the individual. The article of manufacture can comprise a container and a label or package insert on or associated with the container. Suitable containers include, for example, bottles, vials, syringes, *etc.* The containers may be formed from a variety of materials such as glass or plastic. Generally, the container holds a composition which is effective for treating a disease or disorder described herein, and may have a sterile access port (for example the container may be an intravenous solution bag or a vial having a stopper pierceable by a hypodermic injection needle). At least one active agent in the composition is an anti-CD40 antibody of the application. The label or package insert indicates that the composition is used for treating the particular condition. The label or package insert will further comprise instructions for administering the anti-CD40 antibody composition to the patient. Articles of manufacture and kits comprising combinatorial therapies described herein are also contemplated.

Package insert refers to instructions customarily included in commercial packages of therapeutic products that contain information about the indications, usage, dosage, administration, contraindications and/or warnings concerning the use of such therapeutic products. In some embodiments, the package insert indicates that the composition is used for treating disease or condition associated with CD40 signaling (such as autoimmune disease and/or inflammatory disease and/or cancer and/or transplantation-related disease). In some embodiments, the package insert indicates that the composition is used for treating disease or condition selected from the group consisting of Systemic Lupus Erythematosus (SLE), discoid lupus, lupus nephritis, inflammatory arthritis: including but not limited to juvenile arthritis, rheumatoid arthritis, psoriatic arthritis, Reiter's syndrome, ankylosing spondylitis and gouty arthritis, organ or tissue transplant rejection: hyperacute, acute or chronic rejection and/or graft-versus-host disease, multiple sclerosis, hyper IgE syndrome, polyarteritis nodosa, primary biliary cirrhosis, inflammatory bowel disease, Crohn's disease, celiac disease (gluten-sensitive bowel disease), autoimmune hepatitis, pernicious anemia, autoimmune hemolytic anemia, scleroderma, myasthenia gravis, idiopathic thrombocytopenic purpura, autoimmune thyroiditis, Grave's disease, Hasimoto's thyroiditis, immune complex diseases, Chronic Fatigue Immune Dysfunction Syndrome (CFIDS), polymyositis, dermatomyositis, cryoglobulinemia, thrombolysis, cardiomyopathy, pemphigus vulgaris, interstitial pulmonary fibrosis, type I and type II diabetes, type 1, 2, 3 and 4 delayed-type hypersensitivity, allergic or allergic disorders, unwanted/unintended immune reactions to therapeutic proteins(e.g., factor VII in hemophilia), asthma, Rich-Strauss syndrome (allergic granulomatosis), atopic dermatitis, allergic and irritant contact dermatitis, urticaria, IgE-mediated allergy, atherosclerosis, vasculitis, idiopathic inflammatory myopathy, hemolytic diseases, Alzheimer's disease, chronic inflammatory demyelinating polyneuropathy, Sjogren's disease, psoriasis, hidradenitis suppurativa, suppurative urethritis, sarcoidosis, ulcerative colitis, cancer: including but not limited to Non-Hodgkins lymphoma, chronic lymphocytic leukemia, multiple myeloma, B-cell lymphoma, high-level/mid-level/lower B-cell lymphoma, acute B-cell lymphoblastic leukemia, myeloblastic leukemia and Hodgkin's disease, nephritis, respiratory distress syndrome, epididymitis, glomerulonephritis, hyperthyroidism.

Additionally, the article of manufacture may further comprise a second container comprising a pharmaceutically-acceptable buffer, such as bacteriostatic water for injection (BWFI), phosphate-buffered saline, Ringer's solution or dextrose solution. It may further include other materials desirable from a commercial and user standpoint, including other buffers, diluents, filters, needles, and syringes.

Kits are also provided that are useful for various purposes, *e.g.,* for treatment of disease or condition associated with CD40 signaling (*e.g*., autoimmune disease and/or inflammatory disease and/or cancer and/or transplantation-related disease), or for delivering an anti-CD40 antibody (such as a full-length anti-CD40 antibody) to a cell producing CD40 of the individual, optionally in combination with the articles of manufacture. Kits of the application include one or more containers comprising anti-CD40 antibody composition (or unit dosage form and/or article of manufacture), and in some embodiments, further comprise another agent (such as the agents described herein) and/or instructions for use in accordance with any of the methods described herein. The kit may further comprise a description of selection of individuals suitable for treatment. Instructions supplied in the kits of the application are typically written instructions on a label or package insert (*e.g.,* a paper sheet included in the kit), but machine-readable instructions (*e.g*., instructions carried on a magnetic or optical storage disk) are also acceptable.

For example, in some embodiments, the kit comprises a composition comprising an anti-CD40 antibody (such as a full-length anti-CD40 antibody). In some embodiments, the kit comprises a) a composition comprising any one of the anti-CD40 antibodies described herein, and b) an effective amount of at least one other agent, wherein the other agent enhances the effects (e.g., treatment effect, detecting effect) of the anti-CD40 antibody. In some embodiments, the kit comprises a) a composition comprising any one of the anti-CD40 antibodies described herein, and b) instructions for administering the anti-CD40 antibody composition to an individual for treatment of a disease or condition associated with CD40 signaling (e.g., autoimmune disease and/or inflammatory disease and/or cancer and/or transplantation-related disease). In some embodiments, the kit comprises a) a composition comprising any one of the anti-CD40 antibodies described herein, b) an effective amount of at least one other agent, wherein the other agent enhances the effect (e.g., treatment effect, detecting effect) of the anti-CD40 antibody, and c) instructions for administering the anti-CD40 antibody composition and the other agent(s) to an individual for treatment of a disease or condition associated with CD40 signaling (*e.g*., autoimmune disease and/or inflammatory disease and/or cancer and/or transplantation-related disease. The anti-CD40 antibody and the other agent(s) can be present in separate containers or in a single container. For example, the kit may comprise one distinct composition or two or more compositions wherein one composition comprises an anti-CD40 antibody and another composition comprises another agent.

In some embodiments, the kit comprises a nucleic acid (or a set of nucleic acids) encoding an anti-CD40 antibody (such as a full-length anti-CD40 antibody). In some embodiments, the kit comprises a) a nucleic acid (or a set of nucleic acids) encoding an anti-CD40 antibody, and b) a host cell for expressing the nucleic acid (or a set of nucleic acids). In some embodiments, the kit comprises a) a nucleic acid (or a set of nucleic acids) encoding an anti-CD40 antibody, and b) instructions for i) expressing the anti-CD40 antibody in a host cell, ii) preparing a composition comprising the anti-CD40 antibody, and iii) administering the composition comprising the anti-CD40 antibody to an individual for the treatment of a disease or condition associated with CD40 signaling (e.g., autoimmune disease and/or inflammatory disease and/or cancer and/or transplantation-related disease. In some embodiments, the kit comprises a) a nucleic acid (or a set of nucleic acids) encoding an anti-CD40 antibody, b) a host cell for expressing the nucleic acid (or a set of nucleic acids), and c) instructions for i) expressing the anti-CD40 antibody in the host cell, ii) preparing a composition comprising the anti-CD40 antibody, and iii) administering the composition comprising the anti-CD40 antibody to an individual for the treatment of a disease or condition associated with CD40 signaling (e.g., autoimmune disease and/or inflammatory disease and/or cancer and/or transplantation-related disease).

The kits of the application are in suitable packaging. Suitable packaging includes, but is not limited to, vials, bottles, jars, flexible packaging (*e.g*., sealed Mylar or plastic bags), and the like. Kits may optionally provide additional components such as buffers and interpretative information. The present application thus also provides articles of manufacture, which include vials (such as sealed vials), bottles, jars, flexible packaging, and the like.

The instructions relating to the use of the anti-CD40 antibody compositions generally include information as to dosage, dosing schedule, and route of administration for the intended treatment. The containers may be unit doses, bulk packages (e.g., multi-dose packages) or sub-unit doses. For example, kits may be provided that contain sufficient dosages of an anti-CD40 antibody (such as a full-length anti-CD40 antibody) as disclosed herein to provide effective treatment of an individual for an extended period, such as any of a week, 8 days, 9 days, 10 days, 11 days, 12 days, 13 days, 2 weeks, 3 weeks, 4 weeks, 6 weeks, 8 weeks, 3 months, 4 months, 5 months, 7 months, 8 months, 9 months, or more. Kits may also include multiple unit doses of the anti-CD40 antibody and pharmaceutical compositions and instructions for use and packaged in quantities sufficient for storage and use in pharmacies, for example, hospital pharmacies and compounding pharmacies.

Those skilled in the art will recognize that several embodiments are possible within the scope and spirit of this application. The application will now be described in greater detail by reference to the following non-limiting examples. The following examples further illustrate the application but, of course, should not be construed as in any way limiting its scope.

### EXAMPLES

In the experimental disclosure which follows, the following abbreviations apply: CD40 (Clusters of differentiation 40); Bavih-hCD40 (Biotin-hCD40-Avi-10His)

### Example 1: Generation of recombinant CD40/CD40L and selection of anti-CD40 scFv antibodies

### Generation of recombinant CD40 extracellular domain lCD40L antigen

The extracellular domain sequence of human CD40 (hCD40) (synthesized by Shanghai Generay Biotech Co., Ltd,) was subcloned into mammalian expression vector and expressed in 293F cells. His-tag, mouse Fc-tag, human Fc-tag, Avi-tag or other conventionally used tags were used to tag human CD40 extracellular domain, and then several forms of fusion protein containing recombinant CD40 extracellular domain were constructed and expressed, including CD40-His, CD40-Avi-His, CD40-mFc, and CD40-hFc. "his or His" stands for His-tag, "mFc" stands for mouse Fc-tag, "hFc" stands for human Fc-tag, and "Avi" stands for Avi tag.

As described above, the recombinant vector expressing the extracellular domain of Rhesus monkey CD40 (RheCD40) was constructed and expressed in 293F cells.

Additionally, the full-length sequence of human CD40L (synthesized by GenScript Biotech Corporation, Nanjing) was subcloned into mammalian expression vector and expressed in 293F cells. Fc-tag or other conventionally used tags were used to tag human CD40L. A dimer form of recombinant CD40L-Fc fusion protein was produced (hereon referred to as Fc-3*G4S-CD40L).

The expression and purification of recombinant CD40 extracellular domain or CD40L fusion protein, including human or Rhesus monkey CD40-His, CD40-Avi-His, CD40-mFc, CD40-hFc and Fc-3*G4S-CD40L, was carried out according to manufacturer's protocol. Briefly, 293F cells were transfected with the expression vectors containing genes for the fusion proteins described above, respectively, and cultured at 37°C, 5% CO₂ and 120rpm for 5 days. The culture media was collected respectively.

Recombinant proteins with His-tag were purified using Ni Sepharose purification according to manufacturer's protocol. Specifically, the Qiagen Ni-NTA superflow cartridges were used for immobilized metal affinity chromatography (IMAC) analysis. The cartridges were first equilibrated with buffer A 1 (50mM Na₃PO₄, 0.15M NaCl, pH 7.2) with a flow rate of 150cm/h. The culture supernatant, whose pHwas adjusted to 7.2, flowed through the cartridges at room temperature (RT) with a flow rate of 150cm/h. Next, 6×column volumes of buffer A1 was used to equilibrate the cartridges at the flow rate of 150cm/h. 10 × column volumes of 50mM PB solution (containing 0.15M NaCl and 0.2M Imidazole, pH 7.2) was used to wash the cartridges and the elution was collected.

The fusion proteins with Fc-tag were purified using protein A resin based on the protocol provided by manufacturer's guide. Specifically, Protein A column was first equilibrated with 6×column volumes of PBS buffer (containing 50mM PBS and 0.15M NaCl, pH7.2), at a flow rate of 150cm/h. The culture supernatant, whose pH was adjusted to 7.2, flowed through the column at room temperature with a flow rate of 150cm/h. Upon full equilibration, 50mM sodium citrate (pH3.5) was used for elution and the elution was collected.

### Generation of biotinylated CD40 extracellular domain antigen

hCD40-Avi-His was biotinylated by using biotin ligase B0101A (GeneCopoeia) according to the manufacturer's protocol. Briefly, buffer A/B and BirA ligase were added to hCD40-Avi-His and incubated for 2 hours at 30°C. The biotinylated hCD40-Avi-His was referred to as Bavih- hCD40. The efficiency of biotinylation was measured by ELISA, determined to be at least 70%.

### Selection of Anti-CD40 scFv antibodies

Generation of scFv antibody phage display library: Balb/c mice were immunized with human CD40 extracellular domain antigen and adjuvant. After immunization, serum was collected from each immunized animal and analyzed for IgG titer by ELISA. After several rounds of immunization, peripheral blood, lymph nodes and spleen were used to establish a phage library. Briefly, RNAs were extracted from peripheral blood, lymph nodes and spleen of the immunized mice and then reverse-transcribed into cDNAs. V_{H} and V_{K} fragments were amplified using V_{H}- and V_{K}-specific primers. Upon gel extraction and purification, scFvs were generated by linking V_{H} and V_{K}, and were cloned into the phage display plasmid pDNA5, which were then electroporated into *TGI* competent cells to generate scFv antibody phage display library.

Selection of anti-CD40 scFv antibodies: After several rounds of panning, scFvs which specifically bound to human CD40 were isolated from the phage display library. Briefly, scFv phage library at 2×10¹¹ PFU was added to Bavih-hCD40 and incubated at 37°C for 2 h. The phages binding with hCD40 were captured by streptavidin magnetic beads and the unbound phages were washed off. After washing 8-15 times (the times of washing increased with increasing number of selection rounds) with TBST solution, the phages specifically binding to hCD40 were eluted with Glycine-HCl solution (pH2.2). *TGI* cells in exponentially growing phase were infected with those phages. After adding ampicillin and incubating for 1 h, helper phage were added and incubated overnight at 28°C and 200rpm shaking. Next day, the culture medium was collected and centrifugated, then, the supernatant was harvested and subjected to the next round of screening until the positive scFv antibody library was obtained.

*Selection of anti-CD40 scFv antibodies using ELISA:* The phages enriched from the previous MACS panning were screened by ELISA. Briefly, hCD40 was dissolved in PBS and coated on 96-well plates with 0. 1µg/well followed by incubation at 4 °C overnight. The 96-well plates were washed with PBST before adding antibodies. 90 µL of PBS containing 4% skim milk was added to each well, and then 10µL of supernatant containing phage-scFv was added to the corresponding well and incubated for 1-2h at 37°C. After washing with PBST solution, the HRP labeled M13 mab (Sino Biological, 11973-MM05T-H) was added with 100µL/well and incubated for 1h at 37°C. After washing three times with PBST, 100µL of TMB was added to each well. The plates were incubated for 10min at room temperature avoiding light contact. The development reaction was terminated using 2 M H₂SO₄. Plates were read for the absorbance at 450 nm using a microplate reader. Positive phages were selected for sequencing and those with correct sequences were scaled up in culture. The plasmids were extracted from the phages and stored for further use. A panel of positive scFv antibodies were obtained at the end of screening.

### Example 2: Generation and characterization of full-length anti-CD40 chimeric antibodies

### Generation of full-length anti-CD40 chimeric antibodies

The positive scFv antibodies were reformatted as chimeric antibody molecules with a human IgG1 or IgG4 heavy chain constant domain, and a human kappa light chain constant domain. V_{H} and V_{L} were amplified from the phage expression vectors and introduced into eukaryotic expression vectors pTT5-L1 (containing kappa constant domain) and pTT5-H1 (containing IgG1 heavy chain constant domain), or pTT5-H4 (containing IgG4 heavy chain constant domain), respectively. Plasmids expressing the light or heavy chains were extracted and used to co-transfect 293F cells. After the cells were cultured at 37°C, 8% CO₂ and 120rpm for 5 days, the culture media was purified using Protein A affinity chromatography. Briefly, Protein A column was first equilibrated with 6 × column volumes of 50mM PBS buffer (containing 0.15M NaCl, pH7.2) at a flow rate of 150cm/h. The supernatant of the culture media (pH was adjusted to 7.2) was passed through the column at 150cm/h. Upon further equilibration, 50mM sodium citrate (pH3.5) was used to wash the column and the elution was collected. The obtained full-length antibodies were then subjected to further biochemical and biological analysis.

HEK-Blue^{™} CD40L NF-κB Activation Assay: HEK-Blue^{™} CD40L cell lines (InvivoGen) stably express human CD40 and NF-κB-induced secreted embryonic alkaline phosphatase (SEAP). Activation of CD40 on HEKBlue^{™} CD40L cells induces downstream signaling events leading to activation of NF-κB and secretion of SEAP, which can be measured by QUANTI-Blue^{™} substrate conversion rate. Briefly, according to the vendor's protocol, HEK-Blue^{™} CD40L cells were maintained and seeded into 96 well culture plates with 100µL/well (1×10⁵ cells/mL). 50µL Fc-3*G4S-CD40L(0.32µg/mL) and serial dilution of CD40 antibodies starting at a concentration of 1.2µg/ml were pre-mixed and added into the 96 well culture plates. After incubation at 37°C, 5% CO₂ for 24h, 40µL aliquots of the supernatant were mixed with 160µL pre-warmed QUANTI-Blue^{™} (Invivogen) solution, and incubated for 60-90 minutes. The absorbance at 650nm was read. IC₅₀ was calculated by PRISMI.

As shown in Table 5, all the selected anti-CD40 antibodies D1-D7 could inhibit human CD40L-induced NF-κB activation, and exhibited comparable or better inhibition efficacy than the reference antibody ASKP1240(an anti-CD40 antibody, by Kyowa Kirin).

**Table 5**

| Antibody | IC₅₀ (µg/mL) |
|---|---|
| D1 | 0.02150 |
| D2 | 0.01707 |
| D3 | 0.02243 |
| D4 | 0.01672 |
| D5 | 0.01418 |
| D6 | 0.00712 |
| D7 | 0.01702 |
| ASKP1240 | 0.01108 |

*ELISA Binding Assay:* The selected monoclonal full-length antibodies D1, D2, D3, D5, D6, or D7 were subjected to binding assays with CD40 extracellular domain of different species, respectively. The assay was designed to identify full-length antibodies that cross-reacted with CD40 of different species. Briefly, the human or Rhesus monkey CD40 prepared in example 1 was dissolved in PBS and coated on 96-well plates with 0. 1µg/well followed by incubation at 4 °C overnight. The 96-well plates were washed with TBST before adding antibodies, blocked with 5% milk for 1-2 h at 37°C and washed again with TBST. Firstly, each antibody sample was diluted to 10 µg/mL, and then serially diluted at a 1:3 ratio. 100µL of serially diluted antibodies were added to each well and incubated for 1 h at 37°C. After washing with TBST for 6 times, 100µL of the secondary antibody (goat anti-human IgG- HRP (Sigma, A8667)) was added to each well and incubated for 1 h at 37°C. After washing with TBST for 3 times, TMB was added with 100 µL/well and incubated for 10-20 minutes at 37°C. 2M H₂SO₄ was used to stop the reaction and plates were read for the absorbance at 450 nm. The ELISA results were then analyzed and the binding curves were generated by PRISM.

As shown in Table 6, all the selected anti-CD40 antibodies and the reference antibody ASKP1240 exhibited great binding affinity to hCD40 and RheCD40.

**Table 6**

| Antibody | EC₅₀ with hCD40 (µg/mL) | EC₅₀ with RheCD40 (µg/mL) |
|---|---|---|
| D1 | 0.2359 | 0.2274 |
| D2 | 0.1929 | 0.4732 |
| D3 | 0.2236 | 0.0743 |
| D5 | 0.2740 | 0.6266 |
| D6 | 0.1760 | 0.2435 |
| D7 | 0.2606 | 0.1328 |
| ASKP1240 | 0.2107 | 0.1813 |

### Inhibition of CD95 expression in Ramos cells by anti-CD40 antibody:

Ramos cell is a human Burkitt's lymphoma-derived cell line and express CD40 and CD95 (Fas) on cell surface. CD40L can induce increased expression of CD95 on the surface of Ramos cells. The ability of the anti-CD40 antibody D1, D6, D7 or the reference antibody ASKP1240 to inhibit Fc-3*G4S-CD40L induced CD95 expression in Ramos cells was determined according to the following protocol.

Ramos cell suspension was inoculated into a 96-well plate at 1.0×10⁶ cells/mL with 100µL/well. 50µL Fc-3*G4S-CD40L(4µg/mL) and serial dilution of CD40 antibody starting at a concentration of 1µg/mL were pre-mixed and added into the 96 well assay plates containing the cells. After incubation at 37°C, 5% CO₂ for 24h, the cells were collected and then labeled with APC-labeled anti-CD95 antibodies (BioLegend, 305612). FACS analysis was performed to obtain the mean fluorescence intensity (MFI) at different antibody concentrations.

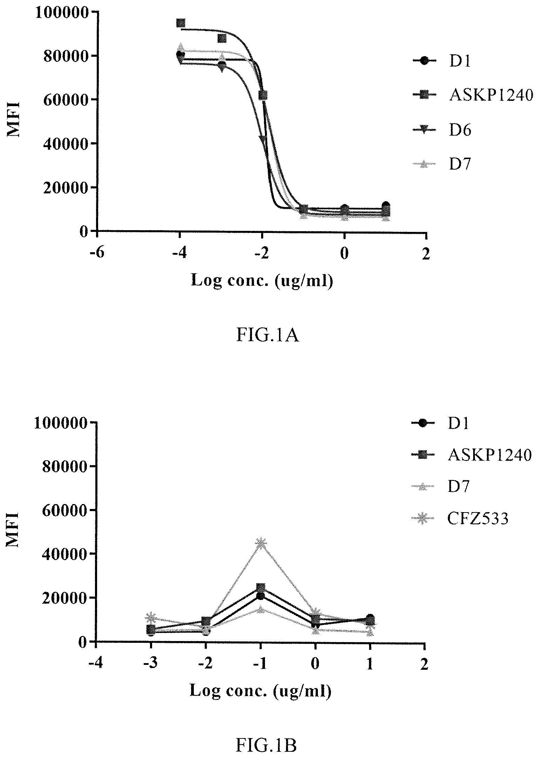

As shown in FIG.1A, anti-CD40 antibody D1, D6 or D7 could inhibit human CD40L-induced CD95 expression and exhibited comparable or better inhibiting efficacy than the reference antibody ASKP1240.

### Effect of cross-linking by anti-immunoglobulin antibody:

CD40 belongs to the TNF receptor superfamily, the multimerization of which on cells can lead to CD40 pathway activation. In order to test whether antibodies in the present invention are likely to have side effects such as activation of antibody cross-linking, anti-human IgG antibody was used to cross-link CD40 antibodies. If the co-treatment of human IgG antibody and the anti-CD40 antibody of the invention causes an increased expression of CD95 on the surface of Ramos cells, it indicates that the CD40 pathway in the cells is activated, and that the anti-CD40 antibody has the side effect of antibody cross-linking activation. CD95 expression in Ramos cells was determined according to the following protocol.

Ramos cell suspension was inoculated into a 96-well plate at 1.0×10⁶ cells/ml with 100µL/well. 50µL anti-human IgG antibody (4µg/mL) and serial dilution of CD40 antibody D1, D7, ASKP1240 or CFZ533 starting at a concentration of 10µg/mL were pre-mixed and added to the 96 well assay plates containing the cells. After incubation at 37°C, 5% CO₂ for 24h, the cells were collected and then labeled with APC-labeled anti-CD95 antibodies (BioLegend, 305612). FACS analysis was performed to obtain the mean fluorescence intensity (MFI) at different antibody concentrations.

As shown in FIG.1B, anti-CD40 antibodies D1, D7 or ASKP1240 exhibited little CD40 agonistic activity even cross-linking, however, the reference antibody CFZ533 showed agonistic activity.

### Inhibition of CD23 expression in PBMCs by anti-CD40 antibody:

Human Peripheral Blood Mononuclear Cells (PBMCs) contain B cells and can be used to characterize the effect of CD40 antibody on B cell activation. CD23 expression could be induced by CD40L and was considered as an indicator of B cells activation. The ability of the anti-CD40 antibodies D1, D6, D7 and ASKP1240 to inhibit Fc-3*G4S-CD40L induced CD23 expression in B cells was determined according to the following protocol.

PBMC suspension was inoculated into a 96-well plate at 8.0×10⁶ cells/mL with 100µL/well. 50µL Fc-3*G4S-CD40L(4µg/mL) and serial dilution of CD40 antibodies D1, D6, D7 or ASKP1240 starting at a concentration of 1.2µg/mL were pre-mixed and added into the 96 well assay plates containing the cells. After incubation at 37°C, 5% CO₂ for 48h, the cells were collected and then labeled with APC-labeled anti-CD19 antibodies (Biolegend, 302212) and FITC -labeled anti-CD23 antibodies (Biolegend, 338506). FACS analysis was performed to obtain the mean fluorescence intensity (MFI) at different antibody concentrations.

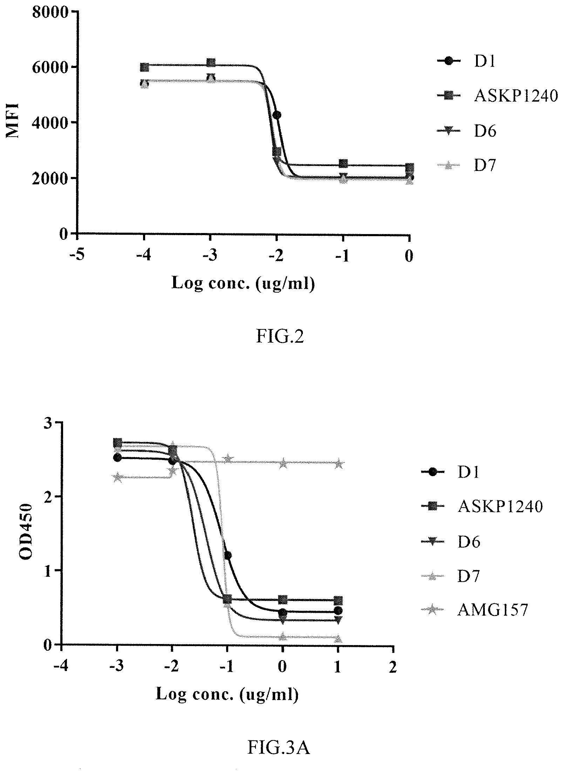

As shown in FIG.2, anti-CD40 antibodies D1, D6 and D7 exhibited comparable efficacy to the reference antibody ASKP1240 in inhibiting human CD40L induced CD23 expression in B cells.

### Measurement of IL12p40 and TNF-α by ELISA:

Monocytes were isolated from PBMC according to the Manual EasySep^{™} protocol (STEMCELL, Vancouver, Canada). Isolated monocytes were plated in complete media containing IL-4 (100ng/mL) and GM- CSF (50ng/mL) and incubated for 6 days at 37°C, 5% CO₂, where freshly media containing the same concentration of cytokines was replaced every other day. iDCs (immature dendritic cells) were harvested at day 6. iDCs secreted IL 12p40 and TNF-α after being stimulated by CD40L for 24 hours. The ability of the anti-CD40 antibody D1, D6, D7 or ASKP1240 to inhibit Fc-3*G4S-CD40L induced IL12p40 and TNF-α secretion in iDCs was determined according to the following protocol.

iDC suspension was inoculated into a 96-well plate at 1.0×10⁷ cells/mL with 100µL/well. 50µL Fc-3*G4S-CD40L(4µg/mL) and serial dilution of CD40 antibodies starting at a concentration of 40µg/mL were pre-mixed and added to the 96 well assay plates containing the cells. After incubated at 37°C, 5% CO₂ for 24h, supernatants were collected and then analyzed by ELISA using human IL-12 (p40) ELISA Kit (biolegend, 430704) and human TNF-α ELISA Kit (biolegend, 430204). Plates were read for the absorbance at 450 nm. Among them, an irrelevant antibody AMG157 (TSLP antibody, Amgen) was used as negative control, and ASKP1240 was used as positive control.

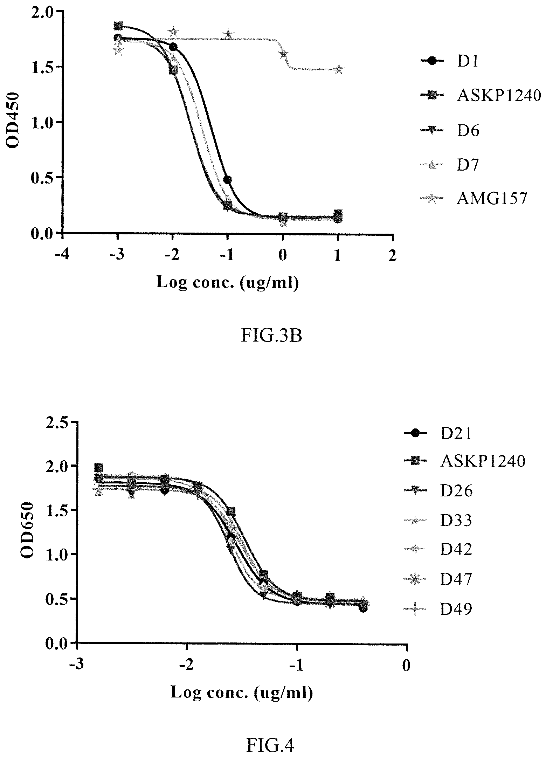

The results showed that anti-CD40 antibodies D1, D6 and D7 were able to inhibit the production of IL12p40 (Fig. 3A) and TNF-α (Fig. 3B), and the inhibitory effect was comparable to that of the reference antibody ASKP1240, while the negative control antibody AMG157 had no inhibitory activity.

### Example 3: Humanization of Anti-CD40 Antibodies

Based on the typical structure of V_{H}/V_{L}, CDR of the mouse antibody D1, heavy and light chain variable region sequences were aligned with the sequences in antibody Germline database to obtain human germline templates of high homology. The CDR regions of the mouse antibody were grafted into selected human germline templates to produce humanized variable regions, which were then recombined with corresponding human IgG constant regions (preferably IgG4 heavy chain and kappa light chain). The embedded residues, the residues that interact directly with the CDR regions, and the residues that have an important effect on the conformation of V_{L} and V_{H} were then reverse-mutated based on the three-dimensional structure of the mouse antibody, and chemically labile amino acid residues in the CDR regions were optimized to obtain the final humanized molecule D11. The heavy chain variable region sequence of D11 was shown in SEQ ID NO: 88 and the light chain variable region sequence of D11 was shown in SEQ ID NO: 127.

### Example 4: Characterization of the humanized Antibody

Activity of the humanized anti-CD40 antibody D 11 was tested according to the corresponding protocol as described in example 2, including the assays of HEK-Blue^{™} CD40L NF-κB Activation and Inhibition of CD95 expression in Ramos cells by anti-CD40 antibody.

The results showed that the humanized anti-CD40 antibody D11 exhibited slightly decreased in biological activity, affinity and dissociation constant as compared to its parent antibody D1 (data not shown).

### Example 5: Improved the affinity and the biological activity of the antibodies

In order to improve the affinity and the activity of the humanized anti-CD40 antibody, D11 was selected as the original antibody for affinity maturation. Mutations were performed in the CDR regions of the scFv D11, establishing the corresponding phage scFv display library. Variants that were able to bind human CD40 with high affinity and low dissociation rate were selected after screening, and their biological activities were assessed by the HEK-Blue^{™} CD40L NF-κB Activation Assay. ScFv antibodies that showed great affinity or biological activity were used to reformat as full-length antibodies according to the corresponding protocol as described in example 2. The full-length antibodies were screened by using the HEK-Blue^{™} CD40L NF-κB Activation Assay and then the selected optimized antibodies (D21-D51) were further subjected to biochemical and biological analysis.

### HEK⁻BlueTM CD40L NF-κB Activation Assay

Using the corresponding protocol described in Example 2, HEK-Blue^{™} CD40L NF-κB Activation Assay was performed with the full-length antibodies after affinity maturation from the humanized antibody to test the abilities of the optimized antibodies (reformatted as human IgG4) in inhibiting NF-κB Activation.

As shown in Table 7 and FIG.4, the optimized antibodies showed high ability to inhibit CD40L-induced NF-κB Activation and the inhibitory effects of these antibodies were better than or comparable to the reference antibody ASKP1240.

**Table 7**

| Antibody | IC₅₀ (µg/mL) | Antibody | IC₅₀ (µg/mL) |
|---|---|---|---|
| D21 | 0.01023 | D37 | 0.02111 |
| D22 | 0.02185 | D38 | 0.01558 |
| D23 | 0.01687 | D39 | 0.02995 |
| D24 | 0.01641 | D40 | 0.01421 |
| D25 | 0.02726 | D41 | 0.01944 |
| D26 | 0.00908 | D42 | 0.00962 |
| D27 | 0.02982 | D43 | 0.01677 |
| D28 | 0.01241 | D44 | 0.02009 |
| D29 | 0.03421 | D45 | 0.01568 |
| D30 | 0.01802 | D46 | 0.01118 |
| D31 | 0.01393 | D47 | 0.01140 |
| D32 | 0.01638 | D48 | 0.03069 |
| D33 | 0.01222 | D49 | 0.01118 |
| D34 | 0.03375 | D50 | 0.01418 |
| D35 | 0.02328 | D51 | 0.01837 |
| D36 | 0.02120 | ASPK1240 | 0.01336 |

### Affinity of anti-CD40 antibodies

### Characterization of the binding affinity and dissociation constant (Kd) of the optimized antibodies:

The binding affinity of the optimized anti-CD40 antibodies with human CD40 was characterized using Biacore 8K (GE). Anti-CD40 antibodies were stabilized on sensor chip CM5. The affinities for hCD40 at various concentrations were measured. Using the SPR technology, the association and dissociation rates were measured, and binding affinity was determined.

As shown in Table 8, the dissociation rate of the optimized anti-CD40 antibodies decreased significantly, and the binding affinity of D21, D26, D33, D42, D47 or D49 with human CD40 was better than or comparable to that of the reference antibody ASKP1240.

**Table 8**

| Antibody | Kon (1/Ms) | Koff (1/s) | Kd(M) |
|---|---|---|---|
| D21 | 2.384 E+5 | 6.055 E-04 | 2.540 E-09 |
| D26 | 2.175 E+5 | 5.960 E-04 | 2.740 E-09 |
| D33 | 3.035 E+5 | 4.935 E-04 | 1.626 E-09 |
| D42 | 2.207 E+5 | 2.773 E-04 | 1.256 E-09 |
| D47 | 1.907 E+5 | 3.026 E-04 | 1.587 E-09 |
| D49 | 1.961 E+5 | 2.950 E-04 | 1.505 E-09 |
| ASKP1240 | 2.976 E+5 | 6.169 E-04 | 2.073 E-09 |

### CD40 ELISA Binding Assay:

The biding affinity of the optimized full length CD40 antibodies with human or rhesus monkey CD40 was evaluated by ELISA according to the corresponding protocol as described in Example 2, and ASKP1240 was used as control.

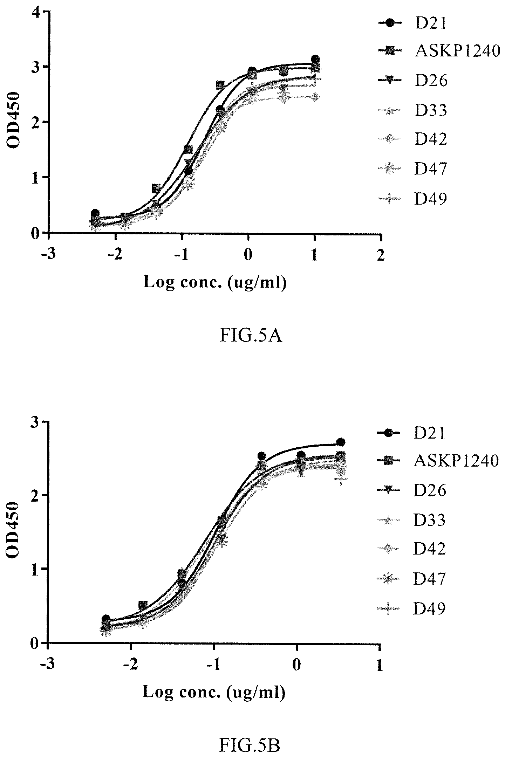

As shown in FIGs.5A-5B, the optimized antibodies D21, D26, D33, D42, D47 and D49 (reformatted as human IgG4) produced after affinity maturation from humanized antibody D11 all exhibited comparable affinity with human CD40 (FIG.5A) or rhesus monkey CD40 (FIG.5B) when compared to the reference antibody ASKP1240.

### Inhibition of CD95 expression in Ramos cells by anti-CD40 antibody:

The ability of the optimized anti-CD40 antibody D21, D26, D33, D42, D47, D49 or the reference antibody ASKP1240 (reformatted as human IgG4) in inhibiting Fc-3*G4S-CD40L induced CD95 expression in Ramos cells was determined by FACS according to the corresponding protocol as described in Example 2.

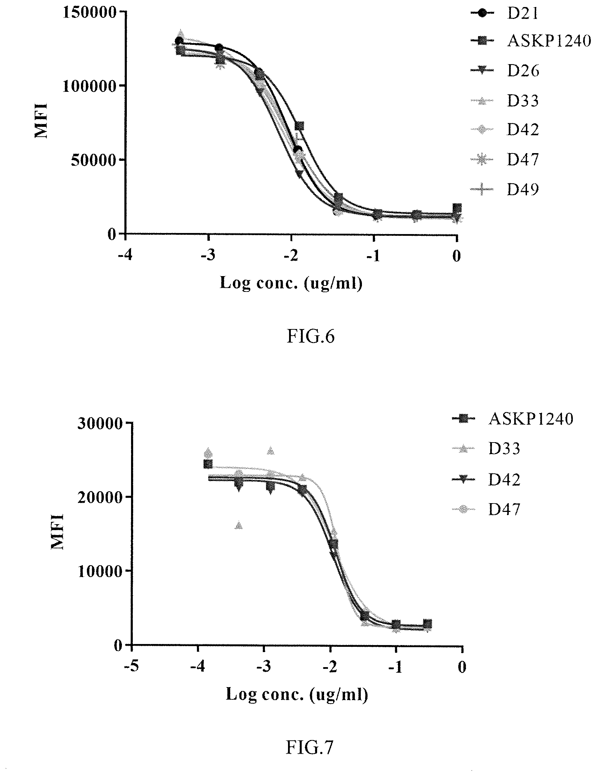

As shown in FIG.6, the optimized anti-CD40 antibody D21, D26, D33, D42, D47 or D49 exhibited better efficacy in inhibiting human CD40L induced CD95 expression than the reference antibody ASKP1240.

### Effect of cross-linking by anti-immunoglobulin antibody:

As described in Example 2, FACS was used to determine whether the optimized anti-CD40 antibody D33, D42 or D47 (reformatted as human IgG4) has the side effect of antibody cross-linking activation.

The results showed that after cross-linking by anti-human IgG antibody, the optimized anti-CD40 antibody D33, D42, D47 or ASKP1240 did not exhibit CD40 agonistic activity (data not shown).

### Cross-reactivity of anti-CD40 antibodies:

The cross-reactivities between anti-CD40 antibodies and the protein homologous with CD40 were detected. It was well known that there is high sequence homology among TNFR superfamily members. When compared with CD40 sequence, TNFRSF11A (38.95% homology) and TNFR2 (37.20% homology) are with the highest homology. The cross-reactions between the two proteins and anti-CD40 antibody were detected by ELISA, respectively. Briefly, human TNFRSF11A (Sino biological, 16078-h08h), human TNFR2 (Sino biological, 10417-h08h) or the human CD40 extracellular domain protein prepared in example 1 was coated on ELISA plate and incubated at 4°C overnight, respectively. After serial dilution, the optimized full-length anti-CD40 antibodies were added into 96 well plates and incubated respectively. After washing with TBST solution, the anti-human IgG-HRP antibody was added and incubated at RT. After washing with PBST, TMB was added into each well for development. Plates were read for the absorbance at 450 nm.

The results showed that even at a high concentration of 10µg/mL, the optimized antibody D33, D42 or D47 (reformatted as human IgG4) did not cross react with TNFRSF11A or TNFR2 (data not shown).

### Inhibition of CD23 expression in PBMCs by anti-CD40 antibody:

The ability of the optimized anti-CD40 antibody D33, D42 or D47 (reformatted as human IgG4) in inhibiting Fc-3*G4S-CD40L induced CD23 expression in PBMCs was determined by FACS according to the corresponding protocol as described in Example 2.

As shown in FIG.7, the optimized anti-CD40 antibody D33, D42 or D47 exhibited comparable efficacy in inhibiting human CD40L induced CD23 expression on B cells to the reference antibody ASKP1240.

### Example 6: T-Cell Dependent Antibody Responses in human CD40 transgenic mice

A T-cell dependent antibody response (TDAR) assay was performed in human CD40 transgenic mice. MBL Associated Serine Protease 2 (MASP2), which was emulsified in complete Freund's adjuvant, was administered to each animal on Day 0 with a dose of 100µg/animal . On Day 0, 7 and 14, mice in each experimental group were injected with anti-CD40 antibody (e.g., D33, D42 or D47) intravenously at a dose of 3mpk, and mice in the control group were injected with equal volume of PBS. Serum sample was collected from each animal at Day 7, 14 and 21 (relative to the first administration of MASP2) and then analyzed for the titer of anti-MASP2 IgM and IgG antibody by ELISA.

The results showed that the optimized anti-CD40 antibody D33, D42 or D47 almost blocked the generation of anti-MASP2 IgG (FIG.8A) and IgM (FIG.8B) antibodies completely at the dose of 3mpk.

To further evaluate whether anti-CD40 antibody D33, D42 or D47 could still inhibit T cell dependent antibody response at the dose of 1mpk, the following experiments were carried out. Keyhole limpet hemocyanin (KLH) was emulsified in complete Freund's adjuvant and administered to each animal on Day 0 with a dose of 100µg/animal. On Day 0, 7 and 14, mice in each experimental group were injected with anti-CD40 antibody (e.g., D33, D42, D47 or ASKP1240) intravenously at a dose of 1mpk, and mice in the control group were injected with equal volume of PBS, respectively. Serum sample was collected from each animal at Day 7, 14, 21 (relative to the first administration of MASP2) and then analyzed for the titer of anti-KLH IgM and IgG antibody by ELISA.

The results showed that the optimized anti-CD40 antibody D33, D42 or D47 blocked the production of anti-KLH IgG (FIG.8C) and IgM (FIG.8D) antibodies at the dose of 1mpk and exhibited comparable efficacy to the reference antibody ASKP1240.

### Example 7: Pharmacokinetics of anti-CD40 antibodies

PK value in SD rats: 12 female SD rats were randomly separated into four groups, with 3 in each group. Rats in each group were injected intravenously with 10mpk of K21, K24, K30 or ASKP1240, respectively. Blood was first collected before injection, then collected at 5min, 1h,5h, Id, 3d, 7d, 10d, 14d and 17d after injection, and plasma was obtained after centrifugation. The concentration of antibody was measured by ELISA. Pharmacokinetic parameters were determined by non-compartmental analysis (NCA) using WinNonlin^{®} software version 6.0. Using the respective mean concentration values for each antibody, all PK parameters including maximum serum concentration (Cmax), terminal half-life (T_{1/2}), apparent volume of distribution (Vd), clearance (Cl), mean residence time (MRTlast) and area under the curve from the dosing time to the last measureable concentration (AUClast) were determined using a linear trapezoidal rule with linear interpolation and uniform weighting.

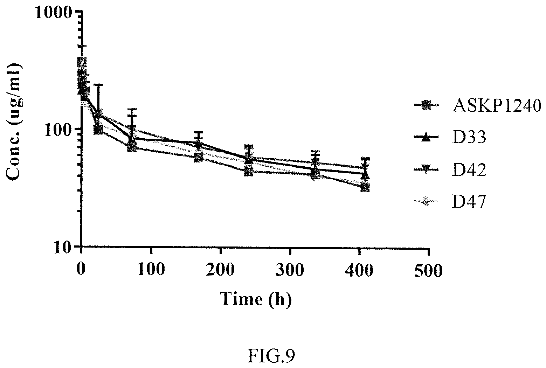

The results showed that during the trial period, there were no abnormal clinical manifestations related to the administration. The optimized anti-CD40 antibodies D33, D42, D47 and ASKP1240 exhibited similar drug serum concentrations curves in animals (FIG.9). There were no significant statistical differences in other pharmacokinetic parameters (data not shown).

## Claims

1. An isolated anti-CD40 antibody or antigen binding fragment thereof, wherein the anti-CD40 antibody or antigen binding fragment comprises:
a heavy chain variable domain (V_{H}) comprising:
a heavy chain complementarity determining region (HC-CDR) 1 comprising GYX₁VN (SEQ ID NO: 81), wherein X₁ is L, T, or V;
an HC-CDR2 comprising X₁IX₂PX₃X₄GX₅TX₆YNQKFKG (SEQ ID NO: 82), wherein X₁ is L or M, X₂ is A, N, or V, X₃ is E, K, L, M, R, S, T, or Y, X₄ is H, N, S, or T, X₅ is G or R, and X₆ is A, S, T, or W; and
an HC-CDR3 comprising SX₁X₂X₃X₄PYAX₅DY (SEQ ID NO: 83), wherein X₁ is G, I, L, M, P, R, S, or Y, X₂ is W or Y, X₃ is S or V, X₄ is D, K, L, N, R, T, or V, and X₅ is A, G, M, N, Q, or S;
and a light chain variable domain (V_{L}) comprising:
a light chain complementarity determining region (LC-CDR) 1 comprising KASQNVHTXiVA (SEQ ID NO: 84), wherein X₁ is A, H, N, or R;
an LC-CDR2 comprising LX₁SDRRT (SEQ ID NO: 85), wherein X₁ is A or R; and
an LC-CDR3 comprising LQX₁WX₂X₃PLT (SEQ ID NO: 86), wherein X₁ is A or H, X₂ is N, S, or T, and X₃ is A, Q, R, S, or Y.

2. An isolated anti-CD40 antibody or antigen binding fragment thereof, comprises:
a V_{H} comprising:
an HC-CDR1 comprising the amino acid sequence of any one of SEQ ID NOs: 1-3, or a variant thereof comprising up to about 3 amino acid substitutions;
an HC-CDR2 comprising the amino acid sequence of any one of SEQ ID NOs: 9-26, or a variant thereof comprising up to about 3 amino acid substitutions; and
an HC-CDR3 comprising the amino acid sequence of any one of SEQ ID NOs: 32-52, or a variant thereof comprising up to about 3 amino acid substitutions;
and a V_{L} comprising:
an LC-CDR1 comprising the amino acid sequence of any one of SEQ ID NOs: 58-61, or a variant thereof comprising up to about 3 amino acid substitutions;
an LC-CDR2 comprising the amino acid sequence of any one of SEQ ID NOs: 65-66, or a variant thereof comprising up to about 3 amino acid substitutions; and
an LC-CDR3 comprising the amino acid sequence of any one of SEQ ID NOs: 70-77, or a variant thereof comprising up to about 3 amino acid substitutions.

3. An isolated anti-CD40 antibody or antigen binding fragment thereof, comprises a V_{H} comprising an HC-CDR1, an HC-CDR2, and an HC-CDR3 of the V_{H} comprising the amino acid sequence of any one of SEQ ID NOs: 87-119; and a V_{L} comprising an LC-CDR1, an LC-CDR2, and an LC-CDR3 of the V_{L} comprising the amino acid sequence of any one of SEQ ID NOs: 126-138.

4. The isolated anti-CD40 antibody or antigen binding fragment of any one of claims 1-3, comprises:
(i) a V_{H} comprising an HC-CDR1 comprising the amino acid sequence of SEQ ID NO: 1, an HC-CDR2 comprising the amino acid sequence of SEQ ID NO: 9, and an HC-CDR3 comprising the amino acid sequence of SEQ ID NO: 32, or a variant thereof comprising up to about 5 amino acid substitutions in the HC-CDRs; and a V_{L} comprising an LC-CDR1 comprising the amino acid sequence of SEQ ID NO: 58, an LC-CDR2 comprising the amino acid sequence of SEQ ID NO: 65, and an LC-CDR3 comprising the amino acid sequence of SEQ ID NO: 70, or a variant thereof comprising up to about 5 amino acid substitutions in the LC-CDRs;
(ii) a V_{H} comprising an HC-CDR1 comprising the amino acid sequence of SEQ ID NO: 1, an HC-CDR2 comprising the amino acid sequence of SEQ ID NO: 10, and an HC-CDR3 comprising the amino acid sequence of SEQ ID NO: 32, or a variant thereof comprising up to about 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70, or a variant thereof comprising up to about 5 amino acid substitutions in the LC-CDRs;
(iii) a V_{H} comprising an HC-CDR1 comprising the amino acid sequence of SEQ ID NO: 1, an HC-CDR2 comprising the amino acid sequence of SEQ ID NO: 13, and an HC-CDR3 comprising the amino acid sequence of SEQ ID NO: 34, or a variant thereof comprising up to about 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70, or a variant thereof comprising up to about 5 amino acid substitutions in the LC-CDRs;
(iv) a V_{H} comprising an HC-CDR1 comprising the amino acid sequence of SEQ ID NO: 1, an HC-CDR2 comprising the amino acid sequence of SEQ ID NO: 15, and an HC-CDR3 comprising the amino acid sequence of SEQ ID NO: 37, or a variant thereof comprising up to about 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 73, or a variant thereof comprising up to about 5 amino acid substitutions in the LC-CDRs;
(v) a V_{H} comprising an HC-CDR1 comprising the amino acid sequence of SEQ ID NO: 1, an HC-CDR2 comprising the amino acid sequence of SEQ ID NO: 20, and an HC-CDR3 comprising the amino acid sequence of SEQ ID NO: 42, or a variant thereof comprising up to about 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70, or a variant thereof comprising up to about 5 amino acid substitutions in the LC-CDRs;
(vi) a V_{H} comprising an HC-CDR1 comprising the amino acid sequence of SEQ ID NO: 1, an HC-CDR2 comprising the amino acid sequence of SEQ ID NO: 14, and an HC-CDR3 comprising the amino acid sequence of SEQ ID NO: 43, or a variant thereof comprising up to about 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70, or a variant thereof comprising up to about 5 amino acid substitutions in the LC-CDRs;
(vii) a V_{H} comprising an HC-CDR1 comprising the amino acid sequence of SEQ ID NO: 1, an HC-CDR2 comprising the amino acid sequence of SEQ ID NO: 15, and an HC-CDR3 comprising the amino acid sequence of SEQ ID NO: 51, or a variant thereof comprising up to about 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 70, or a variant thereof comprising up to about 5 amino acid substitutions in the LC-CDRs;
(viii)a V_{H} comprising an HC-CDR1 comprising the amino acid sequence of SEQ ID NO: 1, an HC-CDR2 comprising the amino acid sequence of SEQ ID NO: 11, and an HC-CDR3 comprising the amino acid sequence of SEQ ID NO: 48, or a variant thereof comprising up to about 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 71, or a variant thereof comprising up to about 5 amino acid substitutions in the LC-CDRs; or
(ix) a V_{H} comprising an HC-CDR1 comprising the amino acid sequence of SEQ ID NO: 1, an HC-CDR2 comprising the amino acid sequence of SEQ ID NO: 18, and an HC-CDR3 comprising the amino acid sequence of SEQ ID NO: 47, or a variant thereof comprising up to about 5 amino acid substitutions in the HC-CDRs; and a V_{L} comprising an LC-CDR1 comprising the amino acid sequence of SEQ ID NO: 59, an LC-CDR2 comprising the amino acid sequence of SEQ ID NO: 65, and an LC-CDR3 comprising the amino acid sequence of SEQ ID NO: 71, or a variant thereof comprising up to about 5 amino acid substitutions in the LC-CDRs.

5. The isolated anti-CD40 antibody or antigen binding fragment of any one of claims 1-4, comprises:
a V_{H} comprising the amino acid sequence of any one of SEQ ID NOs: 87-119, or a variant thereof having at least about 90% sequence identity to the amino acid sequence of any one of SEQ ID NOs: 87-119; and a V_{L} comprising the amino acid sequence of any one of SEQ ID NOs: 126-138, or a variant thereof having at least about 90% sequence identity to the amino acid sequence of any one of SEQ ID NOs: 126-138.

6. The isolated anti-CD40 antibody or antigen binding fragment of claim 5, comprises:
(i) a V_{H} comprising the amino acid sequence of SEQ ID NO: 87, or a variant thereof having at least about 90% sequence identity to the amino acid sequence of SEQ ID NO: 87; and a V_{L} comprising the amino acid sequence of SEQ ID NO: 126, or a variant thereof having at least about 90% sequence identity to the amino acid sequence of SEQ ID NO: 126;
(ii) a V_{H} comprising the amino acid sequence of SEQ ID NO: 88, or a variant thereof having at least about 90% sequence identity to the amino acid sequence of SEQ ID NO: 88; and a V_{L} comprising the amino acid sequence of SEQ ID NO: 127, or a variant thereof having at least about 90% sequence identity to the amino acid sequence of SEQ ID NO: 127;
(iii) a V_{H} comprising the amino acid sequence of SEQ ID NO: 89, or a variant thereof having at least about 90% sequence identity to the amino acid sequence of SEQ ID NO: 89; and a V_{L} comprising the amino acid sequence of SEQ ID NO: 128, or a variant thereof having at least about 90% sequence identity to the amino acid sequence of SEQ ID NO: 128;
(iv) a V_{H} comprising the amino acid sequence of SEQ ID NO: 94, or a variant thereof having at least about 90% sequence identity to the amino acid sequence of SEQ ID NO: 94; and a V_{L} comprising the amino acid sequence of SEQ ID NO: 128, or a variant thereof having at least about 90% sequence identity to the amino acid sequence of SEQ ID NO: 128;
(v) a V_{H} comprising the amino acid sequence of SEQ ID NO: 96, or a variant thereof having at least about 90% sequence identity to the amino acid sequence of SEQ ID NO: 96; and a V_{L} comprising the amino acid sequence of SEQ ID NO: 132, or a variant thereof having at least about 90% sequence identity to the amino acid sequence of SEQ ID NO: 132;
(vi) a V_{H} comprising the amino acid sequence of SEQ ID NO: 101, or a variant thereof having at least about 90% sequence identity to the amino acid sequence of SEQ ID NO: 101; and a V_{L} comprising the amino acid sequence of SEQ ID NO: 128, or a variant thereof having at least about 90% sequence identity to the amino acid sequence of SEQ ID NO: 128;
(vii) a V_{H} comprising the amino acid sequence of SEQ ID NO: 110, or a variant thereof having at least about 90% sequence identity to the amino acid sequence of SEQ ID NO: 110; and a V_{L} comprising the amino acid sequence of SEQ ID NO: 128, or a variant thereof having at least about 90% sequence identity to the amino acid sequence of SEQ ID NO: 128;
(viii)a V_{H} comprising the amino acid sequence of SEQ ID NO: 114, or a variant thereof having at least about 90% sequence identity to the amino acid sequence of SEQ ID NO: 114; and a V_{L} comprising the amino acid sequence of SEQ ID NO: 128, or a variant thereof having at least about 90% sequence identity to the amino acid sequence of SEQ ID NO: 128;
(ix) a V_{H} comprising the amino acid sequence of SEQ ID NO: 115, or a variant thereof having at least about 90% sequence identity to the amino acid sequence of SEQ ID NO: 115; and a V_{L} comprising the amino acid sequence of SEQ ID NO: 129, or a variant thereof having at least about 90% sequence identity to the amino acid sequence of SEQ ID NO: 129; or
(x) a V_{H} comprising the amino acid sequence of SEQ ID NO: 117, or a variant thereof having at least about 90% sequence identity to the amino acid sequence of SEQ ID NO: 117; and a V_{L} comprising the amino acid sequence of SEQ ID NO: 129, or a variant thereof having at least about 90% sequence identity to the amino acid sequence of SEQ ID NO: 129.

7. An isolated anti-CD40 antibody or antigen binding fragment thereof, comprises:
a V_{H} comprising an HC-CDR1, an HC-CDR2, and an HC-CDR3 of a V_{H} comprising the amino acid sequence of SEQ ID NO: 120; and a V_{L} comprising an LC-CDR1, an LC-CDR2, and an LC-CDR3 of a V_{L} comprising the amino acid sequence of SEQ ID NO: 139.

8. The isolated anti-CD40 antibody or antigen binding fragment of claim 7, comprises:
a V_{H} comprising an HC-CDR1 comprising the amino acid sequence of SEQ ID NO: 4, an HC-CDR2 comprising the amino acid sequence of SEQ ID NO: 27, and an HC-CDR3 comprising the amino acid sequence of SEQ ID NO: 53, or a variant thereof comprising up to about 5 amino acid substitutions in the HC-CDRs; and a V_{L} comprising an LC-CDR1 comprising the amino acid sequence of SEQ ID NO: 62, an LC-CDR2 comprising the amino acid sequence of SEQ ID NO: 67, and an LC-CDR3 comprising the amino acid sequence of SEQ ID NO: 78, or a variant thereof comprising up to about 5 amino acid substitutions in the LC-CDRs.

9. The isolated anti-CD40 antibody or antigen binding fragment of any one of claims 7-8, comprises:
a V_{H} comprising the amino acid sequence of SEQ ID NO: 120, or a variant thereof having at least about 90% sequence identity to the amino acid sequence of SEQ ID NO: 120; and a V_{L} comprising the amino acid sequence of SEQ ID NO: 139, or a variant thereof having at least about 90% sequence identity to the amino acid sequence of SEQ ID NO: 139.

10. An isolated anti-CD40 antibody or antigen binding fragment thereof, comprises:
a V_{H} comprising an HC-CDR1, an HC-CDR2, and an HC-CDR3 of a V_{H} comprising the amino acid sequence of SEQ ID NO: 121; and a V_{L} comprising an LC-CDR1, an LC-CDR2, and an LC-CDR3 of a V_{L} comprising the amino acid sequence of SEQ ID NO: 140.

11. The isolated anti-CD40 antibody or antigen binding fragment of claim 10, comprises:
a V_{H} comprising an HC-CDR1 comprising the amino acid sequence of SEQ ID NO: 5, an HC-CDR2 comprising the amino acid sequence of SEQ ID NO: 27, and an HC-CDR3 comprising the amino acid sequence of SEQ ID NO: 54, or a variant thereof comprising up to about 5 amino acid substitutions in the HC-CDRs; and a V_{L} comprising an LC-CDR1 comprising the amino acid sequence of SEQ ID NO: 62, an LC-CDR2 comprising the amino acid sequence of SEQ ID NO: 67, and an LC-CDR3 comprising the amino acid sequence of SEQ ID NO: 78, or a variant thereof comprising up to about 5 amino acid substitutions in the LC-CDRs.

12. The isolated anti-CD40 antibody or antigen binding fragment of any one of claims 10-11, comprises:
a V_{H} comprising the amino acid sequence of SEQ ID NO: 121, or a variant thereof having at least about 90% sequence identity to the amino acid sequence of SEQ ID NO: 121; and a V_{L} comprising the amino acid sequence of SEQ ID NO: 140, or a variant thereof having at least about 90% sequence identity to the amino acid sequence of SEQ ID NO: 140.

13. An isolated anti-CD40 antibody or antigen binding fragment thereof, comprises:
a V_{H} comprising an HC-CDR1, an HC-CDR2, and an HC-CDR3 of a V_{H} comprising the amino acid sequence of SEQ ID NO: 122; and a V_{L} comprising an LC-CDR1, an LC-CDR2, and an LC-CDR3 of a V_{L} comprising the amino acid sequence of SEQ ID NO: 141.

14. The isolated anti-CD40 antibody or antigen binding fragment of claim 13, comprises:
a V_{H} comprising an HC-CDR1 comprising the amino acid sequence of SEQ ID NO: 6, an HC-CDR2 comprising the amino acid sequence of SEQ ID NO: 28, and an HC-CDR3 comprising the amino acid sequence of SEQ ID NO: 55, or a variant thereof comprising up to about 5 amino acid substitutions in the HC-CDRs; and a V_{L} comprising an LC-CDR1 comprising the amino acid sequence of SEQ ID NO: 63, an LC-CDR2 comprising the amino acid sequence of SEQ ID NO: 68, and an LC-CDR3 comprising the amino acid sequence of SEQ ID NO: 79, or a variant thereof comprising up to about 5 amino acid substitutions in the LC-CDRs.

15. The isolated anti-CD40 antibody or antigen binding fragment of any one of claims 13-14, comprises:
a V_{H} comprising the amino acid sequence of SEQ ID NO: 122, or a variant thereof having at least about 90% sequence identity to the amino acid sequence of SEQ ID NO: 122; and a V_{L} comprising the amino acid sequence of SEQ ID NO: 141, or a variant thereof having at least about 90% sequence identity to the amino acid sequence of SEQ ID NO: 141.

16. An isolated anti-CD40 antibody or antigen binding fragment thereof, comprises:
a V_{H} comprising an HC-CDR1, an HC-CDR2, and an HC-CDR3 of a V_{H} comprising the amino acid sequence of SEQ ID NO: 123; and a V_{L} comprising an LC-CDR1, an LC-CDR2, and an LC-CDR3 of a V_{L} comprising the amino acid sequence of SEQ ID NO: 142.

17. The isolated anti-CD40 antibody or antigen binding fragment of claim 16, comprises:
a V_{H} comprising an HC-CDR1 comprising the amino acid sequence of SEQ ID NO: 7, an HC-CDR2 comprising the amino acid sequence of SEQ ID NO: 29, and an HC-CDR3 comprising the amino acid sequence of SEQ ID NO: 56, or a variant thereof comprising up to about 5 amino acid substitutions in the HC-CDRs; and a V_{L} comprising an LC-CDR1 comprising the amino acid sequence of SEQ ID NO: 64, an LC-CDR2 comprising the amino acid sequence of SEQ ID NO: 69, and an LC-CDR3 comprising the amino acid sequence of SEQ ID NO: 80, or a variant thereof comprising up to about 5 amino acid substitutions in the LC-CDRs.

18. The isolated anti-CD40 antibody or antigen binding fragment of any one of claims 16-17, comprises:
a V_{H} comprising the amino acid sequence of SEQ ID NO: 123, or a variant thereof having at least about 90% sequence identity to the amino acid sequence of SEQ ID NO: 123; and a V_{L} comprising the amino acid sequence of SEQ ID NO: 142, or a variant thereof having at least about 90% sequence identity to the amino acid sequence of SEQ ID NO: 142.

19. An isolated anti-CD40 antibody or antigen binding fragment thereof, comprises:
a V_{H} comprising an HC-CDR1, an HC-CDR2, and an HC-CDR3 of a V_{H} comprising the amino acid sequence of SEQ ID NO: 124; and a V_{L} comprising an LC-CDR1, an LC-CDR2, and an LC-CDR3 of a V_{L} comprising the amino acid sequence of SEQ ID NO: 143.

20. The isolated anti-CD40 antibody or antigen binding fragment of claim 19, comprises:
a V_{H} comprising an HC-CDR1 comprising the amino acid sequence of SEQ ID NO: 8, an HC-CDR2 comprising the amino acid sequence of SEQ ID NO: 30, and an HC-CDR3 comprising the amino acid sequence of SEQ ID NO: 57, or a variant thereof comprising up to about 5 amino acid substitutions in the HC-CDRs; and a V_{L} comprising an LC-CDR1 comprising the amino acid sequence of SEQ ID NO: 62, an LC-CDR2 comprising the amino acid sequence of SEQ ID NO: 67, and an LC-CDR3 comprising the amino acid sequence of SEQ ID NO: 80, or a variant thereof comprising up to about 5 amino acid substitutions in the LC-CDRs.

21. The isolated anti-CD40 antibody or antigen binding fragment of any one of claims 19-20, comprises:
a V_{H} comprising the amino acid sequence of SEQ ID NO: 124, or a variant thereof having at least about 90% sequence identity to the amino acid sequence of SEQ ID NO: 124; and a V_{L} comprising the amino acid sequence of SEQ ID NO: 143, or a variant thereof having at least about 90% sequence identity to the amino acid sequence of SEQ ID NO: 143.

22. An isolated anti-CD40 antibody or antigen binding fragment thereof, comprises:
a V_{H} comprising an HC-CDR1, an HC-CDR2, and an HC-CDR3 of a V_{H} comprising the amino acid sequence of SEQ ID NO: 125; and a V_{L} comprising an LC-CDR1, an LC-CDR2, and an LC-CDR3 of a V_{L} comprising the amino acid sequence of SEQ ID NO: 144.

23. The isolated anti-CD40 antibody or antigen binding fragment of claim 22, comprises:
a V_{H} comprising an HC-CDR1 comprising the amino acid sequence of SEQ ID NO: 4, an HC-CDR2 comprising the amino acid sequence of SEQ ID NO: 31, and an HC-CDR3 comprising the amino acid sequence of SEQ ID NO: 53, or a variant thereof comprising up to about 5 amino acid substitutions in the HC-CDRs; and a V_{L} comprising an LC-CDR1 comprising the amino acid sequence of SEQ ID NO: 62, an LC-CDR2 comprising the amino acid sequence of SEQ ID NO: 68, and an LC-CDR3 comprising the amino acid sequence of SEQ ID NO: 79, or a variant thereof comprising up to about 5 amino acid substitutions in the LC-CDRs.

24. The isolated anti-CD40 antibody or antigen binding fragment of any one of claims 22-23, comprises:
a V_{H} comprising the amino acid sequence of SEQ ID NO: 125, or a variant thereof having at least about 90% sequence identity to the amino acid sequence of SEQ ID NO: 125; and a V_{L} comprising the amino acid sequence of SEQ ID NO: 144, or a variant thereof having at least about 90% sequence identity to the amino acid sequence of SEQ ID NO: 144.

25. The isolated anti-CD40 antibody or antigen binding fragment of any one of claims 1-24, wherein the anti-CD40 antibody or antigen binding fragment comprises an Fc fragment.

26. The isolated anti-CD40 antibody or antigen binding fragment of claim 25, wherein the anti-CD40 antibody or antigen binding fragment is a full-length IgG antibody.

27. The isolated anti-CD40 antibody or antigen binding fragment of claim 26, wherein the anti-CD40 antibody or antigen binding fragment is a full-length IgG1, IgG2, IgG3, or IgG4 antibody.

28. The isolated anti-CD40 antibody or antigen binding fragment of any one of claims 1-27, wherein the anti-CD40 antibody or antigen binding fragment is a chimeric, humanized or human antibody.

29. The isolated anti-CD40 antibody or antigen binding fragment of any one of claims 1-24, wherein the antigen binding fragment is selected from the group consisting of a Fab, a Fab', a F(ab)'2, a Fab'-SH, a single-chain Fv (scFv), an Fv fragment, a dAb, a Fd, a nanobody, a diabody, and a linear antibody.

30. An isolated nucleic acid molecule that encodes the anti-CD40 antibody or antigen binding fragment of any one of claims 1-29.

31. A vector comprises the nucleic acid molecule of claim 30.

32. An isolated host cell comprises the isolated anti-CD40 antibody or antigen binding fragment of any one of claims 1-29, the isolated nucleic acid of claim 30, or the vector of claim 31.

33. A method of producing an isolated anti-CD40 antibody or antigen binding fragment, comprising:
a) culturing the host cell of claim 32 under conditions effective to express the anti-CD40 antibody or antigen binding fragment; and
b) obtaining the expressed anti-CD40 antibody or antigen binding fragment from the host cell.

34. A pharmaceutical composition comprises the anti-CD40 antibody or antigen binding fragment of any one of claims 1-29, the nucleic acid of claim 30, the vector of claim 31, or the isolated host cell of claim 32, and a pharmaceutically acceptable carrier.

35. Use of the isolated anti-CD40 antibody or antigen binding fragment of any one of claims 1-29, the nucleic acid of claim 30, the vector of claim 31, the isolated host cell of claim 32, the antibody or antigen binding fragment produced according to claim 33, or the pharmaceutical composition of claim 34 in the manufacture of a medicament for treating a disease or condition in an individual in need.

36. A method of treating a disease or condition in an individual in need thereof, comprises administering to the individual an effective amount of the anti-CD40 antibody or antigen binding fragment according to any one of claims 1-29, the nucleic acid of claim 30, the vector of claim 31, the isolated host cell of claim 32, the antibody or antigen binding fragment produced according to claim 33 or the pharmaceutical composition of claim 34.

37. The use of claim 35 or the method of claim 36, wherein the disease or condition is an autoimmune disease, inflammatory disease, cancer and/or transplantation-related disease.

38. The use of claim 35 or the method of claim 36, wherein the disease or condition is selected from the group consisting of Systemic Lupus Erythematosus (SLE), discoid lupus, lupus nephritis, inflammatory arthritis: including but not limited to juvenile arthritis, rheumatoid arthritis, psoriatic arthritis, Reiter's syndrome, ankylosing spondylitis and gouty arthritis, organ or tissue transplant rejection: hyperacute, acute or chronic rejection and/or graftversus-host disease, multiple sclerosis, hyper IgE syndrome, polyarteritis nodosa, primary biliary cirrhosis, inflammatory bowel disease, Crohn's disease, celiac disease (glutensensitive bowel disease), autoimmune hepatitis, pernicious anemia, autoimmune hemolytic anemia, scleroderma, myasthenia gravis, idiopathic thrombocytopenic purpura, autoimmune thyroiditis, Grave's disease, Hasimoto's thyroiditis, immune complex diseases, Chronic Fatigue Immune Dysfunction Syndrome (CFIDS), polymyositis, dermatomyositis, cryoglobulinemia, thrombolysis, cardiomyopathy, pemphigus vulgaris, interstitial pulmonary fibrosis, type I and type II diabetes, type 1, 2, 3 and 4 delayed-type hypersensitivity, allergic or allergic disorders, unwanted/unintended immune reactions to therapeutic proteins(e.g., factor VII in hemophilia), asthma, Rich-Strauss syndrome (allergic granulomatosis), atopic dermatitis, allergic and irritant contact dermatitis, urticaria, IgE-mediated allergy, atherosclerosis, vasculitis, idiopathic inflammatory myopathy, hemolytic diseases, Alzheimer's disease, chronic inflammatory demyelinating polyneuropathy, Sjogren's disease, psoriasis, hidradenitis suppurativa, suppurative urethritis, sarcoidosis, ulcerative colitis, cancer: including but not limited to Non-Hodgkins lymphoma, chronic lymphocytic leukemia, multiple myeloma, B-cell lymphoma, high-level/mid-level/lower B-cell lymphoma, acute B-cell lymphoblastic leukemia, myeloblastic leukemia and Hodgkin's disease, nephritis, respiratory distress syndrome, epididymitis, glomerulonephritis, hyperthyroidism.
